# EUROPÄISCHE PATENTSCHRIFT

(11) **EP 1 908 842 B1**
(45) Veröffentlichungstag und Bekanntmachung des Hinweises auf die Patenterteilung: **18.09.2013**
(21) Anmeldenummer: 07123785.3
(22) Anmeldetag: 15.12.2004
(51) Int. Cl.: C12N 15/77, C07K 14/34, C12N 1/21, C12P 13/08, C12P 13/12

(54) **PEF-TU Expressionseinheiten**
PEF-TU expression units
Unités d'expression PEF-TU

(30) Priorität: 18.12.2003 DE 10359594
(43) Veröffentlichungstag der Anmeldung: 09.04.2008
(62) Teilanmeldung aus: 04803887.1
(73) Patentinhaber: BASF SE, 67056 Ludwigshafen (DE)
(72) Erfinder: Kröger, Burkhard, 67117 Limburgerhof (DE); Zelder, Oskar, 67346 Speyer (DE); Klopprogge, Corinna, 68239 Mannheim (DE); Schröder, Hartwig, 69226 Nußloch (DE); Haefner, Stefan, 67346 Speyer (DE)

(56) Entgegenhaltungen:
- WO-A-02/40679
- WO-A-03/040180
- DATABASE Geneseq [Online] 26. September 2001 (2001-09-26), "C glutamicum coding sequence fragment SEQ ID NO: 7060." XP002359696 gefunden im EBI accession no. GSN:AAH68525 Database accession no. AAH68525 -& EP 1 108 790 A (KYOWA HAKKO KOGYO CO., LTD) 20. Juni 2001 (2001-06-20)
- PÁTEK M ET AL: "Promoters of Corynebacterium glutamicum" JOURNAL OF BIOTECHNOLOGY, ELSEVIER SCIENCE PUBLISHERS B.V., AMSTERDAM, NL, Bd. 104, Nr. 1-3, 4. September 2003 (2003-09-04), Seiten 311-323, XP002323247 ISSN: 0168-1656
- TIELEMAN L N ET AL: "Growth phase-dependent transcription of the Streptomyces ramocissimus tuf1 gene occurs from two promoters." JOURNAL OF BACTERIOLOGY. JUN 1997, Bd. 179, Nr. 11, Juni 1997 (1997-06), Seiten 3619-3624, XP002360417 ISSN: 0021-9193
- AN G ET AL: "Evidence for an internal promoter preceding tufA in the str operon of Escherichia coli." JOURNAL OF BACTERIOLOGY. FEB 1982, Bd. 149, Nr. 2, Februar 1982 (1982-02), Seiten 548-553, XP002360418 ISSN: 0021-9193
- DATABASE GENBANK [Online] 08 August 2002 NAKAGAWA S. ET AL: 'Corynebacterium glutamicum ATCC 13032 DNA, complete genome, section 2/10.' Database accession no. AP005275
- DATABASE BIOSIS [Online] BIOSCIENCES INFORMATION SERVICE, PHILADELPHIA, PA, US 1992 WEIJLAND A ET AL: 'ELONGATION FACTOR TU A MOLECULAR SWITCH IN PROTEIN BIOSYNTHESIS' Database accession no. PREV199293138481 & WEIJLAND A ET AL: "ELONGATION FACTOR TU A MOLECULAR SWITCH IN PROTEIN BIOSYNTHESIS", MOLECULAR MICROBIOLOGY, vol. 6, no. 6, 1992, pages 683-688, ISSN: 0950-382X

### SEQUENCE LISTING

<110> BASF Aktiengesellschaft
<120> P EF-TU-Expressionseinheiten
<130> PF 55183/Mec
<140> 20030320
   <141>
<160> 42
<210> 1
   <211> 186
   <212> DNA
   <213> Corynebacterium glutamicum
<220>
   <223> EFTU\RXA01284\PROMOTOR
<400> 1
   ggccgttacc ctgcgaatgt ccacagggta gctggtagtt tgaaaatcaa cgccgttgcc 60
   cttaggattc agtaactggc acattttgta atgcgctaga tctgtgtgct cagtcttcca 120
   ggctgcttat cacagtgaaa gcaaaaccaa ttcgtggctg cgaaagtcgt agccaccacg 180
   aagtcc                                                            186
<210> 2
   <211> 199
   <212> DNA
   <213> Corynebacterium glutamicum
<220>
   <223> EFTU\RXA01284\GESAMTE
<400> 2
   ggccgttacc ctgcgaatgt ccacagggta gctggtagtt tgaaaatcaa cgccgttgcc 60
   cttaggattc agtaactggc acattttgta atgcgctaga tctgtgtgct cagtcttcca 120
   ggctgcttat cacagtgaaa gcaaaaccaa ttcgtggctg cgaaagtcgt agccaccacg 180
   aagtccagga ggacataca                                               199
<210> 3
   <211> 1365
   <212> DNA
   <213> Corynebacterium glutamicum
<220>
   <223> RXA00077
<400> 3
   atgaatgatg agaatattca aagctccaac tatcagccat tcccgagttt tgacgattgg 60
   aaacagatcg aggtgtcgct cttagatgtc atcgaatcct cacgccattt ttctgatttg 120
   aaagatagca ctgatcgttc tgcgttagat gctgcgctag agagagcaaa aagagctgcc 180
   gcagttgata ccaatgccat agaaggaatc ttccaaactg atcgcggttt tacccataca 240
   gttgcaacgc aggtaggggc ttgggagcaa caaatggcga tgaaaggcaa acatgttaag 300
   cctgcgtttg acgatactct agaaggcttt gagtatgttc tcgatgcagt aactggtaga 360
   actccaatct ctcagcaatg gattagaaat ttgcacgccg tcattctgcg gagccaagaa 420
   agccacgagg tttttacagc cgttggagtc caaaatcagg cgcttcagaa aggcgagtat 480
   aaaactcagc caaatagtcc acagcgctca gatggatctg tacatgcata cgccccagtt 540
   gaagatactc ctgctgaaat ggctagattt atttcagaac ttgaatctaa ggaattctta 600
   gcagccgaga aggttattca agctgcctat gcccactatg ctttcgtatg tattcatcct 660
   tttgcagatg ggaatggacg agttgcacga gccttggcta gtgtttttct atacaaagat 720
   cctggtgtcc ctctcgtaat ctaccaagat caacgcagag attacatcca tgctctagaa 780
   gcagcggaca agaataaccc gctcctgctg attagattct ttgctgaacg agtgaccgat 840
   actattaact ctattatcgt tgatctcact accccgatcg cgggtaaatc tggttcggct 900
   aagctttcgg atgcgctacg ccccactcgc gtattaccag aattacatga tgctgcacat 960
   aggctccaag aaagtttatt tacagaaatc cgatctcgat tggatgaaga aggaaaaagg 1020
   aatgggttgg agtttctact tcaacggatt tttatcggtt ccccattcaa tctgccagag 1080
   ggctataacg ctttccctga tagctattgt ctgaccttag ctttcaatag caactctcca 1140
   aaacaaatct tccacccgct atccatagta atagcagctc gagatgggaa aagagcgagc 1200
   agcgacctcg tggcagctac ttctattgga tacaactttc acgcttacgg acgtgaagtc 1260
   gagcctgttg ttactgaaag ctttcgagaa cgtgtgaaaa tttacgccga cgggattgta 1320
   gatcacttct taaccgaact ggctaaaaag tttcaacaga attaa 1365
<210> 4
   <211> 454
   <212> PRT
   <213> Corynebacterium glutamicum
<400> 4
   Met Asn Asp Glu Asn Ile Gln Ser Ser Asn Tyr Gln Pro Phe Pro Ser
    1               5                   10                  15
   Phe Asp Asp Trp Lys Gln Ile Glu Val Ser Leu Leu Asp Val Ile Glu
               20                  25                  30
   Ser Ser Arg His Phe Ser Asp Leu Lys Asp Ser Thr Asp Arg Ser Ala
           35                  40                  45
   Leu Asp Ala Ala Leu Glu Arg Ala Lys Arg Ala Ala Ala Val Asp Thr
       50                  55                  60
   Asn Ala Ile Glu Gly Ile Phe Gln Thr Asp Arg Gly Phe Thr His Thr
   65                  70                  75                  80
   Val Ala Thr Gln Val Gly Ala Trp Glu Gln Gln Met Ala Met Lys Gly
                   85                  90                  95
   Lys His Val Lys Pro Ala Phe Asp Asp Thr Leu Glu Gly Phe Glu Tyr
               100                 105                 110
   Val Leu Asp Ala Val Thr Gly Arg Thr Pro Ile Ser Gln Gln Trp Ile
           115                 120                 125
   Arg Asn Leu His Ala Val Ile Leu Arg Ser Gln Glu Ser His Glu Val
       130                 135                 140
   Phe Thr Ala Val Gly Val Gln Asn Gln Ala Leu Gln Lys Gly Glu Tyr
   145                 150                 155                 160
   Lys Thr Gln Pro Asn Ser Pro Gln Arg Ser Asp Gly Ser Val His Ala
                   165                 170                 175
   Tyr Ala Pro Val Glu Asp Thr Pro Ala Glu Met Ala Arg Phe Ile Ser
               180                 185                 190
   Glu Leu Glu Ser Lys Glu Phe Leu Ala Ala Glu Lys Val Ile Gln Ala
           195                 200                 205
   Ala Tyr Ala His Tyr Ala Phe Val Cys Ile His Pro Phe Ala Asp Gly
       210                 215                 220
   Asn Gly Arg Val Ala Arg Ala Leu Ala Ser Val Phe Leu Tyr Lys Asp
   225 230 235 240
   Pro Gly Val Pro Leu Val Ile Tyr Gln Asp Gln Arg Arg Asp Tyr Ile
   245 250 255
   His Ala Leu Glu Ala Ala Asp Lys Asn Asn Pro Leu Leu Leu Ile Arg
   260 265 270
   Phe Phe Ala Glu Arg Val Thr Asp Thr Ile Asn Ser Ile Ile Val Asp
   275 280 285
   Leu Thr Thr Pro Ile Ala Gly Lys Ser Gly Ser Ala Lys Leu Ser Asp
   290 295 300
   Ala Leu Arg Pro Thr Arg Val Leu Pro Glu Leu His Asp Ala Ala His
   305 310 315 320
   Arg Leu Gln Glu Ser Leu Phe Thr Glu Ile Arg Ser Arg Leu Asp Glu
   325 330 335
   Glu Gly Lys Arg Asn Gly Leu Glu Phe Leu Leu Gln Arg Ile Phe Ile
   340 345 350
   Gly Ser Pro Phe Asn Leu Pro Glu Gly Tyr Asn Ala Phe Pro Asp Ser
   355 360 365
   Tyr Cys Leu Thr Leu Ala Phe Asn Ser Asn Ser Pro Lys Gln Ile Phe
   370 375 380
   His Pro Leu Ser Ile Val Ile Ala Ala Arg Asp Gly Lys Arg Ala Ser
   385 390 395 400
   Ser Asp Leu Val Ala Ala Thr Ser Ile Gly Tyr Asn Phe His Ala Tyr
   405 410 415
   Gly Arg Glu Val Glu Pro Val Val Thr Glu Ser Phe Arg Glu Arg Val
   420 425 430
   Lys Ile Tyr Ala Asp Gly Ile Val Asp His Phe Leu Thr Glu Leu Ala
   435 440 445
   Lys Lys Phe Gln Gln Asn
   450
<210> 5
   <211> 52
   <212> DNA
   <213> Corynebacterium glutamicum
<220>
   <223> SEQ_ID_5
<400> 5
   cccgggatcc gctagcggcg cgccggccgg cccggtgtga aataccgcac ag 52
<210> 6
   <211> 53
   <212> DNA
   <213> Corynebacterium glutamicum
<220>
   <223> SEQ_ID_6
<400> 6
   tctagactcg agcggccgcg gccggccttt aaattgaaga cgaaagggcc tcg 53
<210> 7
   <211> 47
   <212> DNA
   <213> Corynebacterium glutamicum
<220>
   <223> SEQ_ID_7
<400> 7
   gagatctaga cccggggatc cgctagcggg ctgctaaagg aagcgga 47
<210> 8
   <211> 38
   <212> DNA
   <213> Corynebacterium glutamicum
<220>
   <223> SEQ_ID_8
<400> 8
   gagaggcgcg ccgctagcgt gggcgaagaa ctccagca 38
<210> 9
   <211> 34
   <212> DNA
   <213> Corynebacterium glutamicum
<220>
   <223> SEQ_ID_9
<400> 9
   gagagggcgg ccgcgcaaag tcccgcttcg tgaa 34
<210> 10
   <211> 34
   <212> DNA
   <213> Corynebacterium glutamicum
<220>
   <223> SEQ_ID_10
<400> 10
   gagagggcgg ccgctcaagt cggtcaagcc acgc 34
<210> 11
   <211> 140
   <212> DNA
   <213> Corynebacterium glutamicum
<220>
   <223> SEQ_ID_11
<400> 11
   tcgaatttaa atctcgagag gcctgacgtc gggcccggta ccacgcgtca tatgactagt 60
   tcggacctag ggatatcgtc gacatcgatg ctcttctgcg ttaattaaca attgggatcc 120
   tctagacccg ggatttaaat 140
<210> 12
   <211> 140
   <212> DNA
   <213> Corynebacterium glutamicum
<220>
   <223> SEQ
<400> 12
   gatcatttaa atcccgggtc tagaggatcc caattgttaa ttaacgcaga agagcatcga 60
   tgtcgacgat atccctaggt ccgaactagt catatgacgc gtggtaccgg gcccgacgtc 120
   aggcctctcg agatttaaat 140
<210> 13
   <211> 5091
   <212> DNA
   <213> Corynebacterium glutamicum
<220>
   <223> PCLIK5MCS
<220>
   <221> misc_feature
   <222> (469)..(4662)
   <223> KanR
<220>
   <221> misc_feature
   <222> (1527)..(2387)
   <223> Ori EC(_{pMB})
<220>
   <221> misc_feature
   <222> (2533)..(3207)
   <223> Orf1
<220>
   <221> misc_feature
   <222> (3541)..(4662)
   <223> Rep Protein
<400> 13
   tcgatttaaa tctcgagagg cctgacgtcg ggcccggtac cacgcgtcat atgactagtt 60
   cggacctagg gatatcgtcg acatcgatgc tcttctgcgt taattaacaa ttgggatcct 120
   ctagacccgg gatttaaatc gctagcgggc tgctaaagga agcggaacac gtagaaagcc 180
   agtccgcaga aacggtgctg accccggatg aatgtcagct actgggctat ctggacaagg 240
   gaaaacgcaa gcgcaaagag aaagcaggta gcttgcagtg ggcttacatg gcgatagcta 300
   gactgggcgg ttttatggac agcaagcgaa ccggaattgc cagctggggc gccctctggt 360
   aaggttggga agccctgcaa agtaaactgg atggctttct tgccgccaag gatctgatgg 420
   cgcaggggat caagatctga tcaagagaca ggatgaggat cgtttcgcat gattgaacaa 480
   gatggattgc acgcaggttc tccggccgct tgggtggaga ggctattcgg ctatgactgg 540
   gcacaacaga caatcggctg ctctgatgcc gccgtgttcc ggctgtcagc gcaggggcgc 600
   ccggttcttt ttgtcaagac cgacctgtcc ggtgccctga atgaactgca ggacgaggca 660
   gcgcggctat cgtggctggc cacgacgggc gttccttgcg cagctgtgct cgacgttgtc 720
   actgaagcgg gaagggactg gctgctattg ggcgaagtgc cggggcagga tctcctgtca 780
   tctcaccttg ctcctgccga gaaagtatcc atcatggctg atgcaatgcg gcggctgcat 840
   acgcttgatc cggctacctg cccattcgac caccaagcga aacatcgcat cgagcgagca 900
   cgtactcgga tggaagccgg tcttgtcgat caggatgatc tggacgaaga gcatcagggg 960
   ctcgcgccag ccgaactgtt cgccaggctc aaggcgcgca tgcccgacgg cgaggatctc 1020
   gtcgtgaccc atggcgatgc ctgcttgccg aatatcatgg tggaaaatgg ccgcttttct 1080
   ggattcatcg actgtggccg gctgggtgtg gcggaccgct atcaggacat agcgttggct 1140
   acccgtgata ttgctgaaga gcttggcggc gaatgggctg accgcttcct cgtgctttac 1200
   ggtatcgccg ctcccgattc gcagcgcatc gccttctatc gccttcttga cgagttcttc 1260
   tgagcgggac tctggggttc gaaatgaccg accaagcgac gcccaacctg ccatcacgag 1320
   atttcgattc caccgccgcc ttctatgaaa ggttgggctt cggaatcgtt ttccgggacg 1380
   ccggctggat gatcctccag cgcggggatc tcatgctgga gttcttcgcc cacgctagcg 1440
   gcgcgccggc cggcccggtg tgaaataccg cacagatgcg taaggagaaa ataccgcatc 1500
   aggcgctctt ccgcttcctc gctcactgac tcgctgcgct cggtcgttcg gctgcggcga 1560
   gcggtatcag ctcactcaaa ggcggtaata cggttatcca cagaatcagg ggataacgca 1620
   ggaaagaaca tgtgagcaaa aggccagcaa aaggccagga accgtaaaaa ggccgcgttg 1680
   ctggcgtttt tccataggct ccgcccccct gacgagcatc acaaaaatcg acgctcaagt 1740
   cagaggtggc gaaacccgac aggactataa agataccagg cgtttccccc tggaagctcc 1800
   ctcgtgcgct ctcctgttcc gaccctgccg cttaccggat acctgtccgc ctttctccct 1860
   tcgggaagcg tggcgctttc tcatagctca cgctgtaggt atctcagttc ggtgtaggtc 1920
   gttcgctcca agctgggctg tgtgcacgaa ccccccgttc agcccgaccg ctgcgcctta 1980
   tccggtaact atcgtcttga gtccaacccg gtaagacacg acttatcgcc actggcagca 2040
   gccactggta acaggattag cagagcgagg tatgtaggcg gtgctacaga gttcttgaag 2100
   tggtggccta actacggcta cactagaagg acagtatttg gtatctgcgc tctgctgaag 2160
   ccagttacct tcggaaaaag agttggtagc tcttgatccg gcaaacaaac caccgctggt 2220
   agcggtggtt tttttgtttg caagcagcag attacgcgca gaaaaaaagg atctcaagaa 2280
   gatcctttga tcttttctac ggggtctgac gctcagtgga acgaaaactc acgttaaggg 2340
   attttggtca tgagattatc aaaaaggatc ttcacctaga tccttttaaa ggccggccgc 2400
   ggccgcgcaa agtcccgctt cgtgaaaatt ttcgtgccgc gtgattttcc gccaaaaact 2460
   ttaacgaacg ttcgttataa tggtgtcatg accttcacga cgaagtacta aaattggccc 2520
   gaatcatcag ctatggatct ctctgatgtc gcgctggagt ccgacgcgct cgatgctgcc 2580
   gtcgatttaa aaacggtgat cggatttttc cgagctctcg atacgacgga cgcgccagca 2640
   tcacgagact gggccagtgc cgcgagcgac ctagaaactc tcgtggcgga tcttgaggag 2700
   ctggctgacg agctgcgtgc tcggccagcg ccaggaggac gcacagtagt ggaggatgca 2760
   atcagttgcg cctactgcgg tggcctgatt cctccccggc ctgacccgcg aggacggcgc 2820
   gcaaaatatt gctcagatgc gtgtcgtgcc gcagccagcc gcgagcgcgc caacaaacgc 2880
   cacgccgagg agctggaggc ggctaggtcg caaatggcgc tggaagtgcg tcccccgagc 2940
   gaaattttgg ccatggtcgt cacagagctg gaagcggcag cgagaattat cgcgatcgtg 3000
   gcggtgcccg caggcatgac aaacatcgta aatgccgcgt ttcgtgtgcc gtggccgccc 3060
   aggacgtgtc agcgccgcca ccacctgcac cgaatcggca gcagcgtcgc gcgtcgaaaa 3120
   agcgcacagg cggcaagaag cgataagctg cacgaatacc tgaaaaatgt tgaacgcccc 3180
   gtgagcggta actcacaggg cgtcggctaa cccccagtcc aaacctggga gaaagcgctc 3240
   aaaaatgact ctagcggatt cacgagacat tgacacaccg gcctggaaat tttccgctga 3300
   tctgttcgac acccatcccg agctcgcgct gcgatcacgt ggctggacga gcgaagaccg 3360
   ccgcgaattc ctcgctcacc tgggcagaga aaatttccag ggcagcaaga cccgcgactt 3420
   cgccagcgct tggatcaaag acccggacac ggagaaacac agccgaagtt ataccgagtt 3480
   ggttcaaaat cgcttgcccg gtgccagtat gttgctctga cgcacgcgca gcacgcagcc 3540
   gtgcttgtcc tggacattga tgtgccgagc caccaggccg gcgggaaaat cgagcacgta 3600
   aaccccgagg tctacgcgat tttggagcgc tgggcacgcc tggaaaaagc gccagcttgg 3660
   atcggcgtga atccactgag cgggaaatgc cagctcatct ggctcattga tccggtgtat 3720
   gccgcagcag gcatgagcag cccgaatatg cgcctgctgg ctgcaacgac cgaggaaatg 3780
   acccgcgttt tcggcgctga ccaggctttt tcacataggc tgagccgtgg ccactgcact 3840
   ctccgacgat cccagccgta ccgctggcat gcccagcaca atcgcgtgga tcgcctagct 3900
   gatcttatgg aggttgctcg catgatctca ggcacagaaa aacctaaaaa acgctatgag 3960
   caggagtttt ctagcggacg ggcacgtatc gaagcggcaa gaaaagccac tgcggaagca 4020
   aaagcacttg ccacgcttga agcaagcctg ccgagcgccg ctgaagcgtc tggagagctg 4080
   atcgacggcg tccgtgtcct ctggactgct ccagggcgtg ccgcccgtga tgagacggct 4140
   tttcgccacg ctttgactgt gggataccag ttaaaagcgg ctggtgagcg cctaaaagac 4200
   accaagggtc atcgagccta cgagcgtgcc tacaccgtcg ctcaggcggt cggaggaggc 4260
   cgtgagcctg atctgccgcc ggactgtgac cgccagacgg attggccgcg acgtgtgcgc 4320
   ggctacgtcg ctaaaggcca gccagtcgtc cctgctcgtc agacagagac gcagagccag 4380
   ccgaggcgaa aagctctggc cactatggga agacgtggcg gtaaaaaggc cgcagaacgc 4440
   tggaaagacc caaacagtga gtacgcccga gcacagcgag aaaaactagc taagtccagt 4500
   caacgacaag ctaggaaagc taaaggaaat cgcttgacca ttgcaggttg gtttatgact 4560
   gttgagggag agactggctc gtggccgaca atcaatgaag ctatgtctga atttagcgtg 4620
   tcacgtcaga ccgtgaatag agcacttaag gtctgcgggc attgaacttc cacgaggacg 4680
   ccgaaagctt cccagtaaat gtgccatctc gtaggcagaa aacggttccc ccgtagggtc 4740
   tctctcttgg cctcctttct aggtcgggct gattgctctt gaagctctct aggggggctc 4800
   acaccatagg cagataacgt tccccaccgg ctcgcctcgt aagcgcacaa ggactgctcc 4860
   caaagatctt caaagccact gccgcgactg ccttcgcgaa gccttgcccc gcggaaattt 4920
   cctccaccga gttcgtgcac acccctatgc caagcttctt tcaccctaaa ttcgagagat 4980
   tggattctta ccgtggaaat tcttcgcaaa aatcgtcccc tgatcgccct tgcgacgttg 5040
   gcgtcggtgc cgctggttgc gcttggcttg accgacttga tcagcggccg c 5091
<210> 14
   <211> 28
   <212> DNA
   <213> Corynebacterium glutamicum
<220>
   <223> SEQ_ID_14
<400> 14
   gcgcggtacc tagactcacc ccagtgct 28
<210> 15
   <211> 30
   <212> DNA
   <213> Corynebacterium glutamicum
<220>
   <223> SEQ_ID_15
<400> 15
   ctctactagt ttagatgtag aactcgatgt 30
<210> 16
   <211> 6349
   <212> DNA
   <213> Corynebacterium glutamicum
<220>
   <223> PCLIK5MCS_PMETA_META
<220>
   <221> misc_feature
   <222> (42)..(177)
   <223> PmetA
<220>
   <221> misc_feature
   <222> (178)..(1311)
   <223> metA
<220>
   <221> misc_feature
   <222> (1727)..(2518)
   <223> KanR
<220>
   <221> misc_feature
   <222> (2785)..(3645)
   <223> Orf1 (complementary)
<220>
   <221> misc_feature
   <222> (3791)..(4465)
   <223> Ori-EC (pMP)
<220>
   <221> misc_feature
   <222> (4799)..(5920)
   <223> Rep Protein
<400> 16
   tcgatttaaa tctcgagagg cctgacgtcg ggcccggtac ctagactcac cccagtgctt 60
   aaagcgctgg gtttttcttt ttcagactcg tgagaatgca aactagacta gacagagctg 120
   tccatataca ctggacgaag ttttagtctt gtccacccag aacaggcggt tattttcatg 180
   cccaccctcg cgccttcagg tcaacttgaa atccaagcga tcggtgatgt ctccaccgaa 240
   gccggagcaa tcattacaaa cgctgaaatc gcctatcacc gctggggtga ataccgcgta 300
   gataaagaag gacgcagcaa tgtcgttctc atcgaacacg ccctcactgg agattccaac 360
   gcagccgatt ggtgggctga cttgctcggt cccggcaaag ccatcaacac tgatatttac 420
   tgcgtgatct gtaccaacgt catcggtggt tgcaacggtt ccaccggacc tggctccatg 480
   catccagatg gaaatttctg gggtaatcgc ttccccgcca cgtccattcg tgatcaggta 540
   aacgccgaaa aacaattcct cgacgcactc ggcatcacca cggtcgccgc agtacttggt 600
   ggttccatgg gtggtgcccg caccctagag tgggccgcaa tgtacccaga aactgttggc 660
   gcagctgctg ttcttgcagt ttctgcacgc gccagcgcct ggcaaatcgg cattcaatcc 720
   gcccaaatta aggcgattga aaacgaccac cactggcacg aaggcaacta ctacgaatcc 780
   ggctgcaacc cagccaccgg actcggcgcc gcccgacgca tcgcccacct cacctaccgt 840
   ggcgaactag aaatcgacga acgcttcggc accaaagccc aaaagaacga aaacccactc 900
   ggtccctacc gcaagcccga ccagcgcttc gccgtggaat cctacttgga ctaccaagca 960
   gacaagctag tacagcgttt cgacgccggc tcctacgtct tgctcaccga cgccctcaac 1020
   cgccacgaca ttggtcgcga ccgcggaggc ctcaacaagg cactcgaatc catcaaagtt 1080
   ccagtccttg tcgcaggcgt agataccgat attttgtacc cctaccacca gcaagaacac 1140
   ctctccagaa acctgggaaa tctactggca atggcaaaaa tcgtatcccc tgtcggccac 1200
   gatgctttcc tcaccgaaag ccgccaaatg gatcgcatcg tgaggaactt cttcagcctc 1260
   atctccccag acgaagacaa cccttcgacc tacatcgagt tctacatcta aactagttcg 1320
   gacctaggga tatcgtcgac atcgatgctc ttctgcgtta attaacaatt gggatcctct 1380
   agacccggga tttaaatcgc tagcgggctg ctaaaggaag cggaacacgt agaaagccag 1440
   tccgcagaaa cggtgctgac cccggatgaa tgtcagctac tgggctatct ggacaaggga 1500
   aaacgcaagc gcaaagagaa agcaggtagc ttgcagtggg cttacatggc gatagctaga 1560
   ctgggcggtt ttatggacag caagcgaacc ggaattgcca gctggggcgc cctctggtaa 1620
   ggttgggaag ccctgcaaag taaactggat ggctttcttg ccgccaagga tctgatggcg 1680
   caggggatca agatctgatc aagagacagg atgaggatcg tttcgcatga ttgaacaaga 1740
   tggattgcac gcaggttctc cggccgcttg ggtggagagg ctattcggct atgactgggc 1800
   acaacagaca atcggctgct ctgatgccgc cgtgttccgg ctgtcagcgc aggggcgccc 1860
   ggttcttttt gtcaagaccg acctgtccgg tgccctgaat gaactgcagg acgaggcagc 1920
   gcggctatcg tggctggcca cgacgggcgt tccttgcgca gctgtgctcg acgttgtcac 1980
   tgaagcggga agggactggc tgctattggg cgaagtgccg gggcaggatc tcctgtcatc 2040
   tcaccttgct cctgccgaga aagtatccat catggctgat gcaatgcggc ggctgcatac 2100
   gcttgatccg gctacctgcc cattcgacca ccaagcgaaa catcgcatcg agcgagcacg 2160
   tactcggatg gaagccggtc ttgtcgatca ggatgatctg gacgaagagc atcaggggct 2220
   cgcgccagcc gaactgttcg ccaggctcaa ggcgcgcatg cccgacggcg aggatctcgt 2280
   cgtgacccat ggcgatgcct gcttgccgaa tatcatggtg gaaaatggcc gcttttctgg 2340
   attcatcgac tgtggccggc tgggtgtggc ggaccgctat caggacatag cgttggctac 2400
   ccgtgatatt gctgaagagc ttggcggcga atgggctgac cgcttcctcg tgctttacgg 2460
   tatcgccgct cccgattcgc agcgcatcgc cttctatcgc cttcttgacg agttcttctg 2520
   agcgggactc tggggttcga aatgaccgac caagcgacgc ccaacctgcc atcacgagat 2580
   ttcgattcca ccgccgcctt ctatgaaagg ttgggcttcg gaatcgtttt ccgggacgcc 2640
   ggctggatga tcctccagcg cggggatctc atgctggagt tcttcgccca cgctagcggc 2700
   gcgccggccg gcccggtgtg aaataccgca cagatgcgta aggagaaaat accgcatcag 2760
   gcgctcttcc gcttcctcgc tcactgactc gctgcgctcg gtcgttcggc tgcggcgagc 2820
   ggtatcagct cactcaaagg cggtaatacg gttatccaca gaatcagggg ataacgcagg 2880
   aaagaacatg tgagcaaaag gccagcaaaa ggccaggaac cgtaaaaagg ccgcgttgct 2940
   ggcgtttttc cataggctcc gcccccctga cgagcatcac aaaaatcgac gctcaagtca 3000
   gaggtggcga aacccgacag gactataaag ataccaggcg tttccccctg gaagctccct 3060
   cgtgcgctct cctgttccga ccctgccgct taccggatac ctgtccgcct ttctcccttc 3120
   gggaagcgtg gcgctttctc atagctcacg ctgtaggtat ctcagttcgg tgtaggtcgt 3180
   tcgctccaag ctgggctgtg tgcacgaacc ccccgttcag cccgaccgct gcgccttatc 3240
   cggtaactat cgtcttgagt ccaacccggt aagacacgac ttatcgccac tggcagcagc 3300
   cactggtaac aggattagca gagcgaggta tgtaggcggt gctacagagt tcttgaagtg 3360
   gtggcctaac tacggctaca ctagaaggac agtatttggt atctgcgctc tgctgaagcc 3420
   agttaccttc ggaaaaagag ttggtagctc ttgatccggc aaacaaacca ccgctggtag 3480
   cggtggtttt tttgtttgca agcagcagat tacgcgcaga aaaaaaggat ctcaagaaga 3540
   tcctttgatc ttttctacgg ggtctgacgc tcagtggaac gaaaactcac gttaagggat 3600
   tttggtcatg agattatcaa aaaggatctt cacctagatc cttttaaagg ccggccgcgg 3660
   ccgcgcaaag tcccgcttcg tgaaaatttt cgtgccgcgt gattttccgc caaaaacttt 3720
   aacgaacgtt cgttataatg gtgtcatgac cttcacgacg aagtactaaa attggcccga 3780
   atcatcagct atggatctct ctgatgtcgc gctggagtcc gacgcgctcg atgctgccgt 3840
   cgatttaaaa acggtgatcg gatttttccg agctctcgat acgacggacg cgccagcatc 3900
   acgagactgg gccagtgccg cgagcgacct agaaactctc gtggcggatc ttgaggagct 3960
   ggctgacgag ctgcgtgctc ggccagcgcc aggaggacgc acagtagtgg aggatgcaat 4020
   cagttgcgcc tactgcggtg gcctgattcc tccccggcct gacccgcgag gacggcgcgc 4080
   aaaatattgc tcagatgcgt gtcgtgccgc agccagccgc gagcgcgcca acaaacgcca 4140
   cgccgaggag ctggaggcgg ctaggtcgca aatggcgctg gaagtgcgtc ccccgagcga 4200
   aattttggcc atggtcgtca cagagctgga agcggcagcg agaattatcg cgatcgtggc 4260
   ggtgcccgca ggcatgacaa acatcgtaaa tgccgcgttt cgtgtgccgt ggccgcccag 4320
   gacgtgtcag cgccgccacc acctgcaccg aatcggcagc agcgtcgcgc gtcgaaaaag 4380
   cgcacaggcg gcaagaagcg ataagctgca cgaatacctg aaaaatgttg aacgccccgt 4440
   gagcggtaac tcacagggcg tcggctaacc cccagtccaa acctgggaga aagcgctcaa 4500
   aaatgactct agcggattca cgagacattg acacaccggc ctggaaattt tccgctgatc 4560
   tgttcgacac ccatcccgag ctcgcgctgc gatcacgtgg ctggacgagc gaagaccgcc 4620
   gcgaattcct cgctcacctg ggcagagaaa atttccaggg cagcaagacc cgcgacttcg 4680
   ccagcgcttg gatcaaagac ccggacacgg agaaacacag ccgaagttat accgagttgg 4740
   ttcaaaatcg cttgcccggt gccagtatgt tgctctgacg cacgcgcagc acgcagccgt 4800
   gcttgtcctg gacattgatg tgccgagcca ccaggccggc gggaaaatcg agcacgtaaa 4860
   ccccgaggtc tacgcgattt tggagcgctg ggcacgcctg gaaaaagcgc cagcttggat 4920
   cggcgtgaat ccactgagcg ggaaatgcca gctcatctgg ctcattgatc cggtgtatgc 4980
   cgcagcaggc atgagcagcc cgaatatgcg cctgctggct gcaacgaccg aggaaatgac 5040
   ccgcgttttc ggcgctgacc aggctttttc acataggctg agccgtggcc actgcactct 5100
   ccgacgatcc cagccgtacc gctggcatgc ccagcacaat cgcgtggatc gcctagctga 5160
   tcttatggag gttgctcgca tgatctcagg cacagaaaaa cctaaaaaac gctatgagca 5220
   ggagttttct agcggacggg cacgtatcga agcggcaaga aaagccactg cggaagcaaa 5280
   agcacttgcc acgcttgaag caagcctgcc gagcgccgct gaagcgtctg gagagctgat 5340
   cgacggcgtc cgtgtcctct ggactgctcc agggcgtgcc gcccgtgatg agacggcttt 5400
   tcgccacgct ttgactgtgg gataccagtt aaaagcggct ggtgagcgcc taaaagacac 5460
   caagggtcat cgagcctacg agcgtgccta caccgtcgct caggcggtcg gaggaggccg 5520
   tgagcctgat ctgccgccgg actgtgaccg ccagacggat tggccgcgac gtgtgcgcgg 5580
   ctacgtcgct aaaggccagc cagtcgtccc tgctcgtcag acagagacgc agagccagcc 5640
   gaggcgaaaa gctctggcca ctatgggaag acgtggcggt aaaaaggccg cagaacgctg 5700
   gaaagaccca aacagtgagt acgcccgagc acagcgagaa aaactagcta agtccagtca 5760
   acgacaagct aggaaagcta aaggaaatcg cttgaccatt gcaggttggt ttatgactgt 5820
   tgagggagag actggctcgt ggccgacaat caatgaagct atgtctgaat ttagcgtgtc 5880
   acgtcagacc gtgaatagag cacttaaggt ctgcgggcat tgaacttcca cgaggacgcc 5940
   gaaagcttcc cagtaaatgt gccatctcgt aggcagaaaa cggttccccc gtagggtctc 6000
   tctcttggcc tcctttctag gtcgggctga ttgctcttga agctctctag gggggctcac 6060
   accataggca gataacgttc cccaccggct cgcctcgtaa gcgcacaagg actgctccca 6120
   aagatcttca aagccactgc cgcgactgcc ttcgcgaagc cttgccccgc ggaaatttcc 6180
   tccaccgagt tcgtgcacac ccctatgcca agcttctttc accctaaatt cgagagattg 6240
   gattcttacc gtggaaattc ttcgcaaaaa tcgtcccctg atcgcccttg cgacgttggc 6300
   gtcggtgccg ctggttgcgc ttggcttgac cgacttgatc agcggccgc 6349
<210> 17
   <211> 29
   <212> DNA
   <213> Corynebacterium glutamicum
<220>
   <223> SEQ_ID_17
<400> 17
   gagactcgag ggccgttacc ctgcgaatg 29
<210> 18
   <211> 40
   <212> DNA
   <213> Corynebacterium glutamicum
<220>
   <223> SEQ_ID_18
<400> 18
   cctgaaggcg cgagggtggg cattgtatgt cctcctggac 40
<210> 19
   <211> 19
   <212> DNA
   <213> Corynebacterium glutamicum
<220>
   <223> SEQ_ID_19
<400> 19
   cccaccctcg cgccttcag 19
<210> 20
   <211> 18
   <212> DNA
   <213> Corynebacterium glutamicum
<220>
   <223> SEQ_ID_20
<400> 20
   ctgggtacat tgcggccc 18
<210> 21
   <211> 6394
   <212> DNA
   <213> Corynebacterium glutamicum
<220>
   <223> PCLIK5MCS
<220>
   <221> misc_feature
   <222> (18)..(216)
   <223> Peftu
<220>
   <221> misc_feature
   <222> (217)..(1350)
   <223> metA
<220>
   <221> misc_feature
   <222> (1772)..(2563)
   <223> KanR
<220>
   <221> misc_feature
   <222> (2830)..(3690)
   <223> Ori-EX (pMB)
<220>
   <221> misc_feature
   <222> (3836)..(4510)
   <223> Orf1
<220>
   <221> misc_feature
   <222> (4844)..(6965)
   <223> Rep Protein
<400> 21
   tcgatttaaa tctcgagggc cgttaccctg cgaatgtcca cagggtagct ggtagtttga 60
   aaatcaacgc cgttgccctt aggattcagt aactggcaca ttttgtaatg cgctagatct 120
   gtgtgctcag tcttccaggc tgcttatcac agtgaaagca aaaccaattc gtggctgcga 180
   aagtcgtagc caccacgaag tccaggagga catacaatgc ccaccctcgc gccttcaggt 240
   caacttgaaa tccaagcgat cggtgatgtc tccaccgaag ccggagcaat cattacaaac 300
   gctgaaatcg cctatcaccg ctggggtgaa taccgcgtag ataaagaagg acgcagcaat 360
   gtcgttctca tcgaacacgc cctcactgga gattccaacg cagccgattg gtgggctgac 420
   ttgctcggtc ccggcaaagc catcaacact gatatttact gcgtgatctg taccaacgtc 480
   atcggtggtt gcaacggttc caccggacct ggctccatgc atccagatgg aaatttctgg 540
   ggtaatcgct tccccgccac gtccattcgt gatcaggtaa acgccgaaaa acaattcctc 600
   gacgcactcg gcatcaccac ggtcgccgca gtacttggtg gttccatggg tggtgcccgc 660
   accctagagt gggccgcaat gtacccagaa actgttggcg cagctgctgt tcttgcagtt 720
   tctgcacgcg ccagcgcctg gcaaatcggc attcaatccg cccaaattaa ggcgattgaa 780
   aacgaccacc actggcacga aggcaactac tacgaatccg gctgcaaccc agccaccgga 840
   ctcggcgccg cccgacgcat cgcccacctc acctaccgtg gcgaactaga aatcgacgaa 900
   cgcttcggca ccaaagccca aaagaacgaa aacccactcg gtccctaccg caagcccgac 960
   cagcgcttcg ccgtggaatc ctacttggac taccaagcag acaagctagt acagcgtttc 1020
   gacgccggct cctacgtctt gctcaccgac gccctcaacc gccacgacat tggtcgcgac 1080
   cgcggaggcc tcaacaaggc actcgaatcc atcaaagttc cagtccttgt cgcaggcgta 1140
   gataccgata ttttgtaccc ctaccaccag caagaacacc tctccagaaa cctgggaaat 1200
   ctactggcaa tggcaaaaat cgtatcccct gtcggccacg atgctttcct caccgaaagc 1260
   cgccaaatgg atcgcatcgt gaggaacttc ttcagcctca tctccccaga cgaagacaac 1320
   ccttcgacct acatcgagtt ctacatctaa catatgacta gttcggacct agggatatcg 1380
   tcgacatcga tgctcttctg cgttaattaa caattgggat cctctagacc cgggatttaa 1440
   atcgctagcg ggctgctaaa ggaagcggaa cacgtagaaa gccagtccgc agaaacggtg 1500
   ctgaccccgg atgaatgtca gctactgggc tatctggaca agggaaaacg caagcgcaaa 1560
   gagaaagcag gtagcttgca gtgggcttac atggcgatag ctagactggg cggttttatg 1620
   gacagcaagc gaaccggaat tgccagctgg ggcgccctct ggtaaggttg ggaagccctg 1680
   caaagtaaac tggatggctt tcttgccgcc aaggatctga tggcgcaggg gatcaagatc 1740
   tgatcaagag acaggatgag gatcgtttcg catgattgaa caagatggat tgcacgcagg 1800
   ttctccggcc gcttgggtgg agaggctatt cggctatgac tgggcacaac agacaatcgg 1860
   ctgctctgat gccgccgtgt tccggctgtc agcgcagggg cgcccggttc tttttgtcaa 1920
   gaccgacctg tccggtgccc tgaatgaact gcaggacgag gcagcgcggc tatcgtggct 1980
   ggccacgacg ggcgttcctt gcgcagctgt gctcgacgtt gtcactgaag cgggaaggga 2040
   ctggctgcta ttgggcgaag tgccggggca ggatctcctg tcatctcacc ttgctcctgc 2100
   cgagaaagta tccatcatgg ctgatgcaat gcggcggctg catacgcttg atccggctac 2160
   ctgcccattc gaccaccaag cgaaacatcg catcgagcga gcacgtactc ggatggaagc 2220
   cggtcttgtc gatcaggatg atctggacga agagcatcag gggctcgcgc cagccgaact 2280
   gttcgccagg ctcaaggcgc gcatgcccga cggcgaggat ctcgtcgtga cccatggcga 2340
   tgcctgcttg ccgaatatca tggtggaaaa tggccgcttt tctggattca tcgactgtgg 2400
   ccggctgggt gtggcggacc gctatcagga catagcgttg gctacccgtg atattgctga 2460
   agagcttggc ggcgaatggg ctgaccgctt cctcgtgctt tacggtatcg ccgctcccga 2520
   ttcgcagcgc atcgccttct atcgccttct tgacgagttc ttctgagcgg gactctgggg 2580
   ttcgaaatga ccgaccaagc gacgcccaac ctgccatcac gagatttcga ttccaccgcc 2640
   gccttctatg aaaggttggg cttcggaatc gttttccggg acgccggctg gatgatcctc 2700
   cagcgcgggg atctcatgct ggagttcttc gcccacgcta gcggcgcgcc ggccggcccg 2760
   gtgtgaaata ccgcacagat gcgtaaggag aaaataccgc atcaggcgct cttccgcttc 2820
   ctcgctcact gactcgctgc gctcggtcgt tcggctgcgg cgagcggtat cagctcactc 2880
   aaaggcggta atacggttat ccacagaatc aggggataac gcaggaaaga acatgtgagc 2940
   aaaaggccag caaaaggcca ggaaccgtaa aaaggccgcg ttgctggcgt ttttccatag 3000
   gctccgcccc cctgacgagc atcacaaaaa tcgacgctca agtcagaggt ggcgaaaccc 3060
   gacaggacta taaagatacc aggcgtttcc ccctggaagc tccctcgtgc gctctcctgt 3120
   tccgaccctg ccgcttaccg gatacctgtc cgcctttctc ccttcgggaa gcgtggcgct 3180
   ttctcatagc tcacgctgta ggtatctcag ttcggtgtag gtcgttcgct ccaagctggg 3240
   ctgtgtgcac gaaccccccg ttcagcccga ccgctgcgcc ttatccggta actatcgtct 3300
   tgagtccaac ccggtaagac acgacttatc gccactggca gcagccactg gtaacaggat 3360
   tagcagagcg aggtatgtag gcggtgctac agagttcttg aagtggtggc ctaactacgg 3420
   ctacactaga aggacagtat ttggtatctg cgctctgctg aagccagtta ccttcggaaa 3480
   aagagttggt agctcttgat ccggcaaaca aaccaccgct ggtagcggtg gtttttttgt 3540
   ttgcaagcag cagattacgc gcagaaaaaa aggatctcaa gaagatcctt tgatcttttc 3600
   tacggggtct gacgctcagt ggaacgaaaa ctcacgttaa gggattttgg tcatgagatt 3660
   atcaaaaagg atcttcacct agatcctttt aaaggccggc cgcggccgcg caaagtcccg 3720
   cttcgtgaaa attttcgtgc cgcgtgattt tccgccaaaa actttaacga acgttcgtta 3780
   taatggtgtc atgaccttca cgacgaagta ctaaaattgg cccgaatcat cagctatgga 3840
   tctctctgat gtcgcgctgg agtccgacgc gctcgatgct gccgtcgatt taaaaacggt 3900
   gatcggattt ttccgagctc tcgatacgac ggacgcgcca gcatcacgag actgggccag 3960
   tgccgcgagc gacctagaaa ctctcgtggc ggatcttgag gagctggctg acgagctgcg 4020
   tgctcggcca gcgccaggag gacgcacagt agtggaggat gcaatcagtt gcgcctactg 4080
   cggtggcctg attcctcccc ggcctgaccc gcgaggacgg cgcgcaaaat attgctcaga 4140
   tgcgtgtcgt gccgcagcca gccgcgagcg cgccaacaaa cgccacgccg aggagctgga 4200
   ggcggctagg tcgcaaatgg cgctggaagt gcgtcccccg agcgaaattt tggccatggt 4260
   cgtcacagag ctggaagcgg cagcgagaat tatcgcgatc gtggcggtgc ccgcaggcat 4320
   gacaaacatc gtaaatgccg cgtttcgtgt gccgtggccg cccaggacgt gtcagcgccg 4380
   ccaccacctg caccgaatcg gcagcagcgt cgcgcgtcga aaaagcgcac aggcggcaag 4440
   aagcgataag ctgcacgaat acctgaaaaa tgttgaacgc cccgtgagcg gtaactcaca 4500
   gggcgtcggc taacccccag tccaaacctg ggagaaagcg ctcaaaaatg actctagcgg 4560
   attcacgaga cattgacaca ccggcctgga aattttccgc tgatctgttc gacacccatc 4620
   ccgagctcgc gctgcgatca cgtggctgga cgagcgaaga ccgccgcgaa ttcctcgctc 4680
   acctgggcag agaaaatttc cagggcagca agacccgcga cttcgccagc gcttggatca 4740
   aagacccgga cacggagaaa cacagccgaa gttataccga gttggttcaa aatcgcttgc 4800
   ccggtgccag tatgttgctc tgacgcacgc gcagcacgca gccgtgcttg tcctggacat 4860
   tgatgtgccg agccaccagg ccggcgggaa aatcgagcac gtaaaccccg aggtctacgc 4920
   gattttggag cgctgggcac gcctggaaaa agcgccagct tggatcggcg tgaatccact 4980
   gagcgggaaa tgccagctca tctggctcat tgatccggtg tatgccgcag caggcatgag 5040
   cagcccgaat atgcgcctgc tggctgcaac gaccgaggaa atgacccgcg ttttcggcgc 5100
   tgaccaggct ttttcacata ggctgagccg tggccactgc actctccgac gatcccagcc 5160
   gtaccgctgg catgcccagc acaatcgcgt ggatcgccta gctgatctta tggaggttgc 5220
   tcgcatgatc tcaggcacag aaaaacctaa aaaacgctat gagcaggagt tttctagcgg 5280
   acgggcacgt atcgaagcgg caagaaaagc cactgcggaa gcaaaagcac ttgccacgct 5340
   tgaagcaagc ctgccgagcg ccgctgaagc gtctggagag ctgatcgacg gcgtccgtgt 5400
   cctctggact gctccagggc gtgccgcccg tgatgagacg gcttttcgcc acgctttgac 5460
   tgtgggatac cagttaaaag cggctggtga gcgcctaaaa gacaccaagg gtcatcgagc 5520
   ctacgagcgt gcctacaccg tcgctcaggc ggtcggagga ggccgtgagc ctgatctgcc 5580
   gccggactgt gaccgccaga cggattggcc gcgacgtgtg cgcggctacg tcgctaaagg 5640
   ccagccagtc gtccctgctc gtcagacaga gacgcagagc cagccgaggc gaaaagctct 5700
   ggccactatg ggaagacgtg gcggtaaaaa ggccgcagaa cgctggaaag acccaaacag 5760
   tgagtacgcc cgagcacagc gagaaaaact agctaagtcc agtcaacgac aagctaggaa 5820
   agctaaagga aatcgcttga ccattgcagg ttggtttatg actgttgagg gagagactgg 5880
   ctcgtggccg acaatcaatg aagctatgtc tgaatttagc gtgtcacgtc agaccgtgaa 5940
   tagagcactt aaggtctgcg ggcattgaac ttccacgagg acgccgaaag cttcccagta 6000
   aatgtgccat ctcgtaggca gaaaacggtt cccccgtagg gtctctctct tggcctcctt 6060
   tctaggtcgg gctgattgct cttgaagctc tctagggggg ctcacaccat aggcagataa 6120
   cgttccccac cggctcgcct cgtaagcgca caaggactgc tcccaaagat cttcaaagcc 6180
   actgccgcga ctgccttcgc gaagccttgc cccgcggaaa tttcctccac cgagttcgtg 6240
   cacaccccta tgccaagctt ctttcaccct aaattcgaga gattggattc ttaccgtgga 6300
   aattcttcgc aaaaatcgtc ccctgatcgc ccttgcgacg ttggcgtcgg tgccgctggt 6360
   tgcgcttggc ttgaccgact tgatcagcgg ccgc 6394
<210> 22
   <211> 35
   <212> DNA
   <213> Corynebacterium glutamicum
<220>
   <223> SEQ_ID_22
<400> 22
   gagagagaga cgcgtcccag tggctgagac gcatc 35
<210> 23
   <211> 34
   <212> DNA
   <213> Corynebacterium glutamicum
<220>
   <223> SEQ_ID_23
<400> 23
   ctctctctgt cgacgaattc aatcttacgg cctg 34
<210> 24
   <211> 4323
   <212> DNA
   <213> Corynebacterium glutamicum
<220>
   <223> PCLIK5
<400> 24
   tcgagaggcc tgacgtcggg cccggtacca cgcgtcatat gactagttcg gacctaggga 60
   tatcgtcgac atcgatgctc ttctgcgtta attaacaatt gggatcctct agacccggga 120
   tttaaatcgc tagcgggctg ctaaaggaag cggaacacgt agaaagccag tccgcagaaa 180
   cggtgctgac cccggatgaa tgtcagctac tgggctatct ggacaaggga aaacgcaagc 240
   gcaaagagaa agcaggtagc ttgcagtggg cttacatggc gatagctaga ctgggcggtt 300
   ttatggacag caagcgaacc ggaattgcca gctggggcgc cctctggtaa ggttgggaag 360
   ccctgcaaag taaactggat ggctttcttg ccgccaagga tctgatggcg caggggatca 420
   agatctgatc aagagacagg atgaggatcg tttcgcatga ttgaacaaga tggattgcac 480
   gcaggttctc cggccgcttg ggtggagagg ctattcggct atgactgggc acaacagaca 540
   atcggctgct ctgatgccgc cgtgttccgg ctgtcagcgc aggggcgccc ggttcttttt 600
   gtcaagaccg acctgtccgg tgccctgaat gaactgcagg acgaggcagc gcggctatcg 660
   tggctggcca cgacgggcgt tccttgcgca gctgtgctcg acgttgtcac tgaagcggga 720
   agggactggc tgctattggg cgaagtgccg gggcaggatc tcctgtcatc tcaccttgct 780
   cctgccgaga aagtatccat catggctgat gcaatgcggc ggctgcatac gcttgatccg 840
   gctacctgcc cattcgacca ccaagcgaaa catcgcatcg agcgagcacg tactcggatg 900
   gaagccggtc ttgtcgatca ggatgatctg gacgaagagc atcaggggct cgcgccagcc 960
   gaactgttcg ccaggctcaa ggcgcgcatg cccgacggcg aggatctcgt cgtgacccat 1020
   ggcgatgcct gcttgccgaa tatcatggtg gaaaatggcc gcttttctgg attcatcgac 1080
   tgtggccggc tgggtgtggc ggaccgctat caggacatag cgttggctac ccgtgatatt 1140
   gctgaagagc ttggcggcga atgggctgac cgcttcctcg tgctttacgg tatcgccgct 1200
   cccgattcgc agcgcatcgc cttctatcgc cttcttgacg agttcttctg agcgggactc 1260
   tggggttcga aatgaccgac caagcgacgc ccaacctgcc atcacgagat ttcgattcca 1320
   ccgccgcctt ctatgaaagg ttgggcttcg gaatcgtttt ccgggacgcc ggctggatga 1380
   tcctccagcg cggggatctc atgctggagt tcttcgccca cgctagcggc gcgccggccg 1440
   gcccggtgtg aaataccgca cagatgcgta aggagaaaat accgcatcag gcgctcttcc 1500
   gcttcctcgc tcactgactc gctgcgctcg gtcgttcggc tgcggcgagc ggtatcagct 1560
   cactcaaagg cggtaatacg gttatccaca gaatcagggg ataacgcagg aaagaacatg 1620
   tgagcaaaag gccagcaaaa ggccaggaac cgtaaaaagg ccgcgttgct ggcgtttttc 1680
   cataggctcc gcccccctga cgagcatcac aaaaatcgac gctcaagtca gaggtggcga 1740
   aacccgacag gactataaag ataccaggcg tttccccctg gaagctccct cgtgcgctct 1800
   cctgttccga ccctgccgct taccggatac ctgtccgcct ttctcccttc gggaagcgtg 1860
   gcgctttctc atagctcacg ctgtaggtat ctcagttcgg tgtaggtcgt tcgctccaag 1920
   ctgggctgtg tgcacgaacc ccccgttcag cccgaccgct gcgccttatc cggtaactat 1980
   cgtcttgagt ccaacccggt aagacacgac ttatcgccac tggcagcagc cactggtaac 2040
   aggattagca gagcgaggta tgtaggcggt gctacagagt tcttgaagtg gtggcctaac 2100
   tacggctaca ctagaaggac agtatttggt atctgcgctc tgctgaagcc agttaccttc 2160
   ggaaaaagag ttggtagctc ttgatccggc aaacaaacca ccgctggtag cggtggtttt 2220
   tttgtttgca agcagcagat tacgcgcaga aaaaaaggat ctcaagaaga tcctttgatc 2280
   ttttctacgg ggtctgacgc tcagtggaac gaaaactcac gttaagggat tttggtcatg 2340
   agattatcaa aaaggatctt cacctagatc cttttaaagg ccggccgcgg ccgccatcgg 2400
   cattttcttt tgcgttttta tttgttaact gttaattgtc cttgttcaag gatgctgtct 2460
   ttgacaacag atgttttctt gcctttgatg ttcagcagga agctcggcgc aaacgttgat 2520
   tgtttgtctg cgtagaatcc tctgtttgtc atatagcttg taatcacgac attgtttcct 2580
   ttcgcttgag gtacagcgaa gtgtgagtaa gtaaaggtta catcgttagg atcaagatcc 2640
   atttttaaca caaggccagt tttgttcagc ggcttgtatg ggccagttaa agaattagaa 2700
   acataaccaa gcatgtaaat atcgttagac gtaatgccgt caatcgtcat ttttgatccg 2760
   cgggagtcag tgaacaggta ccatttgccg ttcattttaa agacgttcgc gcgttcaatt 2820
   tcatctgtta ctgtgttaga tgcaatcagc ggtttcatca cttttttcag tgtgtaatca 2880
   tcgtttagct caatcatacc gagagcgccg tttgctaact cagccgtgcg ttttttatcg 2940
   ctttgcagaa gtttttgact ttcttgacgg aagaatgatg tgcttttgcc atagtatgct 3000
   ttgttaaata aagattcttc gccttggtag ccatcttcag ttccagtgtt tgcttcaaat 3060
   actaagtatt tgtggccttt atcttctacg tagtgaggat ctctcagcgt atggttgtcg 3120
   cctgagctgt agttgccttc atcgatgaac tgctgtacat tttgatacgt ttttccgtca 3180
   ccgtcaaaga ttgatttata atcctctaca ccgttgatgt tcaaagagct gtctgatgct 3240
   gatacgttaa cttgtgcagt tgtcagtgtt tgtttgccgt aatgtttacc ggagaaatca 3300
   gtgtagaata aacggatttt tccgtcagat gtaaatgtgg ctgaacctga ccattcttgt 3360
   gtttggtctt ttaggataga atcatttgca tcgaatttgt cgctgtcttt aaagacgcgg 3420
   ccagcgtttt tccagctgtc aatagaagtt tcgccgactt tttgatagaa catgtaaatc 3480
   gatgtgtcat ccgcattttt aggatctccg gctaatgcaa agacgatgtg gtagccgtga 3540
   tagtttgcga cagtgccgtc agcgttttgt aatggccagc tgtcccaaac gtccaggcct 3600
   tttgcagaag agatattttt aattgtggac gaatcaaatt cagaaacttg atatttttca 3660
   tttttttgct gttcagggat ttgcagcata tcatggcgtg taatatggga aatgccgtat 3720
   gtttccttat atggcttttg gttcgtttct ttcgcaaacg cttgagttgc gcctcctgcc 3780
   agcagtgcgg tagtaaaggt taatactgtt gcttgttttg caaacttttt gatgttcatc 3840
   gttcatgtct ccttttttat gtactgtgtt agcggtctgc ttcttccagc cctcctgttt 3900
   gaagatggca agttagttac gcacaataaa aaaagaccta aaatatgtaa ggggtgacgc 3960
   caaagtatac actttgccct ttacacattt taggtcttgc ctgctttatc agtaacaaac 4020
   ccgcgcgatt tacttttcga cctcattcta ttagactctc gtttggattg caactggtct 4080
   attttcctct tttgtttgat agaaaatcat aaaaggattt gcagactacg ggcctaaaga 4140
   actaaaaaat ctatctgttt cttttcattc tctgtatttt ttatagtttc tgttgcatgg 4200
   gcataaagtt gcctttttaa tcacaattca gaaaatatca taatatctca tttcactaaa 4260
   taatagtgaa cggcaggtat atgtgatggg ttaaaaagga tcggcggccg ctcgatttaa 4320
   atc                                                               4323
<210> 25
   <211> 5860
   <212> DNA
   <213> Corynebacterium glutamicum
<220>
   <223> PCIS_LYSC
<220>
   <221> misc_feature
   <222> (155)..(1420)
   <223> CDS lysC
<220>
   <221> misc_feature
   <222> (1974)..(2765)
   <223> KanR
<220>
   <221> misc_feature
   <222> (3032)..(3892)
   <223> Ori-EC (pMP)
<220>
   <221> C_region
   <222> (3913)..(3934)
   <223> sacB downstream (complement)
<220>
   <221> misc_feature
   <222> (3935)..(5356)
   <223> CDS: sacB complement; (Bacillus subtilis)
<220>
   <221> promoter
   <222> (5357)..(5819)
   <223> Promotor sacB (complement)
<400> 25
   cccggtacca cgcgtcccag tggctgagac gcatccgcta aagccccagg aaccctgtgc 60
   agaaagaaaa cactcctctg gctaggtaga cacagtttat aaaggtagag ttgagcgggt 120
   aactgtcagc acgtagatcg aaaggtgcac aaaggtggcc ctggtcgtac agaaatatgg 180
   cggttcctcg cttgagagtg cggaacgcat tagaaacgtc gctgaacgga tcgttgccac 240
   caagaaggct ggaaatgatg tcgtggttgt ctgctccgca atgggagaca ccacggatga 300
   acttctagaa cttgcagcgg cagtgaatcc cgttccgcca gctcgtgaaa tggatatgct 360
   cctgactgct ggtgagcgta tttctaacgc tctcgtcgcc atggctattg agtcccttgg 420
   cgcagaagcc caatctttca cgggctctca ggctggtgtg ctcaccaccg agcgccacgg 480
   aaacgcacgc attgttgatg tcactccagg tcgtgtgcgt gaagcactcg atgagggcaa 540
   gatctgcatt gttgctggtt tccagggtgt taataaagaa acccgcgatg tcaccacgtt 600
   gggtcgtggt ggttctgaca ccactgcagt tgcgttggca gctgctttga acgctgatgt 660
   gtgtgagatt tactcggacg ttgacggtgt gtataccgct gacccgcgca tcgttcctaa 720
   tgcacagaag ctggaaaagc tcagcttcga agaaatgctg gaacttgctg ctgttggctc 780
   caagattttg gtgctgcgca gtgttgaata cgctcgtgca ttcaatgtgc cacttcgcgt 840
   acgctcgtct tatagtaatg atcccggcac tttgattgcc ggctctatgg aggatattcc 900
   tgtggaagaa gcagtcctta ccggtgtcgc aaccgacaag tccgaagcca aagtaaccgt 960
   tctgggtatt tccgataagc caggcgaggc tgcgaaggtt ttccgtgcgt tggctgatgc 1020
   agaaatcaac attgacatgg ttctgcagaa cgtctcttct gtagaagacg gcaccaccga 1080
   catcaccttc acctgccctc gttccgacgg ccgccgcgcg atggagatct tgaagaagct 1140
   tcaggttcag ggcaactgga ccaatgtgct ttacgacgac caggtcggca aagtctccct 1200
   cgtgggtgct ggcatgaagt ctcacccagg tgttaccgca gagttcatgg aagctctgcg 1260
   cgatgtcaac gtgaacatcg aattgatttc cacctctgag attcgtattt ccgtgctgat 1320
   ccgtgaagat gatctggatg ctgctgcacg tgcattgcat gagcagttcc agctgggcgg 1380
   cgaagacgaa gccgtcgttt atgcaggcac cggacgctaa agttttaaag gagtagtttt 1440
   acaatgacca ccatcgcagt tgttggtgca accggccagg tcggccaggt tatgcgcacc 1500
   cttttggaag agcgcaattt cccagctgac actgttcgtt tctttgcttc cccacgttcc 1560
   gcaggccgta agattgaatt cgtcgacatc gatgctcttc tgcgttaatt aacaattggg 1620
   atcctctaga cccgggattt aaatcgctag cgggctgcta aaggaagcgg aacacgtaga 1680
   aagccagtcc gcagaaacgg tgctgacccc ggatgaatgt cagctactgg gctatctgga 1740
   caagggaaaa cgcaagcgca aagagaaagc aggtagcttg cagtgggctt acatggcgat 1800
   agctagactg ggcggtttta tggacagcaa gcgaaccgga attgccagct ggggcgccct 1860
   ctggtaaggt tgggaagccc tgcaaagtaa actggatggc tttcttgccg ccaaggatct 1920
   gatggcgcag gggatcaaga tctgatcaag agacaggatg aggatcgttt cgcatgattg 1980
   aacaagatgg attgcacgca ggttctccgg ccgcttgggt ggagaggcta ttcggctatg 2040
   actgggcaca acagacaatc ggctgctctg atgccgccgt gttccggctg tcagcgcagg 2100
   ggcgcccggt tctttttgtc aagaccgacc tgtccggtgc cctgaatgaa ctgcaggacg 2160
   aggcagcgcg gctatcgtgg ctggccacga cgggcgttcc ttgcgcagct gtgctcgacg 2220
   ttgtcactga agcgggaagg gactggctgc tattgggcga agtgccgggg caggatctcc 2280
   tgtcatctca ccttgctcct gccgagaaag tatccatcat ggctgatgca atgcggcggc 2340
   tgcatacgct tgatccggct acctgcccat tcgaccacca agcgaaacat cgcatcgagc 2400
   gagcacgtac tcggatggaa gccggtcttg tcgatcagga tgatctggac gaagagcatc 2460
   aggggctcgc gccagccgaa ctgttcgcca ggctcaaggc gcgcatgccc gacggcgagg 2520
   atctcgtcgt gacccatggc gatgcctgct tgccgaatat catggtggaa aatggccgct 2580
   tttctggatt catcgactgt ggccggctgg gtgtggcgga ccgctatcag gacatagcgt 2640
   tggctacccg tgatattgct gaagagcttg gcggcgaatg ggctgaccgc ttcctcgtgc 2700
   tttacggtat cgccgctccc gattcgcagc gcatcgcctt ctatcgcctt cttgacgagt 2760
   tcttctgagc gggactctgg ggttcgaaat gaccgaccaa gcgacgccca acctgccatc 2820
   acgagatttc gattccaccg ccgccttcta tgaaaggttg ggcttcggaa tcgttttccg 2880
   ggacgccggc tggatgatcc tccagcgcgg ggatctcatg ctggagttct tcgcccacgc 2940
   tagcggcgcg ccggccggcc cggtgtgaaa taccgcacag atgcgtaagg agaaaatacc 3000
   gcatcaggcg ctcttccgct tcctcgctca ctgactcgct gcgctcggtc gttcggctgc 3060
   ggcgagcggt atcagctcac tcaaaggcgg taatacggtt atccacagaa tcaggggata 3120
   acgcaggaaa gaacatgtga gcaaaaggcc agcaaaaggc caggaaccgt aaaaaggccg 3180
   cgttgctggc gtttttccat aggctccgcc cccctgacga gcatcacaaa aatcgacgct 3240
   caagtcagag gtggcgaaac ccgacaggac tataaagata ccaggcgttt ccccctggaa 3300
   gctccctcgt gcgctctcct gttccgaccc tgccgcttac cggatacctg tccgcctttc 3360
   tcccttcggg aagcgtggcg ctttctcata gctcacgctg taggtatctc agttcggtgt 3420
   aggtcgttcg ctccaagctg ggctgtgtgc acgaaccccc cgttcagccc gaccgctgcg 3480
   ccttatccgg taactatcgt cttgagtcca acccggtaag acacgactta tcgccactgg 3540
   cagcagccac tggtaacagg attagcagag cgaggtatgt aggcggtgct acagagttct 3600
   tgaagtggtg gcctaactac ggctacacta gaaggacagt atttggtatc tgcgctctgc 3660
   tgaagccagt taccttcgga aaaagagttg gtagctcttg atccggcaaa caaaccaccg 3720
   ctggtagcgg tggttttttt gtttgcaagc agcagattac gcgcagaaaa aaaggatctc 3780
   aagaagatcc tttgatcttt tctacggggt ctgacgctca gtggaacgaa aactcacgtt 3840
   aagggatttt ggtcatgaga ttatcaaaaa ggatcttcac ctagatcctt ttaaaggccg 3900
   gccgcggccg ccatcggcat tttcttttgc gtttttattt gttaactgtt aattgtcctt 3960
   gttcaaggat gctgtctttg acaacagatg ttttcttgcc tttgatgttc agcaggaagc 4020
   tcggcgcaaa cgttgattgt ttgtctgcgt agaatcctct gtttgtcata tagcttgtaa 4080
   tcacgacatt gtttcctttc gcttgaggta cagcgaagtg tgagtaagta aaggttacat 4140
   cgttaggatc aagatccatt tttaacacaa ggccagtttt gttcagcggc ttgtatgggc 4200
   cagttaaaga attagaaaca taaccaagca tgtaaatatc gttagacgta atgccgtcaa 4260
   tcgtcatttt tgatccgcgg gagtcagtga acaggtacca tttgccgttc attttaaaga 4320
   cgttcgcgcg ttcaatttca tctgttactg tgttagatgc aatcagcggt ttcatcactt 4380
   ttttcagtgt gtaatcatcg tttagctcaa tcataccgag agcgccgttt gctaactcag 4440
   ccgtgcgttt tttatcgctt tgcagaagtt tttgactttc ttgacggaag aatgatgtgc 4500
   ttttgccata gtatgctttg ttaaataaag attcttcgcc ttggtagcca tcttcagttc 4560
   cagtgtttgc ttcaaatact aagtatttgt ggcctttatc ttctacgtag tgaggatctc 4620
   tcagcgtatg gttgtcgcct gagctgtagt tgccttcatc gatgaactgc tgtacatttt 4680
   gatacgtttt tccgtcaccg tcaaagattg atttataatc ctctacaccg ttgatgttca 4740
   aagagctgtc tgatgctgat acgttaactt gtgcagttgt cagtgtttgt ttgccgtaat 4800
   gtttaccgga gaaatcagtg tagaataaac ggatttttcc gtcagatgta aatgtggctg 4860
   aacctgacca ttcttgtgtt tggtctttta ggatagaatc atttgcatcg aatttgtcgc 4920
   tgtctttaaa gacgcggcca gcgtttttcc agctgtcaat agaagtttcg ccgacttttt 4980
   gatagaacat gtaaatcgat gtgtcatccg catttttagg atctccggct aatgcaaaga 5040
   cgatgtggta gccgtgatag tttgcgacag tgccgtcagc gttttgtaat ggccagctgt 5100
   cccaaacgtc caggcctttt gcagaagaga tatttttaat tgtggacgaa tcaaattcag 5160
   aaacttgata tttttcattt ttttgctgtt cagggatttg cagcatatca tggcgtgtaa 5220
   tatgggaaat gccgtatgtt tccttatatg gcttttggtt cgtttctttc gcaaacgctt 5280
   gagttgcgcc tcctgccagc agtgcggtag taaaggttaa tactgttgct tgttttgcaa 5340
   actttttgat gttcatcgtt catgtctcct tttttatgta ctgtgttagc ggtctgcttc 5400
   ttccagccct cctgtttgaa gatggcaagt tagttacgca caataaaaaa agacctaaaa 5460
   tatgtaaggg gtgacgccaa agtatacact ttgcccttta cacattttag gtcttgcctg 5520
   ctttatcagt aacaaacccg cgcgatttac ttttcgacct cattctatta gactctcgtt 5580
   tggattgcaa ctggtctatt ttcctctttt gtttgataga aaatcataaa aggatttgca 5640
   gactacgggc ctaaagaact aaaaaatcta tctgtttctt ttcattctct gtatttttta 5700
   tagtttctgt tgcatgggca taaagttgcc tttttaatca caattcagaa aatatcataa 5760
   tatctcattt cactaaataa tagtgaacgg caggtatatg tgatgggtta aaaaggatcg 5820
   gcggccgctc gatttaaatc tcgagaggcc tgacgtcggg 5860
<210> 26
   <211> 38
   <212> DNA
   <213> Corynebacterium glutamicum
<220>
   <223> SEQ_ID_26
<400> 26
   cggcaccacc gacatcatct tcacctgccc tcgttccg 38
<210> 27
   <211> 38
   <212> DNA
   <213> Corynebacterium glutamicum
<220>
   <223> SEQ_ID_27
<400> 27
   cggaacgagg gcaggtgaag atgatgtcgg tggtgccg 38
<210> 28
   <211> 1263
   <212> DNA
   <213> Corynebacterium glutamicum
<220>
   <223> LYSC
<400> 28
   gtggccctgg tcgtacagaa atatggcggt tcctcgcttg agagtgcgga acgcattaga 60
   aacgtcgctg aacggatcgt tgccaccaag aaggctggaa atgatgtcgt ggttgtctgc 120
   tccgcaatgg gagacaccac ggatgaactt ctagaacttg cagcggcagt gaatcccgtt 180
   ccgccagctc gtgaaatgga tatgctcctg actgctggtg agcgtatttc taacgctctc 240
   gtcgccatgg ctattgagtc ccttggcgca gaagcccaat ctttcacggg ctctcaggct 300
   ggtgtgctca ccaccgagcg ccacggaaac gcacgcattg ttgatgtcac tccaggtcgt 360
   gtgcgtgaag cactcgatga gggcaagatc tgcattgttg ctggtttcca gggtgttaat 420
   aaagaaaccc gcgatgtcac cacgttgggt cgtggtggtt ctgacaccac tgcagttgcg 480
   ttggcagctg ctttgaacgc tgatgtgtgt gagatttact cggacgttga cggtgtgtat 540
   accgctgacc cgcgcatcgt tcctaatgca cagaagctgg aaaagctcag cttcgaagaa 600
   atgctggaac ttgctgctgt tggctccaag attttggtgc tgcgcagtgt tgaatacgct 660
   cgtgcattca atgtgccact tcgcgtacgc tcgtcttata gtaatgatcc cggcactttg 720
   attgccggct ctatggagga tattcctgtg gaagaagcag tccttaccgg tgtcgcaacc 780
   gacaagtccg aagccaaagt aaccgttctg ggtatttccg ataagccagg cgaggctgcg 840
   aaggttttcc gtgcgttggc tgatgcagaa atcaacattg acatggttct gcagaacgtc 900
   tcttctgtag aagacggcac caccgacatc accttcacct gccctcgttc cgacggccgc 960
   cgcgcgatgg agatcttgaa gaagcttcag gttcagggca actggaccaa tgtgctttac 1020
   gacgaccagg tcggcaaagt ctccctcgtg ggtgctggca tgaagtctca cccaggtgtt 1080
   accgcagagt tcatggaagc tctgcgcgat gtcaacgtga acatcgaatt gatttccacc 1140
   tctgagattc gtatttccgt gctgatccgt gaagatgatc tggatgctgc tgcacgtgca 1200
   ttgcatgagc agttccagct gggcggcgaa gacgaagccg tcgtttatgc aggcaccgga 1260
   cgc                                                               1263
<210> 29
   <211> 5860
   <212> DNA
   <213> Corynebacterium glutamicum
<220>
   <223> PCIS\LYSC\THR311ILE
<220>
   <221> misc_feature
   <222> (155)..(1420)
   <223> lysC
<220>
   <221> misc_feature
   <222> (1974)..(2765)
   <223> CDS: KanR
<220>
   <221> misc_feature
   <222> (3032)..(3892)
   <223> Ori-EC (pMP) complement
<220>
   <221> C_region
   <222> (3913)..(3934)
   <223> sacB downstream (complement)
<220>
   <221> misc_feature
   <222> (3935)..(5356)
   <223> CDS: sacB (complement)
<220>
   <221> promoter
   <222> (5357)..(5819)
   <223> sacB Promotor (complement)
<400> 29
<210> 30
   <211> 29
   <212> DNA
   <213> Corynebacterium glutamicum
<220>
   <223> CK_352
<400> 30
   cgccaattgt ggccgttacc ctgcgaatg 29
<210> 31
   <211> 40
   <212> DNA
   <213> Corynebacterium glutamicum
<220>
   <223> CK_353
<400> 31
   ttctgtacga ccagggccac tgtatgtcct cctggacttc 40
<210> 32
   <211> 40
   <212> DNA
   <213> Corynebacterium glutamicum
<220>
   <223> CK_354
<400> 32
   gaagtccagg aggacataca gtggccctgg tcgtacagaa 40
<210> 33
   <211> 30
   <212> DNA
   <213> Corynebacterium glutamicum
<220>
   <223> CK_355
<400> 33
   catgcccggg acagcagcaa gttccagcat 30
<210> 34
   <211> 29
   <212> DNA
   <213> Corynebacterium glutamicum
<220>
   <223> CK_356
<400> 34
   cgcgacgtcc gtcccaaaac gatcatgat 29
<210> 35
   <211> 29
   <212> DNA
   <213> Corynebacterium glutamicum
<220>
   <223> CK_357
<400> 35
   cgccaattgc tttgtgcacc tttcgatct 29
<210> 36
   <211> 5670
   <212> DNA
   <213> Corynebacterium glutamicum
<220>
   <223> PCIS
<220>
   <221> misc_feature
   <222> (18)..(633)
   <223> 5' ask
<220>
   <221> misc_feature
   <222> (641)..(840)
   <223> Peftu
<220>
   <221> misc_feature
   <222> (841)..(1460)
   <223> part of ask
<220>
   <221> misc_feature
   <222> (1804)..(2595)
   <223> KanR
<220>
   <221> misc_feature
   <222> (2862)..(3722)
   <223> ori-EC (pMP) complement
<220>
   <221> misc_feature
   <222> (3765)..(5186)
   <223> sacB (complement)
<220>
   <221> misc_feature
   <222> (5187)..(5649)
   <223> PSCB (complement)
<400> 36
   tcgagaggcc tgacgtccgt cccaaaacga tcatgatgcc cacggctacg gtgaggaggg 60
   tagcccagaa gatttcagtt cggcgtagtc ggtagccatt gaatcgtgct gagagcggca 120
   gcgtgaacat cagcgacagg acaagcactg gttgcactac caagagggtg ccgaaaccaa 180
   gtgctactgt ttgtaagaaa tatgccagca tcgcggtact catgcctgcc caccacatcg 240
   gtgtcatcag agcattgagt aaaggtgagc tccttaggga gccatctttt ggggtgcgga 300
   gcgcgatccg gtgtctgacc acggtgcccc atgcgattgt taatgccgat gctagggcga 360
   aaagcacggc gagcagattg ctttgcactt gattcagggt agttgactaa agagttgctc 420
   gcgaagtagc acctgtcact tttgtctcaa atattaaatc gaatatcaat atatggtctg 480
   tttattggaa cgcgtcccag tggctgagac gcatccgcta aagccccagg aaccctgtgc 540
   agaaagaaaa cactcctctg gctaggtaga cacagtttat aaaggtagag ttgagcgggt 600
   aactgtcagc acgtagatcg aaaggtgcac aaagcaattg tggccgttac cctgcgaatg 660
   tccacagggt agctggtagt ttgaaaatca acgccgttgc ccttaggatt cagtaactgg 720
   cacattttgt aatgcgctag atctgtgtgc tcagtcttcc aggctgctta tcacagtgaa 780
   agcaaaacca attcgtggct gcgaaagtcg tagccaccac gaagtccagg aggacataca 840
   gtggccctgg tcgtacagaa atatggcggt tcctcgcttg agagtgcgga acgcattaga 900
   aacgtcgctg aacggatcgt tgccaccaag aaggctggaa atgatgtcgt ggttgtctgc 960
   tccgcaatgg gagacaccac ggatgaactt ctagaacttg cagcggcagt gaatcccgtt 1020
   ccgccagctc gtgaaatgga tatgctcctg actgctggtg agcgtatttc taacgctctc 1080
   gtcgccatgg ctattgagtc ccttggcgca gaagcccaat ctttcacggg ctctcaggct 1140
   ggtgtgctca ccaccgagcg ccacggaaac gcacgcattg ttgatgtcac tccaggtcgt 1200
   gtgcgtgaag cactcgatga gggcaagatc tgcattgttg ctggtttcca gggtgttaat 1260
   aaagaaaccc gcgatgtcac cacgttgggt cgtggtggtt ctgacaccac tgcagttgcg 1320
   ttggcagctg ctttgaacgc tgatgtgtgt gagatttact cggacgttga cggtgtgtat 1380
   accgctgacc cgcgcatcgt tcctaatgca cagaagctgg aaaagctcag cttcgaagaa 1440
   atgctggaac ttgctgctgt cccgggattt aaatcgctag cgggctgcta aaggaagcgg 1500
   aacacgtaga aagccagtcc gcagaaacgg tgctgacccc ggatgaatgt cagctactgg 1560
   gctatctgga caagggaaaa cgcaagcgca aagagaaagc aggtagcttg cagtgggctt 1620
   acatggcgat agctagactg ggcggtttta tggacagcaa gcgaaccgga attgccagct 1680
   ggggcgccct ctggtaaggt tgggaagccc tgcaaagtaa actggatggc tttcttgccg 1740
   ccaaggatct gatggcgcag gggatcaaga tctgatcaag agacaggatg aggatcgttt 1800
   cgcatgattg aacaagatgg attgcacgca ggttctccgg ccgcttgggt ggagaggcta 1860
   ttcggctatg actgggcaca acagacaatc ggctgctctg atgccgccgt gttccggctg 1920
   tcagcgcagg ggcgcccggt tctttttgtc aagaccgacc tgtccggtgc cctgaatgaa 1980
   ctgcaggacg aggcagcgcg gctatcgtgg ctggccacga cgggcgttcc ttgcgcagct 2040
   gtgctcgacg ttgtcactga agcgggaagg gactggctgc tattgggcga agtgccgggg 2100
   caggatctcc tgtcatctca ccttgctcct gccgagaaag tatccatcat ggctgatgca 2160
   atgcggcggc tgcatacgct tgatccggct acctgcccat tcgaccacca agcgaaacat 2220
   cgcatcgagc gagcacgtac tcggatggaa gccggtcttg tcgatcagga tgatctggac 2280
   gaagagcatc aggggctcgc gccagccgaa ctgttcgcca ggctcaaggc gcgcatgccc 2340
   gacggcgagg atctcgtcgt gacccatggc gatgcctgct tgccgaatat catggtggaa 2400
   aatggccgct tttctggatt catcgactgt ggccggctgg gtgtggcgga ccgctatcag 2460
   gacatagcgt tggctacccg tgatattgct gaagagcttg gcggcgaatg ggctgaccgc 2520
   ttcctcgtgc tttacggtat cgccgctccc gattcgcagc gcatcgcctt ctatcgcctt 2580
   cttgacgagt tcttctgagc gggactctgg ggttcgaaat gaccgaccaa gcgacgccca 2640
   acctgccatc acgagatttc gattccaccg ccgccttcta tgaaaggttg ggcttcggaa 2700
   tcgttttccg ggacgccggc tggatgatcc tccagcgcgg ggatctcatg ctggagttct 2760
   tcgcccacgc tagcggcgcg ccggccggcc cggtgtgaaa taccgcacag atgcgtaagg 2820
   agaaaatacc gcatcaggcg ctcttccgct tcctcgctca ctgactcgct gcgctcggtc 2880
   gttcggctgc ggcgagcggt atcagctcac tcaaaggcgg taatacggtt atccacagaa 2940
   tcaggggata acgcaggaaa gaacatgtga gcaaaaggcc agcaaaaggc caggaaccgt 3000
   aaaaaggccg cgttgctggc gtttttccat aggctccgcc cccctgacga gcatcacaaa 3060
   aatcgacgct caagtcagag gtggcgaaac ccgacaggac tataaagata ccaggcgttt 3120
   ccccctggaa gctccctcgt gcgctctcct gttccgaccc tgccgcttac cggatacctg 3180
   tccgcctttc tcccttcggg aagcgtggcg ctttctcata gctcacgctg taggtatctc 3240
   agttcggtgt aggtcgttcg ctccaagctg ggctgtgtgc acgaaccccc cgttcagccc 3300
   gaccgctgcg ccttatccgg taactatcgt cttgagtcca acccggtaag acacgactta 3360
   tcgccactgg cagcagccac tggtaacagg attagcagag cgaggtatgt aggcggtgct 3420
   acagagttct tgaagtggtg gcctaactac ggctacacta gaaggacagt atttggtatc 3480
   tgcgctctgc tgaagccagt taccttcgga aaaagagttg gtagctcttg atccggcaaa 3540
   caaaccaccg ctggtagcgg tggttttttt gtttgcaagc agcagattac gcgcagaaaa 3600
   aaaggatctc aagaagatcc tttgatcttt tctacggggt ctgacgctca gtggaacgaa 3660
   aactcacgtt aagggatttt ggtcatgaga ttatcaaaaa ggatcttcac ctagatcctt 3720
   ttaaaggccg gccgcggccg ccatcggcat tttcttttgc gtttttattt gttaactgtt 3780
   aattgtcctt gttcaaggat gctgtctttg acaacagatg ttttcttgcc tttgatgttc 3840
   agcaggaagc tcggcgcaaa cgttgattgt ttgtctgcgt agaatcctct gtttgtcata 3900
   tagcttgtaa tcacgacatt gtttcctttc gcttgaggta cagcgaagtg tgagtaagta 3960
   aaggttacat cgttaggatc aagatccatt tttaacacaa ggccagtttt gttcagcggc 4020
   ttgtatgggc cagttaaaga attagaaaca taaccaagca tgtaaatatc gttagacgta 4080
   atgccgtcaa tcgtcatttt tgatccgcgg gagtcagtga acaggtacca tttgccgttc 4140
   attttaaaga cgttcgcgcg ttcaatttca tctgttactg tgttagatgc aatcagcggt 4200
   ttcatcactt ttttcagtgt gtaatcatcg tttagctcaa tcataccgag agcgccgttt 4260
   gctaactcag ccgtgcgttt tttatcgctt tgcagaagtt tttgactttc ttgacggaag 4320
   aatgatgtgc ttttgccata gtatgctttg ttaaataaag attcttcgcc ttggtagcca 4380
   tcttcagttc cagtgtttgc ttcaaatact aagtatttgt ggcctttatc ttctacgtag 4440
   tgaggatctc tcagcgtatg gttgtcgcct gagctgtagt tgccttcatc gatgaactgc 4500
   tgtacatttt gatacgtttt tccgtcaccg tcaaagattg atttataatc ctctacaccg 4560
   ttgatgttca aagagctgtc tgatgctgat acgttaactt gtgcagttgt cagtgtttgt 4620
   ttgccgtaat gtttaccgga gaaatcagtg tagaataaac ggatttttcc gtcagatgta 4680
   aatgtggctg aacctgacca ttcttgtgtt tggtctttta ggatagaatc atttgcatcg 4740
   aatttgtcgc tgtctttaaa gacgcggcca gcgtttttcc agctgtcaat agaagtttcg 4800
   ccgacttttt gatagaacat gtaaatcgat gtgtcatccg catttttagg atctccggct 4860
   aatgcaaaga cgatgtggta gccgtgatag tttgcgacag tgccgtcagc gttttgtaat 4920
   ggccagctgt cccaaacgtc caggcctttt gcagaagaga tatttttaat tgtggacgaa 4980
   tcaaattcag aaacttgata tttttcattt ttttgctgtt cagggatttg cagcatatca 5040
   tggcgtgtaa tatgggaaat gccgtatgtt tccttatatg gcttttggtt cgtttctttc 5100
   gcaaacgctt gagttgcgcc tcctgccagc agtgcggtag taaaggttaa tactgttgct 5160
   tgttttgcaa actttttgat gttcatcgtt catgtctcct tttttatgta ctgtgttagc 5220
   ggtctgcttc ttccagccct cctgtttgaa gatggcaagt tagttacgca caataaaaaa 5280
   agacctaaaa tatgtaaggg gtgacgccaa agtatacact ttgcccttta cacattttag 5340
   gtcttgcctg ctttatcagt aacaaacccg cgcgatttac ttttcgacct cattctatta 5400
   gactctcgtt tggattgcaa ctggtctatt ttcctctttt gtttgataga aaatcataaa 5460
   aggatttgca gactacgggc ctaaagaact aaaaaatcta tctgtttctt ttcattctct 5520
   gtatttttta tagtttctgt tgcatgggca taaagttgcc tttttaatca caattcagaa 5580
   aatatcataa tatctcattt cactaaataa tagtgaacgg caggtatatg tgatgggtta 5640
   aaaaggatcg gcggccgctc gatttaaatc                                  5670
<210> 37
   <211> 1005
   <212> DNA
   <213> Corynebacterium glutamicum
<220>
   <223> FRUCTOSE-1,6-BISPHOSPHATASE
<400> 37
   atgaacctaa agaaccccga aacgccagac cgtaaccttg ctatggagct ggtgcgagtt 60
   acggaagcag ctgcactggc ttctggacgt tgggttggac gtggcatgaa gaatgaaggc 120
   gacggtgccg ctgttgacgc catgcgccag ctcatcaact cagtgaccat gaagggcgtc 180
   gttgttatcg gcgagggcga aaaagacgaa gctccaatgc tgtacaacgg cgaagaggtc 240
   ggaaccggct ttggacctga ggttgatatc gcagttgacc cagttgacgg caccaccctg 300
   atggctgagg gtcgccccaa cgcaatttcc attctcgcag ctgcagagcg tggcaccatg 360
   tacgatccat cctccgtctt ctacatgaag aagatcgccg tgggacctga ggccgcaggc 420
   aagatcgaca tcgaagctcc agttgcccac aacatcaacg cggtggcaaa gtccaaggga 480
   atcaaccctt ccgacgtcac cgttgtcgtg cttgaccgtc ctcgccacat cgaactgatc 540
   gcagacattc gtcgtgcagg cgcaaaggtt cgtctcatct ccgacggcga cgttgcaggt 600
   gcagttgcag cagctcagga ttccaactcc gtggacatca tgatgggcac cggcggaacc 660
   ccagaaggca tcatcactgc gtgcgccatg aagtgcatgg gtggcgaaat ccagggcatc 720
   ctggccccaa tgaacgattt cgagcgccag aaggcacacg acgctggtct ggttcttgat 780
   caggttctgc acaccaacga tctggtgagc tccgacaact gctacttcgt ggcaaccggt 840
   gtgaccaacg gtgacatgct ccgtggcgtt tcctaccgcg caaacggcgc aaccacccgt 900
   tccctggtta tgcgcgcaaa gtcaggcacc atccgccaca tcgagtctgt ccaccagctg 960
   tccaagctgc aggaatactc cgtggttgac tacaccaccg cgacc 1005
<210> 38
   <211> 335
   <212> PRT
   <213> Corynebacterium glutamicum
<400> 38
   Met Asn Leu Lys Asn Pro Glu Thr Pro Asp Arg Asn Leu Ala Met Glu
   1               5                   10                  15
   Leu Val Arg Val Thr Glu Ala Ala Ala Leu Ala Ser Gly Arg Trp Val
               20                  25                  30
   Gly Arg Gly Met Lys Asn Glu Gly Asp Gly Ala Ala Val Asp Ala Met
           35                  40                  45
   Arg Gln Leu Ile Asn Ser Val Thr Met Lys Gly Val Val Val Ile Gly
       50                  55                  60
   Glu Gly Glu Lys Asp Glu Ala Pro Met Leu Tyr Asn Gly Glu Glu Val
   65                  70                  75                  80
   Gly Thr Gly Phe Gly Pro Glu Val Asp Ile Ala Val Asp Pro Val Asp
                   85                  90                  95
   Gly Thr Thr Leu Met Ala Glu Gly Arg Pro Asn Ala Ile Ser Ile Leu
               100                 105                 110
   Ala Ala Ala Glu Arg Gly Thr Met Tyr Asp Pro Ser Ser Val Phe Tyr
           115                 120                 125
   Met Lys Lys Ile Ala Val Gly Pro Glu Ala Ala Gly Lys Ile Asp Ile
       130                 135                 140
   Glu Ala Pro Val Ala His Asn Ile Asn Ala Val Ala Lys Ser Lys Gly
   145                 150                 155                 160
   Ile Asn Pro Ser Asp Val Thr Val Val Val Leu Asp Arg Pro Arg His
                   165                 170                 175
   Ile Glu Leu Ile Ala Asp Ile Arg Arg Ala Gly Ala Lys Val Arg Leu
               180                 185                 190
   Ile Ser Asp Gly Asp Val Ala Gly Ala Val Ala Ala Ala Gln Asp Ser
           195                 200                 205
   Asn Ser Val Asp Ile Met Met Gly Thr Gly Gly Thr Pro Glu Gly Ile
       210                 215                 220
   Ile Thr Ala Cys Ala Met Lys Cys Met Gly Gly Glu Ile Gln Gly Ile
   225                 230                 235                 240
   Leu Ala Pro Met Asn Asp Phe Glu Arg Gln Lys Ala His Asp Ala Gly
                   245                 250                 255
   Leu Val Leu Asp Gln Val Leu His Thr Asn Asp Leu Val Ser Ser Asp
               260                 265                 270
   Asn Cys Tyr Phe Val Ala Thr Gly Val Thr Asn Gly Asp Met Leu Arg
           275                 280                 285
   Gly Val Ser Tyr Arg Ala Asn Gly Ala Thr Thr Arg Ser Leu Val Met
       290                 295                 300
   Arg Ala Lys Ser Gly Thr Ile Arg His Ile Glu Ser Val His Gln Leu
   305                 310                 315                 320
   Ser Lys Leu Gln Glu Tyr Ser Val Val Asp Tyr Thr Thr Ala Thr
                   325                 330                 335
<210> 39
   <211> 6
   <212> DNA
   <213> Corynebacterium glutamicum
<220>
   <223> POTENTIELLE_-10-REGION_1
<400> 39 6
   tagttt
<210> 40
   <211> 6
   <212> DNA
   <213> Corynebacterium glutamicum
<220>
   <223> POTENTIELLE_-10-REGION_2
<400> 40
   taggat 6
<210> 41
   <211> 6
   <212> DNA
   <213> Corynebacterium glutamicum
<220>
   <223> POTENTIELLE_-10-REGION_3
<400> 41
   tgcgct 6
<210> 42
   <211> 7
   <212> DNA
   <213> Corynebacterium glutamicum
<220>
   <223> RIBOSOMALE
<400> 42
   aggagga 7

## Beschreibung

Die vorliegende Erfindung betrifft ein Verfahren zur Erhöhung der Transkriptionsrate und/oder Expressionsrate von Genen sowie genetisch veränderte Mikroorganismen der Gattung Corynbacterium mit erhöhter Trankriptionsrate und/oder Expressionsrate sowie Verfahren zur Herstellung von biosynthetischen Produkten durch Kultivierung der genetisch veränderten Mikroorganismen der Gattung Corynbacterium, wie in den Anspruchen definiert.

Verschiedene biosynthetische Produkte, wie beispielsweise Feinchemikalien, wie unter anderem Aminosäuren, Vitamine aber auch Proteine werden über natürliche Stoffwechselprozesse in Zellen hergestellt und werden in vielen Industriezweigen verwendet, einschließlich der Nahrungsmittel-, Futtermittel-, Kosmetik-, Feed-, Food- und pharmazeutischen Industrie. Diese Substanzen, die zusammen als Feinchemikalien/Proteine bezeichnet werden, umfassen unter anderem organische Säuren, sowohl proteinogene als auch nicht-proteinogene Aminosäuren, Nukleotide und Nukleoside, Lipide und Fettsäuren, Diole, Kohlenhydrate, aromatische Verbindungen, Vitamine und Cofaktoren, sowie Proteine und Enzyme. Ihre Produktion erfolgt am zweckmäßigsten im Großmaßstab mittels Anzucht von Bakterien, die entwickelt wurden, um große Mengen der jeweils gewünschten Substanz zu produzieren und sezernieren. Für diesen Zweck besonders geeignete Organismen sind coryneforme Bakterien, grampositive nicht-pathogene Bakterien.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien, wie zum Beispiel die Zuckerkonzentration während der Fermentation, oder die Aufarbeitung zum Produkt, beispielsweise durch lonenaustauschchromatographie aber auch Sprühtrocknung, oder die intrinsischen Leistungseigenschaften des Mikroorganismus selbst betreffen.

Seit einigen Jahren werden ebenfalls Methoden der rekombinanten DNA-Technik zur Stammverbesserung von Feinchemikalien/Proteine produzierender Stämme von Corynebacterium eingesetzt, indem man einzelne Gene amplifiziert und die Auswirkung auf die Produktion von Feinchemikalien/Proteine untersucht.

Andere Wege, um ein Verfahren für die Herstellung Feinchemikalien, Aminosäuren oder Proteine zu entwickeln, oder die Produktivität eines bereits existierenden Verfahrens für die Herstellung Feinchemikalien, Aminosäuren oder Proteine zu erhöhen bzw. zu verbessern, sind die Expression eines oder mehrerer Gene zu erhöhen bzw. zu verändern und oder die Translation einer mRNA durch geeignete Polynukleotidsequenzen zu beeinflussen. Beeinflussung kann in diesem Zusammenhang die Erhöhung, Verringerung, oder auch andere Parameter der Expression von Genen wie zeitliche Expressionsmuster umfassen.

Dem Fachmann sind unterschiedliche Bestandteile von bakteriellen Regulationssequenzen bekannt. Man unterscheidet die Bindungstellen von Regulatoren, auch Operatoren genannt, die Bindungstellen von RNA-Polymerase-Holoenzymen, auch -35 und - 10 Regionen genannt, und die Bindungsstelle von Ribosomaler 16S-RNA, auch Ribosomale Bindungsstelle oder auch Shine-Dalgarno-Sequenz genannt.

Als Sequenz einer Ribosomalen Bindungsstelle, auch Shine-Dalgamo-Sequenz genannt, im Sinne dieser Erfindung werden Polynukleotidsequenzen verstanden, die sich bis zu 20 Basen stromauf des Initiationskodon der Translation befinden.

In der Literatur (E. coli und S. typhimurium, Neidhardt F.C. 1995 ASM Press) wird beschrieben, dass sowohl die Zusammensetzung der Polynukletidsequenz der Shine-Delgarno-Sequenz, die Sequenzabfolge der Basen, aber auch der Abstand einer in der Shine-Delgarno-Sequenz enthaltenen Polynukletidsequenz zum einen wesentlichen Einfluss auf die Initiationstionsrate der Translation hat.

Nukleinsäuresequenzen mit Promotoraktivität können die Bildung von mRNA auf unterschiedliche Weise beeinflussen. Promotoren, deren Aktivität unabhängig von der physiologischen Wachstumsphase des Organismus sind, nennt man konstitutiv. Wiederum andere Promotoren reagieren auf externe chemische, wie physikalische Stimuli wie Sauerstoff, Metabolite, Hitze, pH, etc.. Wiederum andere zeigen in unterschiedlichen Wachstumsphasen eine starke Abhängigkeit ihrer Aktivität. Beispielsweise sind in der Literatur Promotoren beschrieben, die während der exponentiellen Wachstumsphase von Mikroorganismen eine besonders ausgeprägte Aktivität zeigen, oder aber auch genau in der stationären Phase des mikrobiellen Wachstums. Beide Charakteristika von Promotoren können für eine Produktion von Feinchemikalien und Proteine je nach Stoffwechselweg einen günstigen Einfluss auf die Produktivität haben.

Zum Beispiel kann man Promotoren die während des Wachstums die Expresssion eines Gens ausschalten, diese aber nach einem optimalen Wachstum anschalten dazu nutzen, ein Gen zu regulieren, das die Produktion eines Metaboliten kontrolliert. Der veränderte Stamm weist dann die gleichen Wachstumsparameter wie der Ausgangsstamm auf, produziert aber mehr Produkt pro Zelle. Diese Art der Modifizierung kann sowohl den Titer (g Produkt/Liter) als auch die C-Ausbeute (g Produkt/g-C-Quelle) erhöhen.

In Corynebacterium Spezies konnten bereits solche Nukleotidsequenzen isoliert werden, die für eine Erhöhung bzw. eine Abschwächung der Genexpression genutzt werden können. Diese regulierten Promotoren können die Rate, mit der ein Gen transkribiert wird, abhängig von den internen und/oder externen Bedingungen der Zelle erhöhen oder erniedrigen. Zum Teil kann die Anwesenheit eines bestimmten Faktors, bekannt als Inducer, die Rate der Transkrition vom Promotor stimulieren. Inducer können direkt oder aber indirekt die Transkription vom Promotor beeinflussen. Eine andere Klasse von Faktoren, bekannt als Suppressoren ist in der Lage, die Transkription vom Promotor zu reduzieren oder aber zu inhibieren. Wie auch die Inducer, können auch die Suppressoren direkt oder indirekt wirken. Es sind jedoch auch Promotoren bekannt, die über die Temperatur reguliert werden. So kann der Level der Transkription solcher Promotoren zum Beispiel durch eine Erhöhung der Wachstumstemperatur über die normale Wachstumstemperatur der Zelle erhöht oder aber abgeschwächt werden.

Eine geringe Anzahl von Promotoren aus C. glutamicum wurden bis zum heutigen Tag beschrieben. Der Promotor des Malatsynthase-Gens aus C. glutamicum wurde im DE 4440118 beschrieben. Dieser Promotor wurde einem für ein Protein kodierendes Strukturgen vorgeschaltet. Nach Transformation eines solchen Konstrukts in ein coryneformes Bakterium wird die Expression des dem Promotor nachgeschalteten Strukturgen reguliert. Die Expression des Strukturgens wird induziert sobald dem Medium ein ensprechender Induktor zugesetzt wird.

Reinscheid et al., Microbiology 145:503 (1999) haben eine trankriptionelle Fusion zwischen dem pta-ack Promotor aus C. glutamicum und einem Reportergen (Chloramphenicol Acetyltransferase) beschrieben. Zellen von C. glutamicum, die eine solche transkriptionelle Fusion enthalten, wiesen eine erhöhte Expression des Reportergenes bei Wachstum auf Acetat haltigem Medium auf. Im Vergleich dazu zeigten transformierte Zellen, die auf Glucose wuchsen, keine erhöhte Expression dieses Reportergens.

In Pa'tek et al., Microbiology 142:1297 (1996) wurden einige DNA Sequenzen aus C. glutamicum beschrieben, die die Expression eines Reportergens in C. glutamicum Zellen verstärken können, beschrieben. Diese Sequenzen wurden miteinander verglichen, um Consensus-Sequenzen für C. glutamicum Promotoren zu definieren.

Weitere DNA-Sequenzen aus C. glutamicum, die zur Regulation der Genexpression genutzt werden können, sind im Patent WO 02/40679 beschrieben worden. Diese isolierten Polynukleotide stellen Expressionseinheiten aus Corynebakterium glutamicum dar, die entweder zur Erhöhung oder aber zur Verringerung einer Genexpression genutzt werden können. Weiterhin sind in diesem Patent rekombinante Plasmide beschrieben, auf denen die Expressionseinheiten aus Corynebakterium glutamicum mit heterologen Genen assoziiert sind. Die hier beschrieben Methode, Fusion von einem Promotor aus Corynebakterium glutamicum mit einem heterlogen Gen, kann unter anderem zur Regulation der Gene der Aminosäurebiosynthese eingesetzt werden.

Der Erfindung lag die Aufgabe zugrunde weitere Promotoren und/oder Expressionseinheiten mit vorteilhaften Eigenschaften zur Verfügung zustellen.

Demgemäß wurde gefunden, dass man Nukleinsäuren mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
zur Transkription von Genen verwenden kann.

Unter "Transkription" wird erfindungsgemäß der Prozess verstanden, durch den ausgehend von einer DNA-Matrize ein komplementäres RNA-Molekül hergestellt wird. An diesem Prozess sind Proteine wie die RNA-Polymerase sogenannte Sigma-Faktoren und transkriptionelle Regulatorproteine beteiligt. Die synthetisierte RNA dient dann als Matrize im Prozess der Translation, der dann zum biosynthetisch aktiven Protein führt.

Die Bildungsrate, mit der ein biosynthetsich aktives Protein hergestellt wird, ist ein Produkt aus der Rate der Transkription und der Translation. Beide Raten können erfindungsgemäß beeinflusst werden und damit die Rate der Bildung von Produkten in einem Mikroorganismus beeinflussen.

Unter einem "Promotor" oder einer "Nukleinsäure mit Promotoraktivität" wird erfindungsgemäß eine Nukleinsäure verstanden, die in funktioneller Verknüpfung mit einer zu trankripierenden Nukleinsäure, die Transkription dieser Nukleinsäure reguliert.

Unter einer "funktionellen Verknüpfung" versteht man in diesem Zusammenhang beispielsweise die sequentielle Anordnung einer der erfindungsgemäßen Nukleinsäuren mit Promotoraktivität und einer zu transkribierenden Nukleinsäuresequenz und ggf. weiterer regulativer Elemente wie zum Beispiel Nukleinsäureseuqenzen, die die Transkription von Nukleinsäuren gewährleisten, sowie zum Beipsiel einen Terminator derart, dass jedes der regulativen Elemente seine Funktion bei der Transkription der der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu trankribierende Nukleinsäuresequenz hinter (d.h. am 3'-Ende) der erfindungsgemäßen Promotorsequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Promotoraktivität" wird erfindungsgemäß die in einer bestimmten Zeit durch den Promotor gebildete Menge RNA, also die Trankriptionsrate verstanden.

Unter "spezifischer Promotoraktivität" wird erfindungsgemäß die in einer bestimmten Zeit durch den Promotor gebildete Menge RNA pro Promotor verstanden.

Unter dem Begriff "Wildtyp" wird erfindungsgemäß der entsprechende Ausgangsmikroorganismus verstanden.

Je nach Zusammenhang kann unter dem Begriff "Mikroorganismus" der Ausgangsmikroorganismus (Wildtyp) oder ein erfindungsgemäßer, genetisch veränderter Mikroorganismus oder beides verstanden werden.

Vorzugsweise und insbesondere in Fällen, in denen der Mikroorganismus oder der Wildtyp nicht eindeutig zugeordnet werden kann, wird unter "Wildtyp" für die Veränderung oder Verursachung der Promotoraktivität oder Trankriptionsrate, für die Veränderung oder Verursachung der Expressionsaktivität oder Expressionsrate und für die Erhöhung des Gehalts an biosynthetischen Produkten jeweils ein Referenzorganismus verstanden.

In einer bevorzugten Ausführungsform ist dieser Referenzorganismus Corynebakterium glutamicum ATCC 13032.

In einer bevorzugten Ausführungsform werden Ausgangsmikroorganismen verwendet die bereits in der Lage sind, die gewünschte Feinchemikalie herzustellen. Besonders bevorzugt sind dabei unter den besonders bevorzugten Mikroorganismen der Bakterien der Gattung Corynebacterien und den besonders bevorzugten Feinchemikalien L-Lysin, L-Methionin und L-Threonin, diejenigen Ausgangsmikroorganismen die bereits in der Lage sind, L-Lysin, L-Methionin und/oder L-Threonin herzustellen. Dies sind besonderes bevorzugt Corynebakterien bei denen beispielsweise das Gen kodierend für eine Aspartokinase (ask-Gen) dereguliert ist oder die feed-back-Inhibierung aufgehoben oder reduziert ist. Beispielsweise weisen solche Bakterien im ask-Gen eine Mutation auf, die zu einer Reduzierung oder Aufhebung der feed-back-Inhibierung führen, wie beispielsweise die Mutation T311I.

Bei einer "verursachten Promotoraktivität" oder Transkriptionsrate im Bezug auf ein Gen im Vergleich zum Wildtyp wird somit im Vergleich zum Wildtyp die Bildung einer RNA verursacht, die im Wildtyp so nicht vorhanden war.

Bei einer veränderten Promotoraktivität oder Transkriptionsrate im Bezug auf ein Gen im Vergleich zum Wildtyp wird somit im Vergleich zum Wildtyp in einer bestimmten Zeit die gebildete Menge der RNA verändert.

Unter "verändert" wird in diesem Zusammenhang bevorzugt erhöht oder erniedrigt verstanden.

Die erhöhte Promotoraktivität oder Transkriptionsrate kann beispielsweise durch Regulation der Transkription von Genen im Mikroorganismus durch Nukleinsäuren mit Promotoraktivität t erreicht werden, wobei die Gene in Bezug auf die Nukleinsäuren mit Promotoraktivität heterolog sind.

Vorzusgweise wird die Regulation der Transkription von Genen im Mikroorganismus durch Nukleinsäuren mit Promotoraktivität oder durch Nukleinsäuren mit erhöhter spezifischer Promotoraktivität dadurch erreicht, dass man
eine oder mehrere Nukleinsäuren mit Promotoraktivität, gegebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gegebenenfalls mit veränderter spezifischer Promotoraktivität, erfolgt oder
ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität, gegebenenfalls mit veränderter spezifischer Promotoraktivität, erfolgt oder
ein oder mehrere Nukleinsäurekonstrukte, enthaltend eine Nukleinsäure mit Promotoraktivität, gegebenenfalls mit veränderter spezifischer Promotoraktivität, und funktionell verknüpft eine oder mehrere, zu transkribierende Nukleinsäuren, in den Mikroorganismus einbringt.

Die Nukleinsäuren mit Promotoraktivität enthalten
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist.

Die Nukleinsäuresequenz SEQ. ID. NO. 1 stellt die Promotorsequenz des Protein Translation Elongations Faktor TU (P EF-TU) aus Corynebakterium glutamicum dar. SEQ. ID.NO. 1 entspricht der Promotorsequenz des Wildtyps.

Weitere Nukleinsäuren mit Promotoraktivität enthalten eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist.

Weitere natürliche Beispiele für Nukleinsäuren mit Promotoraktivität lassen sich beispielsweise aus verschiedenen Organismen, deren genomische Sequenz bekannt ist, durch Identitätsvergleiche der Nukleinsäuresequenzen aus Datenbanken mit der vorstehend beschriebenen Sequenzen SEQ ID NO: 1 leicht auffinden.

Künstliche Nukleinsäuren mit Promotoraktivität lassen sich ausgehend von der Sequenz SEQ ID NO: 1 durch künstliche Variation und Mutation, beispielsweise durch Substitution, Insertion oder Deletion von Nukleotiden leicht auffinden.

Unter dem Begriff "Substitution" ist in der Beschreibung der Austausch einer oder mehrerer Nukleotide durch ein oder mehrere Nukleotide zu verstehen. "Deletion" ist das Ersetzen eines Nukleotides durch eine direkte Bindung. Insertionen sind Einfügungen von Nukleotiden in die Nukleinsäuresequenz, wobei formal eine direkte Bindung durch ein oder mehrere Nukleotide ersetzt wird.

Unter Identität zwischen zwei Nukleinsäuren wird die Identität der Nukleotide über die jeweils gesamte Nukleinsäurelänge verstanden, insbesondere die Identität die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameter:
   Gap opening penalty 10
   Gap extension penalty 10
   Gap separation penalty range 8
   Gap separation penalty off
   % identity for alignment delay 40
   Residue specific gaps off
   Hydrophilic residue gap off
   Transition weighing 0
Pairwise alignment parameter:
   FAST algorithm on
   K-tuplesize 1
   Gap penalty 3
   Window size 5
   Number of best diagonals 5

Unter einer Nukleinsäuresequenz, die eine Identität von mindestens 90 % mit der Sequenz SEQ ID NO: 1 aufweist, wird dementsprechend eine Nukleinsäuresequenz verstanden, die bei einem Vergleich seiner Sequenz mit der Sequenz SEQ ID NO: 1, insbesondere nach obigen Programmlogarithmus mit obigem Parametersatz eine Identität von mindestens 90 % aufweist.

Besonders bevorzugte Promotoren weisen mit der Nukleinsäuresequenz SEQ. ID. NO. 1 eine Identität von 91 %, bevorzugter 92%, 93%, 94%, 95%, 96%, 97%, 98%, besonders bevorzugt 99% auf.

Die SEQ. ID. NO. 1 ist ohne Funktionszuordnung im Genbank-Eintrag AP005283 beschrieben worden.

Nukleinsäuren mit Promotoraktivität enthalten
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
mit der Maßgabe, dass die Nukleinsäure mit der Sequenz SEQ. ID. NO. 1 ausgenommen ist.

Alle vorstehend erwähnten Nukleinsäuren mit Promotoraktivität sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Unter einer Expressioneinheit wird eine Nukleinsäure mit Expressionsaktivität verstanden, also eine Nukleinsäure verstanden, die in funktioneller Verknüpfung mit einer zu exprimierenden Nukleinsäure oder Gens, die Expression, also die Transkription und die Translation dieser Nukleinsäure oder dieses Gens reguliert.

Unter einer "funktionellen Verknüpfung" versteht man in diesem Zusammenhang beispielsweise die sequentielle Anordnung einer der Expressionseinheit und einer transgen zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter (d.h. am 3'-Ende) der Expressionseinheitssequenz positioniert wird, so dass beide Sequenzen kovalent miteinander verbunden sind. Bevorzugt ist dabei der Abstand zwischen der Expressionseinheitssequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Expressionsaktivität" wird die in einer bestimmten Zeit durch die Expressionseinheit gebildete Menge Protein, also die Expressionsrate verstanden.

Unter "spezifischer Expressionsaktivität" die in einer bestimmten Zeit durch die Expressionseinheit gebildete Menge Protein pro Expressionseinheit verstanden.

Bei einer "verursachten Expressionsaktivität" oder Expressionsrate im Bezug auf ein Gen im Vergleich zum Wildtyp wird somit im Vergleich zum Wildtyp die Bildung eines Proteins verursacht, das im Wildtyp so nicht vorhanden war.

Bei einer "veränderten Expressionsaktivität" oder Expressionsrate im Bezug auf ein Gen im Vergleich zum Wildtyp wird somit im Vergleich zum Wildtyp in einer bestimmten Zeit die gebildete Menge des Proteins verändert.

Unter "verändert" wird in diesem Zusammenhang bevorzugt erhöht oder erniedrigt verstanden.

Dies kann beispielsweise durch Erhöhung oder Reduzierung der spezifischen Aktivität der endogenen Expressionseinheit, beispielsweise durch Mutation der Expressionseinheit oder durch Stimmulierung oder Hemmung der Expressionseinheit erfolgen.

Weiterhin kann die erhöhte Expressionsaktivität oder Expressionsrate beispielsweise durch Regulation der Expression von Genen im Mikroorganismus durch Expressionseinheiten oder durch Expressionseinheiten mit Erhöhter spezifischer Expressionsaktivität erreicht werden, wobei die Gene im Bezug auf die Expressionseinheiten heterolog sind.

Vorzugsweise wird die Regulation der Expression von Genen im Mikroorganismus durch Expressionseinheiten erreicht, dass man
eine oder mehrere Expressionseinheiten in das Genom des Mikroorganismus einbringt, so dass die Expression eines oder mehrerer endogenen Gene unter der Kontrolle der eingebrachten Expressionseinheiten erfolgt oder
ein oder mehrere Gene in das Genom des Mikrorganismus einbringt, so dass die Expression eines oder mehrerer der eingebrachten Gene unter der Kontrolle der endogenenExpressionseinheiten erfolgt oder
ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Die Expressionseinheiten, enthalten eine vorstehend bechriebene Nukleinsäure mit Promotoraktivität und zusätzlich funktionell verknüpft eine Nukleinsäuresequenz, die die Translation von Ribonukleinsäuren gewährleistet.

Vorzugsweise enthält diese Nukleinsäuresequenz, die die Translation von Ribonukleinsäuren gewährleistet, die Nukleinsäuresequenz SEQ. ID. NO. 42 als ribosomale Bindungsstelle.

Die Expressionseinheit enthält:
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist.

Die Nukleinsäuresequenz SEQ. ID. NO. 2 stellt die Nukleinsäuresequenz der Expressionseinheit des Protein Translation Elongations Faktor TU (P EF-TU) aus Corynebakterium glutamicum dar. SEQ. ID.NO. 2 entspricht der Sequenz der Expressionseinheit des Wildtyps.

Die Expressionseinheiten können eine von dieser Sequenz durch Substitution, Insertion oder Deletion von Nukleotiden abgeleitete Sequenz enthalten, die eine Identität von mindestens 90 % auf Nukleinsäureebene mit der Sequenz SEQ. ID. NO. 2 aufweist.

Weitere natürliche Beispiele für vorstehend beschriebene Expressionseinheiten lassen sich beispielsweise aus verschiedenen Organismen, deren genomische Sequenz bekannt ist, durch Identitätsvergleiche der Nukleinsäuresequenzen aus Datenbanken mit der vorstehend beschriebenen Sequenzen SEQ ID NO: 2 leicht auffinden.

Künstliche Sequenzen der vorstehen beschriebenen Expressionseinheiten lassen sich ausgehend von der Sequenz SEQ ID NO: 2 durch künstliche Variation und Mutation, beispielsweise durch Substitution, Insertion oder Deletion von Nukleotiden leicht auffinden.

Unter einer Nukleinsäuresequenz, die eine Identität von mindestens 90 % mit der Sequenz SEQ ID NO: 2 aufweist, wird dementsprechend eine Nukleinsäuresequenz verstanden, die bei einem Vergleich seiner Sequenz mit der Sequenz SEQ ID NO: 2, insbesondere nach obigen Programmlogarithmus mit obigem Parametersatz eine Identität von mindestens 90 % aufweist.

Besonders bevorzugte Expressionseinheiten weisen mit der Nukleinsäuresequenz SEQ. ID. NO. 2 eine Identität von 91 %, bevorzugter 92%, 93%, 94%, 95%, 96%, 97%, 98%, besonders bevorzugt 99% auf.

Umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Die Nukleinsäuresequenz SEQ. ID. NO. 2 kann als Expressionseinheit, d.h. zur Expression von Genen verwendet werden.

Die SEQ. ID. NO. 2 ist ohne Funktionszuordnung im Genbank-Eintrag AP005283 beschrieben worden.

Eine Expressionseinheit enthält eine vorstehend genannte Nukleinsäure mit Promotoraktivität zusätzlich funktionell verknüpft mit einer Nukleinsäuresequenz, die die Translation von Ribonukleinsäuren gewährleistet.

Eine Expressionseinheit enthält bevorzugt
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist
mit der Maßgabe, dass die Nukleinsäure mit der Sequenz SEQ. ID. NO. 2 ausgenommen ist.

Die vorstehend genannten Expressionseinheiten umfassen ein oder mehrere der folgenden genetischen Elemente: eine Minus 10 ("-10") Sequenz; eine Minus 35 ("-35") Sequenz; einen Transkriptionsstart, eine Enhancer Region; und eine Operator Region. Vorzugsweise sind diese genetischen Elemente spezifisch für die Spezies Corynebakterien, speziell für Corynbacterium glutamicum.

Alle vorstehend erwähnten Expressionseinheiten sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Für die Erfindungen in diesem Patent wurden Methoden und Techniken genutzt, die dem Fachmann, der in mikrobiologischen und rekombinanten DNA-Techniken geübt ist, bekannt sind. Methoden und Techniken für das Wachstum von Bakterienzellen, das Einschleusen von isolierten DNA-Molekülen in die Wirtszelle, und die Isolierung, Klonierung und Sequenzierung von isolierten Nukleinsäuremolekülen usw. sind Beispiele für solche Techniken und Methoden. Diese Methoden sind in vielen Standardliteraturstellen beschrieben: Davis et al., Basic Methods In Molecular Biology (1986); J. H. Miller, Experiments in Molecular Genetics, Cold Spring Harbor Laboratory Press, Cold Spring Harbor, New York (1972); J.H. Miller, A Short Course in Bacterial Genetics, Cold Spring Harbor Laboratory Press, Cold Spring Harbor, New York (1992); M. Singer and P. Berg, Genes & Genomes, University Science Books, Mill Valley, Carlifomia (1991); J. Sambrook, E.F. Fritsch and T. Maniatis, Molecular Cloning: A Laboratory Manual, 2nd ed., Cold Spring Harbor Laboratory Press, Cold Spring Harbor, New York (1989); P.B. Kaufmann et al., Handbook of Molcular and Cellular Methods in Biology and Medicine, CRC Press, Boca Raton, Florida (1995); Methods in Plant Molecular Biology and Biotechnology, B.R. Glick and J.E. Thompson, eds., CRC Press, Boca Raton, Florida (1993); and P.F. Smith-Keary, Molecular Genetics of Escherichia coli, The Guilford Press, New York, NY (1989).

Alle Nukleinsäuremoleküle der vorliegenden Erfindung liegen bevorzugt in Form eines isolierten Nukleinsäuremoleküls vor. Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Umfasst sind weiterhin die zu den konkret beschriebenen Nukleotidsequenzen komplementären Nukleinsäuremoleküle oder einen Abschnitt davon.

Die vorstehend genanntenPromotoren und/oder Expressionseinheiten lassen sich beispielsweise besonders vorteilhaft in verbesserten Verfahren zur fermentativen Herstellung von biosynthetischen Produkten wie nachstehend beschrieben verwenden.

Die vorstehend genannten Promotoren und/oder Expressionseinheiten weisen insbesondere den Vorteil auf, dass es sich um starke, konstitutive Promotoren und Expressionseinheiten handelt.

Die vorstehend genannten Nukleinsäuren mit Promotoraktivität können zur Erhöhung der Transkriptionsrate von Genen in Mikroorganismen der Gattung Corynbacterium im Vergleich zum Wildtyp verwendet werden.

Die vorstehend genanntenExpressionseinheiten können zur Erhöhung der Expressionsrate von Genen in Mikroorganismen der Gattung Corynbacterium im Vergleich zum Wildtyp verwendet werden.

Ferner können die vorstehend genannten Nukleinsäuren mit Promotoraktivität und die vorstehend genannten Expressionseinheiten zur Regulation und Verstärkung der Bildung von verschiedenen biosynthetischen Produkten, wie beispielsweise Feinchemikalien, Proteinen, inbesondere Aminosäuren, in Mikroorganismen der Gattung Corynbacterium, dienen.

Die Erfindung betrifft daher ein Verfahren zur Erhöhung der Transkriptionsrate von Genen in Mikroorganismen der Gattung Corynebacterium im Vergleich zum Wildtyp durch
Regulation der Transkription von Genen im Mikroorganismus durch Nukleinsäuren mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist, wobei die Gene in Bezug auf die Nukleinsäuren mit Promotoraktivität heterolog sind.

Die Nukleinsäuresequenz SEQ. ID. NO. 42 stellt vorzugsweise die ribosomale Bindungsstelle der vorstehend genannten Expressionseinheiten, die Sequenzen SEQ. ID. NOs. 39, 40 oder 41 die-10-Region der vorstehend genanntenExpressionseinheiten dar. Veränderungen der Nukleinsäureaequenz in diesen Regionen führen zu einer Veränderung der spezifischen Expressionsaktivität.

Die Nukleinsäuresequenz SEQ. ID. NO. 42 können als ribosomale Bindungsstelle in Expressionseinheiten verwendet werden, die die Expression von Genen in Bakterien der Gattung Corynebacterium oder Brevibacterium ermöglichen.

Die Nukleinsäuresequenzen SEQ. ID. NOs. 39, 40 oder 41 können als -10-Region in Expressionseinheiten verwendet werden, die die Expression von Genen in Bakterien der Gattung Corynebacterium oder Brevibacterium ermöglichen.

Es kann eine Expressionseinheit verwendet werden, die die Expression von Genen in Bakterien der Gattung Corynebacterium oder Brevibacterium ermöglicht, enthaltend die Nukleinsäuresequenz SEQ. ID. NO. 42. Vorzugsweise wird dabei die Nukleinsäuresequenz SEQ. ID. NO. 42. als ribosomale Bindungsstelle verwendet.

Es klann eine Expressionseinheit verwendete werden , die die Expression von Genen in Bakterien der Gattung Corynebacterium oder Brevibacterium ermöglicht, enthaltend mindestens eine der Nukleinsäuresequenzen SEQ. ID. NOs. 39, 40 oder 41. Vorzugsweise wird dabei eine der Nukleinsäuresequenzen SEQ. ID. NOs. 39, 40 oder 41 als-10-Region verwendet.

Gemäß der Erfindung kann die Erhöhung der Transkriptionsrate von Genen in Mikroorganismen der Gattung Corynbacterium im Vergleich zum Wildtyp dadurch erfolgen, dass man die Transkription von Genen im Mikroorganismus durch Nukleinsäuren mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
reguliert, wobei die Gene in Bezug auf die Nukleinsäuren mit Promotoraktivität heterolog sind.

Dies wird bevorzugt dadurch erreicht, dass man
b1) eine oder mehrere vorstehend genannte Nukleinsäuren mit Promotoraktivität in das Genom des Mikroorganismus einbringt, so dass die Transkription eines oder mehrerer endogenen Gene unter der Kontrolle der eingebrachten Nukleinsäure mit Promotoraktivität erfolgt oder
b2) ein oder mehrere Gene in das Genom des Mikrorganismus einbringt, so dass die Transkription eines oder mehrerer der eingebrachten Gene unter der Kontrolle der endogenen, vorstehend genannten Nukleinsäuren mit Promotoraktivität, erfolgt oder
b3) ein oder mehrere Nukleinsäurekonstrukte, enthaltend eine vorstehend genannte Nukleinsäure mit Promotoraktivität und funktionell verknüpft eine oder mehrere, zu transkribierende Nukleinsäuren, in den Mikroorganismus einbringt.

Es ist somit möglich, die Transkriptionsrate eines endogenen Gens des Wildtyps zu erhöhen indem man

gemäß Ausführungsform b1) eine oder mehrere vorstehend genannte Nukleinsäuren mit Promotoraktivität in das Genom des Mikroorganismus einbringt, so dass die Transkription eines oder mehrerer endogenen Gene unter der Kontrolle der eingebrachten Nukleinsäure mit Promotoraktivität erfolgt oder

gemäß Ausführungsform b2) ein oder mehrere endogene Gene in das Genom des Mikrorganismus einbringt, so dass die Transkription eines oder mehrerer der eingebrachten, endogenen Gene unter der Kontrolle der endogenen, vorstehend genannten Nukleinsäuren mit Promotoraktivität erfolgt oder

Gemäß Ausführungsform b3) ein oder mehrere Nukleinsäurekonstrukte, enthaltend eine vorstehend genannte Nukleinsäure mit Promotoraktivität, und funktionell verknüpft eine oder mehrere, zu transkribierende endogene Nukleinsäuren, in den Mikroorganismus einbringt.

Ferner ist es somit möglich, die Transkriptionsrate eines exogenen Gens im Vergleich zum Wildtyps zu erhöhen, indem man

gemäß Ausführungsform b2) ein oder mehrere exogene Gene in das Genom des Mikrorganismus einbringt, so dass die Transkription eines oder mehrerer der eingebrachten, exogenen Gene unter der Kontrolle der endogenen, vorstehend genannten Nukleinsäuren mit Promotoraktivität, erfolgt oder

Gemäß Ausführungsform b3) ein oder mehrere Nukleinsäurekonstrukte, enthaltend eine vorstehend genannte Nukleinsäure mit Promotoraktivität und funktionell verknüpft eine oder mehrere, zu transkribierende exogene Nukleinsäuren, in den Mikroorganismus einbringt.

Die Insertion von Genen gemäß Ausführungsform b2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform b3) kann dabei chromosomal oder extrachromosomal erfolgen. Vorzugsweise erfolgt die Insertion der Nukleinsäurekonstrukte chromosomal. Eine "chromosomale" Integration ist die Insertion eines exogenen DNA-Fragmentes in das Chromosom einer Wirtszelle. Dieser Begriff wird auch für die homologe Rekombination zwischen einem exogenen DNA-Fragment und der entsprechenden Region auf dem Chromosom der Wirtszelle genutzt.

Unter "endogen" werden genetische Informationen, wie beispielsweise Gene, verstanden, die bereits im Wildtypgenom enthalten sind.

Unter "exogen" werden genetische Informationen, wie beispielsweise Gene, verstanden, die im Wildtypgenom nicht enthalten sind.

Unter dem Begriff "Gene" in Bezug auf Regulation der Transkription durch die evorstehend genannten Nukleinsäuren mit Promotoraktivität werden vorzugsweise Nukleinsäuren verstanden, die einen zu transkripierenden Bereich, also beispielsweise einen Bereich der die Translation reguliert, einen kodierenden Bereich, sowie gegebenenfalls weitere Regulationselemente, wie beispielsweise einen Terminator, enthalten.

Unter dem Begriff "Gene" in Bezug auf die nachstehend beschriebene Regulation der Expression durchExpressionseinheiten werden vorzugsweise Nukleinsäuren verstanden, die einen kodierenden Bereich, sowie gegebenenfalls weitere Regulationselemente, wie beispielsweise einen Terminator, enthalten.

Unter einem "kodierenden Bereich" wird eine Nukleinsäuresequenz verstanden, die ein Protein kodiert.

Unter "heterolog" in Bezug auf Nukleinsären mit Promotoraktivität und Gene wird verstanden, dass die verwendeten Gene im Wildtyp nicht unter Regulation der vorstehend genannten Nukleinsäuren mit Promotoraktivität transkribiert werden, sondern das eine neue, im Wildtyp nicht vorkommende funktionelle Verknüpfung entsteht und die funktionelle Kombination aus vorstehend genannter Nukleinsäure mit Promotoraktivität und spezifisches Gen im Wildtyp nicht vorkommt.

Unter "heterolog" in Bezug auf Expressionseinheiten und Gene wird verstanden, dass die verwendeten Gene im Wildtyp nicht unter Regulation der vorstehen genanntenn Expressionseinheiten exprimiert werden, sondern das eine neue, im Wildtyp nicht vorkommende funktionelle Verknüpfung entsteht und die funktionelle Kombination aus vorstehend genannter Expressionseinheit und spezifisches Gen im Wildtyp nicht vorkommt.

Ferner beschrieben ist ein Verfahren zur Erhöhung der Expressionsrate eines Gens in Mikroorganismen der Gattung Corynbacterium im Vergleich zum Wildtyp durch

Regulation der Expression von Genen im Mikroorganismus durch Expressionseinheiten, enthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist, wobei die Gene im Bezug auf die Expressionseinheiten heterolog sind.

Die Erhöhung der Expressionsrate von Genen in Mikroorganismen der Gattung Corynbacterium im Vergleich zum Wildtyp kann dadurch erfolgen, dass man die Expression von Genen im Mikroorganismus durch vorstehend genannte Expressionseinheiten reguliert, wobei die Gene in Bezug auf die Expressionseinheiten heterolog sind.

Dies wird bevorzugt dadurch erreicht, dass man
d1) eine oder mehrere vorstehend genannte Expressionseinheiten in das Genom des Mikroorganismus einbringt, so dass die Expression eines oder mehrerer endogenen Gene unter der Kontrolle der eingebrachten Expressionseinheiten erfolgt oder
d2) ein oder mehrere Gene in das Genom des Mikrorganismus einbringt, so dass die Expression eines oder mehrerer der eingebrachten Gene unter der Kontrolle der vorstehend genannten, endogenen Expressionseinheiten erfolgt oder
d3) ein oder mehrere Nukleinsäurekonstrukte, enthaltend eine vorstehend genannte Expressionseinheit und funktionell verknüpft eine oder mehrere, zu exprimierende Nukleinsäuren, in den Mikroorganismus einbringt.

Es ist somit möglich, die Expressionsrate eines endogenen Gens des Wildtyps zu erhöhen indem man
gemäß Ausführungsform d1) eine oder mehrere vorstehend genannte Expressionseinheiten in das Genom des Mikroorganismus einbringt, so dass die Expression eines oder mehrerer endogenen Gene unter der Kontrolle der eingebrachten Expressionseinheiten erfolgt oder
gemäß Ausführungsform d2) ein oder mehrere Gene in das Genom des Mikrorganismus einbringt, so dass die Expression eines oder mehrerer der eingebrachten Gene unter der Kontrolle der vorstehend genannten, endogenen Expressionseinheiten erfolgt oder
gemäß Ausführungsform d3) ein oder mehrere Nukleinsäurekonstrukte, enthaltend eine vorstehend genannte Expressionseinheit und funktionell verknüpft eine oder mehrere, zu exprimierende Nukleinsäuren, in den Mikroorganismus einbringt.

Ferner ist es somit möglich, die Expressionssrate eines exogenen Gens im Vergleich zum Wildtyps zu erhöhen, indem man
gemäß Ausführungsform d2) ein oder mehrere exogene Gene in das Genom des Mikrorganismus einbringt, so dass die Expression eines oder mehrerer der eingebrachten Gene unter der Kontrolle der vorstehend genannten, endogenen Expressionseinheiten erfolgt oder
gemäß Ausführungsform d3) ein oder mehrere Nukleinsäurekonstrukte, enthaltend eine vorstehend genannte Expressionseinheit, und funktionell verknüpft eine oder mehrere, zu exprimierende, exogene Nukleinsäuren, in den Mikroorganismus einbringt.

Die Insertion von Genen gemäß Ausführungsform d2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform d3) kann dabei chromosomal oder extrachromosomal erfolgen. Vorzugsweise erfolgt die Insertion der Nukleinsäurekonstrukte chromosomal.

Die Nukleinsäurekonstrukte werden im folgenden auch als Expressionskasetten bezeichnet.

In einer bevorzugten Ausführungsform der vorstehend beschriebenen Verfahren zur Erhöhung der Transkriptionsrate und/oder Expressionsrate von Genen in Mikroorganismen der Gattung Corynbacterium sind die Gene ausgewählt aus der Gruppe Nukleinsäuren kodierend ein Protein aus den Biosyntheseweg von Feinchemikalien, wobei die Gene gegebenenfalls weitere Regulationselemente enthalten können.

In einer besonders bevorzugten Ausführungsform der vorstehend beschriebenen Verfahren zur Erhöhung der Transkriptionsrate und/oder Expressionsrate von Genen in Mikroorganismen der Gattung Corynebacterium sind die Gene ausgewählt aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

In einer besondere bevorzugten Ausführungsform sind die Proteine aus dem Biosyntheseweg von Aminosäuren ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

Bevorzugte Proteine und Nukleinsäuren kodierend diese Proteine der vorstehend beschriebenen Proteine aus dem Biosyntheseweg von Aminosäuren sind Proteinsequenzen bzw. Nukleinsäuresequenzen mikrobiellen Ursprungs, vorzusgweise aus Bakterien der Gattung Corynebacterium oder Brevibacterium, bevorzugt aus coryneformen Bakterien, besonders bevorzugt aus Corynebakterium glutamicum.

Beispiele für besonders bevorzugte Proteinsequenzen und die entsprechenden Nukleinsäuresequenzen kodierend diese Proteine aus dem Biosyntheseweg von Aminosäuren, deren Bezugsdokument, sowie deren Bezeichnung im Bezugsdokument sind in Tabelle 1 aufgelistet:

**Tabelle 1**

| Protein | Nukleinsäure kodierend Protein | Bezugsdokument | SEQ. ID. NO. im Bezugsdokument |
|---|---|---|---|
| Aspartatkinase | ask oder lysC | EP1108790 | DNA: 281 |
| | | | Protein: 3781 |
| Aspartat-Semialdehyd-Dehydrogenase | asd | EP1108790 | DNA: 331 |
| | | | Protein: 3831 |
| Dihydrodipicolinate-Synthetase | dapA | WO 0100843 | DNA: 55 |
| | | | Protein: 56 |
| | | | |
| Dihydrodipicolinate-Reduktase | dapB | WO 0100843 | DNA: 35 |
| | | | Protein: 36 |
| | | | |
| Meso-Diaminopimelat-D-Dehydrogenase | ddh | EP1108790 | DNA : 3494 |
| | | | Protein : 6944 |
| Diaminopicolinat-Decarboxylase | lysA | EP1108790 | DNA: 3451 |
| | | | Prot.:6951 |
| Lysin-Exporter | lysE | EP1108790 | DNA: 3455 |
| | | | Parat.: 6955 |
| Arginyl-t-RNA Synthetase | argS | EP1108790 | DNA: 3450 |
| | | | Prot.: 6950 |
| Glucose-6-Phosphat-Dehydrognease | zwf | WO 0100844 | DNA: 243 |
| | | | Prot.: 244 |
| Glycerinaldehyd-3-Phosphat-Dehydrogenase | gap | WO 0100844 | DNA: 187 |
| | | | Prot.: 188 |
| 3-Phosphoglyceratkinase | pgk | WO 0100844 | DNA: 69 |
| | | | Prot.: 70 |
| Pyruvat-Carboxylase | pycA | EP1108790 | DNA: 765 |
| | | | Prot.: 4265 |
| Triosephosphat-Isomerase | tpi | WO 0100844 | DNA: 61 |
| | | | Prot.: 62 |
| Biotin-Ligase | birA | EP1108790 | DNA: 786 |
| | | | Prot.: 4286 |
| PEP-Carboxylase | pck | EP1108790 | DNA: 3470 |
| | | | Prot.: 6970 |
| Homoserin Kinase | thrB | WO 0100843 | DNA: 173 |
| | | | Prot.: 174 |
| Threonin Synthase | thrC | WO 0100843 | DNA: 175 |
| | | | Prot.: 176 |
| Threonin Export Carrier | thrE | WO 0251231 | DNA: 41 |
| | | | Prot.: 42 |
| Threonin Efflux Protein | RXA2390 | WO 0100843 | DNA: 7 |
| | | | Prot.: 8 |
| Threonin Dehydratase | ilvA | EP 1108790 | DNA: 2328 |
| | | | Prot.: 5828 |
| Homoserin-O-Acetyltransferase | metA | EP 1108790 | DNA:727 |
| | | | Prot: 4227 |
| Cystathionin-gamma-synthase | metB | EP 1108790 | DNA:3491 |
| | | | Prot: 6991 |
| Cystathionin-beta-Lyase | metC | EP 1108790 | DNA:2535 |
| | | | Prot: 6035 |
| Coenzym B12-abhängige Methionin-Synthase, - | metH | EP 1108790 | DNA:1663 |
| | | | Prot: 5163 |
| O-Acetylhomoserin-Sulfhydrylase | metY | EP 1108790 | DNA:726 |
| | | | Prot: 4226 |
| Methylentetrahydrofolat-Reduktase | metF | EP 1108790 | DNA:2379 |
| | | | Prot: 5879 |
| D-3-Phosphoglycerat- | serA | EP 1108790 | DNA:1415 |
| Dehydrogenase | | | Prot: 4915 |
| Phosphoserin-Phosphatase 1 | serB | WO 0100843 | DNA: 153 |
| | | | Prot.:154 |
| Phosphoserin-Phosphatase 2 | serB | EP 1108790 | DNA: 467 |
| | | | Prot: 3967 |
| Phosphoserin-Phosphatase 3 | serB | EP 1108790 | DNA: 334 |
| | | | Prot.: 3834 |
| Phosphoserin-Aminotransferase | serC | WO 0100843 | DNA: 151 |
| | | | Prot.: 152 |
| Serin Acetyl-Transferase | cysE | WO 0100843 | DNA: 243 |
| | | | Prot.: 244 |
| Cystein-Synthase I | cysK | EP 1108790 | DNA: 2817 |
| | | | Prot.: 6317 |
| Cystein Synthase II | CysM | EP 1108790 | DNA: 2338 |
| | | | Prot.: 5838 |
| Homoserin-Dehydrogenase | hom | EP 1108790 | DNA: 3452 |
| | | | Prot.: 6952 |
| Coenzym B12-unabhängige Methionin-Synthase | metE | WO 0100843 | DNA:755 |
| | | | Prot.: 756 |
| Serin-Hydroxymethyltransferase | glyA | WO 0100843 | DNA: 143 |
| | | | Prot.: 144 |
| Protein in Sulfat-Reduktion | RXA247 | EP 1108790 | DNA: 3089 |
| | | | Prot.: 6589 |
| Protein in Sulfat-Reduktion | RXA248 | EP 1108790 | DNA: 3090 |
| | | | Prot.: 6590 |
| Sulfatadenyltransferase Untereinheit 1 | CysN | EP 1108790 | DNA: 3092 |
| | | | Prot.: 6592 |
| Sulfatadenyltransferase Untereinheit 2 | CysD | EP 1108790 | DNA: 3093 |
| | | | Prot.: 6593 |
| Phosphoadenosin Phosphosulfat Reduktase | CysH | WO 02729029 | DNA: 7 |
| | | | Prot.: 8 |
| Ferredoxin-Sulfit-Reduktase | RXA073 | WO 0100842 | DNA: 329 |
| | | | Prot.: 330 |
| Ferredoxin NADP Reduktase | RXA076 | WO 0100843 | DNA: 79 |
| | | | Prot.: 80 |
| Transkriptioneller Re- | luxR | WO 0100842 | DNA: 297 |
| gulator LuxR | | | Protein: 298 |
| Transkriptioneller Regulator LysR1 | lysR1 | EP 1108790 | DNA: 676 |
| | | | Protein: 4176 |
| Transkriptioneller Regulator LysR2 | lysR2 | EP 1108790 | DNA: 3228 |
| | | | Protein: 6728 |
| Transkriptioneller Regulator LysR3 | lysR3 | EP 1108790 | DNA: 2200 |
| | | | Protein: 5700 |
| Malat-Quinon-Oxodoreduktase | mqo | WO 0100844 | DNA: 569 |
| | | | Protein: 570 |
| Transketolase | RXA2739 | EP 1108790 | DNA: 1740 |
| | | | Prot: 5240 |
| Transaldolase | RXA2738 | WO 0100844 | DNA: 245 |
| | | | Prot: 246 |
| OPCA | opcA | WO 0100804 | DNA: 79 |
| | | | Prot: 80 |
| 1-Phosphofructokinase 1 | pfk1 | WO0100844 | DNA: 55 |
| | | | Protein: 56 |
| 1-Phosphofructokinase 2 | pfk2 | WO0100844 | DNA: 57 |
| | | | Protein: 58 |
| 6-Phosphofructokinase 1 | 6-pfk1 | EP 1108790 | DNA: 1383 |
| | | | Protein: 4883 |
| 6-Phosphofructokinase 2 | 6-pfk2 | DE 10112992 | DNA: 1 |
| | | | Protein: 2 |
| Fructose-1,6-bisphosphatase 1 | fbr1 | EP1108790 | DNA: 1136 |
| | | | Protein: 4636 |
| Pyruvat Oxidase | poxB | WO 0100844 | DNA : 85 |
| | | | Protein: 86 |
| RXA00655-Regulator | RXA655 | US2003162267 | DNA: 1 |
| | | .2 | Prot.: 2 |
| RXN02910-Regulator | RXN2910 | US2003162267 | DNA: 5 |
| | | .2 | Prot.: 6 |
| 6-phosphogluconolactonase | RXA2735 | WO 0100844 | DNA: 1 |
| | | | Prot.: 2 |

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz der Fructose-1,6-bisphosphatase 2, oder auch fbr2 genannt, (SEQ. ID. NO. 38) und die entsprechenden Nukleinsäuresequenz kodierend eine Fructose-1,6-bisphosphatase 2 (SEQ. ID. NO. 37).

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz des Proteins in Sulfat-Reduktion, oder auch RXA077 genannt, (SEQ. ID. NO. 4) und die entsprechenden Nukleinsäuresequenz kodierend ein Protein in Sulfat-Reduktion (SEQ. ID. NO. 3)

Weitere besonders bevorzugte Proteinsequenzen aus dem Biosyntheseweg von Aminosäuren, weisen jeweils die in Tabelle 1 für dieses Protein angegebene Aminosäuresequenz auf, wobei das jeweilige Protein jeweils an mindestens einer der in Tabelle 2/Spalte2 für diese Aminosäuresequenz angegebenen Aminosäurepositionen eine andere proteinogene Aminosäure aufweist als die jeweilige in Tabelle2/Spalte3 in der gleichen Zeile angegebene Aminosäure. In einer weiter bevorzugten Ausführungsform, weisen die Proteine an mindestens einer der in Tabelle 2/Spalte 2 für die Aminosäuresequenz angegebenenen Aminosäureposition die in Tabelle2/Spalte4 in der gleichen Zeile angegebene Aminosäure auf. Es handelt sich bei den in Tabelle 2 angegebenen Proteine um mutierte Proteine des Biosyntheseweges von Aminosäuren, die besonders vorteilhafte Eigenschaften aufweisen und sich deshalb insbesondere zur Expression der entsprechenden Nukleinsäuren durch den erfinungsgemäßen Promotor und zur Herstellung von Aminosäuren eignen. Beispielsweise führt die Mutation T311I zu einem Ausschalten der feedback-Inhibierung von ask.

Die entsprechenden Nukleinsäuren, die ein vorstehend beschriebenes mutiertes Protein aus Tabelle 2 kodieren, lassen sich durch übliche verfahren herstellen.

Als Ausgangspunkt zur Herstellung der Nukleinsäuresequenzen kodierend ein mutiertes Protein eignet sich beispielsweise das Genom eines *Corynebacterium glutamicum-*Stammes, der von der American Type Culture Collection unter der Bezeichnung ATCC 13032 erhältlich ist oder die in Tabelle 1 in Bezug genommenen Nukleinsäuresequenzen. Für die Rückübersetzung der Aminosäuresequenz der mutierten Proteine in die Nukleinsäuresequenzen kodierend diese Proteine ist es vorteilhaft, die codon usage desjenigen Organismus zu verwenden, in den die Nukleinsäuresequenz eingebracht werden soll oder in der die Nukleinsäuresequenz vorliegt. Beispielsweise ist es vorteihaft für *Corynebakterium glutamicum* die codon usage von *Corynebakterium glutamicum* zu verwenden. Die codon usage des jeweiligen Organismus lässt sich in an sich bekannter Weise aus Datenbanken oder Patentanmeldungen ermitteln, die zumindest ein Protein und ein Gen, das dieses Protein kodiert, aus dem gewünschten Organismus beschreiben.

Die Angaben in Tabelle 2 sind folgendermassen zu verstehen:

In Spalte 1 "Identifikation" wird eine eindeutige Bezeichnung für jede Sequenz in Bezug auf Tabelle 1 aufgeführt.

In Spalte 2 "AS-POS" bezieht sich die jeweilige Zahl auf die Aminosäureposition der entsprechenden Polypeptidsequenz aus Tabelle 1. Eine "26" in der Spalte "AS-POS" bedeutet demzufolge die Aminosäureposition 26 der entsprechend angegebenen Polypeptidsequenz. Die Zählung der Position beginnt N-Terminal bei +1.

In Spalte 3 "AS-Wildtyp" bezeichnet der jeweilige Buchstabe die Aminosäure - dargestellt im Ein-Buchstaben-Code- an der in Spalte 2 angegebenen Position beim entsprechenden Wildtyp-Stamm der Sequenz aus Tabelle 1.

In Spalte 4 "AS-Mutante" bezeichnet der jeweilige Buchstabe die Aminosäure - dargestellt im Ein-Buchstaben-Code- an der in Spalte 2 angegebenen Position beim entsprechenden Mutanten-Stamm.

In Spalte 5 "Funktion" wird die physiologische Funktion der entsprechenden Polypeptidsequenz aufgeführt.

Für ein mutiertes Protein mit einer bestimmten Funktion (Spalte 5) und einer bestimmten Ausgangsaminosäuresequenz (Tabelle 1) werden in den Spalten 2,3 und 4 mindestens eine Mutation, bei einigen Sequenzen auch mehrere Mutationen beschrieben. Diese mehreren Mutationen beziehen sich immer auf die jeweils obenstehende, nächstliegendste Ausgangsaminosäuresequenz (Tabelle 1). Unter dem Begriff "mindestens eine der Aminsäurepositionen" einer bestimmten Aminosäuresequenz wird vorzugsweise mindestens eine der für diese Aminosäuresequenz in Spalte 2, 3 und 4 beschriebenen Mutationen verstanden.

Ein-Buchstaben-Code der proteinogenen Aminosäuren:
A Alanin
C Cystein
D Aspartat
E Glutamat
FPhenylalanin
G Glycin
H His
I Isoleucin
K Lysin
LLeucin
M Methionin
N Asparagin
P Prolin
Q Glutamin
R Arginin
S Serin
TThreonin
V Valin
W Tryptophan
Y Tyrosin

**Tabelle 2**

| Spalte 1 | Spalte 2 | Spalte 3 | Spalte 4 | Spalte 5 |
|---|---|---|---|---|
| Identifikation | AS Position | AS Wildtyp | AS Mutante | Funktion |
| ask | 317 | S | A | Aspartatkinase |
| | 311 | T | I | |
| | 279 | A | T | |
| asd | 66 | D | G | Aspartat-Semialdehyd-Dehydrogenase |
| | 234 | R | H | |
| | 272 | D | E | |
| | 285 | K | E | |
| | 20 | L | F | |
| dapA | 2 | S | A | Dihydrodipicolinat Synthetase |
| | 84 | K | N | |
| | 85 | L | V | |
| dapB | 91 | D | A | Dihydrodipicolinat-Reduktase |
| | 83 | D | N | |
| ddh | 174 | D | E | Meso-Diaminopimelat-D-Dehydrogenase |
| | 235 | F | L | |
| | 237 | S | A | |
| lysA | 265 | A | D | Diaminopicolinat-Decarboxylase |
| | 320 | D | N | |
| | 332 | I | V | |
| argS | 355 | G | D | Arginyl-t-RNA-Synthetase |
| | 156 | A | S | |
| | 513 | V | A | |
| | 540 | H | R | |
| zwf | 8 | S | T | Glucose-6-Phosphat-Dehydrogenase |
| | 150 | T | A | |
| | 321 | G | S | |
| gap | 264 | G | S | Glycerinaldehyd-3-Phosphat-Dehydrogenase |
| pycA | 7 | S | L | Pyruvat-Carboxylase |
| | 153 | E | D | |
| | 182 | A | S | |
| | 206 | A | S | |
| | 227 | H | R | |
| | 455 | A | G | |
| | 458 | P | S | |
| | 639 | S | T | |
| | 1008 | R | H | |
| | 1059 | S | P | |
| | 1120 | D | E | |
| pck | 162 | H | Y | PEP-Carboxylase |
| | 241 | G | D | |
| | 829 | T | R | |
| thrB | 103 | S | A | Homoserin Kinase |
| | 190 | T | A | |
| | 133 | A | V | |
| | 138 | P | S | |
| thrC | 69 | G | R | Threonin Synthase |
| | 478 | T | I | |
| RXA330 | 85 | I | M | Threonin-Efflux Protein |
| | 161 | F | I | |
| | 195 | G | D | |
| hom | 104 | V | I | Homoserin-Deydrogenase |
| | 116 | T | I | |
| | 148 | G | A | |
| | 59 | V | A | |
| | 270 | T | S | |
| | 345 | R | P | |
| | 268 | K | N | |
| | 61 | D | H | |
| | 72 | E | Q | |
| lysR1 | 80 | R | H | transkriptioneller Regulator LysR1 |
| lysR3 | 142 | R | W | transkriptioneller Regulator LysR3 |
| | 179 | A | T | |
| RXA2739 | 75 | N | D | Transketolase |
| | 329 | A | T | |
| | 332 | A | T | |
| | 556 | V | I | |
| RXA2738 | 242 | K | M | Transaldolase |
| opcA | 107 | Y | H | OpcA |
| | 219 | K | N | |
| | 233 | P | S | |
| | 261 | Y | H | |
| | 312 | S | F | |
| | 65 | G | R | Aspartat-1-Decarboxylase |
| | 33 | G | S | 6- Phosphogluconolactonase |

In den vorstehend beschriebenen Verfahren zur Erhöhung der Transkriptionsrate und/oder Expressionsrate von Genen in Mikroorganismen der Gattung Corynbacterium sowie den nachstehend beschriebenen Verfahren zur Herstellung von genetisch veränderten Mikroorganismen der Gattung Corynbacterium, den nachstehend beschriebenen genetisch veränderten Mikroorganoismen und den nachstehend beschriebenen Verfahren zur Herstellung von biosynthetischen Produkten erfolgt das Einbringen der vorstehend genannten Nukleinsäuren mit Promotoraktivität, der vorstehend genannten Expressionseinheiten, der vorstehend beschriebenen Gene und der vorstehend beschriebnen Nukleinsäurekonstrukte oder Expressionskasetten in den Mikroorganismus der Gattung Corynebacterium vorzugsweise durch die SacB-Methode.

Die SacB-Methode ist dem Fachmann bekannt und beispielsweise in Schäfer A, Tauch A, Jäger W, Kalinowski J, Thierbach G, Pühler A.; Small mobilizable multi-purpose cloning vectors derived from the Escherichia coli plasmids pK18 and pK19: selection of defined deletions in the chromosome of Corynebacterium glutamicum, Gene. 1994 Jul 22;145(1):69-73 und Blomfield IC, Vaughn V, Rest RF, Eisenstein BI.; Allelic exchange in Escherichia coli using the Bacillus subtilis sacB gene and a temperature-sensitive pSC101 replicon; Mol Microbiol. 1991 Jun;5(6):1447-57 beschrieben.

In einer bevorzugten Ausführungsform der vorstehend beschriebenen Verfahren erfolgt die Erhöhungder Transkriptionsrate und/oder Expressionsrate von Genen in Mikroorganismen der Gattung Corynbacterium durch Einbringen von vorstehend genannten Nukleinsäuren mit Promotoraktivität bzw. vorstehend genannten Expressionseinheiten in den Mikroorganismus.

In einer weiteren bevorzugten Ausführungsform der vorstehend beschriebenen Verfahren erfolgt die Erhöhungder Transkriptionsrate und/oder Expressionsrate von Genen in Mikroorganismen der Gattung Corynabacterium durch Einbringen der vorstehend beschriebenen Nukleinsäurekonstrukte oder Expressionskasetten in den Mikroorganismus.

Eine Expressionskassette umfasst
mindestens eine vorstehend genannte Expressionseinheit
mindestens eine weitere, zu exprimierende Nukleinsäuresequenz, also ein zu exprimierendes Gen und
gegebenenfalls weitere genetische Kontrollelemente, wie beispielsweise einen Terminator,
wobei mindestens eine Expressionseinheit und eine weitere, zu exprimierende, Nukleinsäuresequenz funktionell miteinander verknüpft sind und die weitere, zu exprimierende, Nukleinsäuresequenz in Bezug auf die Expressionseinheit heterolog ist.

Vorzugsweise ist die zu exprimierende Nukleinsäuresequenz mindestens eine Nukleinsäuren kodierend ein Protein aus den Biosyntheseweg von Feinchemikalien.

Besonders bevorzugt ist die zu exprimierende Nukleinsäuresequenz ausgewählt aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen.

Bevorzugte Proteine aus dem Biosyntheseweg von Aminosäuren sind vorstehend und deren Beispiele in Tabelle 1 und 2 beschrieben.

In den Expressionskassetten ist die physikalische Lage der Expressionseinheit relativ zum zu exprimierenden Gen so gewählt, daß die Expressioneinheit die Transkription und vorzugsweise auch die Translation des zu exprimierenden Gens reguliert und damit die Bildung eines oder mehrerer Proteine ermöglicht. Die "Bildung ermöglichen" beinhaltet dabei die konstitutive Steigerung der Bildung, Abschwächung bzw. Blockierung der Bildung unter spezifischen Bedingungen und oder die Steigerung der Bildung unter spezifischen Bedingungen. Die "Bedingungen" umfassen dabei: (1) Zugabe einer Komponente zum Kulturmedium, (2) Entfernen einer Komponente vom Kulturmedium, (3) Austausch einer Komponente im Kulturmedium durch eine zweite Komponente, (4) Erhöhung der Temperatur des Kulturmediums, (5) Erniedrigung der Temperatur des Kulturmediums, und (6) Regulierung der atmosphärischen Bedingungen, wie z.B. die Sauerstoff- oder Stickstoffkonzentration, in der das Kulturmedium gehalten wird.

Ferner enthält ein Expressionsvektor eine vorstehend beschriebene Expressionskassette.

Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Plasmide eignen sich solche besonders bevorzugt, die in coryneformen Bakterien repliziert werden. Zahlreiche bekannte Plasmidvektoren, wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie z. B. pCLiK5MCS, oder solche, die auf pCG4 (US-A 4,489,160) oder pNG2 (Serwold-Davis et al., FEMS Microbiology Letters 66, 119-124 (1990)) oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Remscheid et al. (Applied and Environmental Microbiology 60,126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Sirnon et al., Bio/ Technology 1,784-791 (1983)), pK18mob oder pK19mob (Schäfer et al., Gene 145,69-73 (1994)), Bernard et al., Journal of Molecular Biology, 234: 534-541 (1993)), pEM1 (Schrumpf et al. 1991, Journal of Bacteriology 173: 4510--4516) oder pBGS8 (Spratt et al., 1986, Gene 41: 337-342) in Frage. Der Plasmidvektor, der das zu amplifizierende Gen enthält, wird anschließend durch Transformation in den gewünschten Stamm von C. glutamicum überführt. Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123,343-347 (1994)) beschrieben.

Die Erfindung betrifft ferner einen genetisch verändertern Mikroorganismus der Gattung Corynbacterium, wobei die genetische Veränderung zu einer Erhöhung der Transkriptionsrate von mindestens einem Gen im Vergleich zum Wildtyp führt und bedingt ist durch

Regulation der Transkription von Genen im Mikroorganismus durch Nukleinsäuren mit Promotorakivität enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist , wobei die Gene in Bezug auf die Nukleinsäuren mit Promotoraktivität heterolog sind.

Wie vorstehend bei den Verfahren beschrieben, wird die Regulation der Transkription von Genen im Mikroorganismus durch vorstehend genannte Nukleinsäuren mit Promotoraktivität dadurch erreicht, dass man
b1) eine oder mehrere vorstehend genannte Nukleinsäuren mit Promotoraktivität in das Genom des Mikroorganismus einbringt, so dass die Transkription eines oder mehrerer endogenen Gene unter der Kontrolle der eingebrachten Nukleinsäure mit Promotoraktivität,erfolgt oder
b2) ein oder mehrere Gene in das Genom des Mikrorganismus einbringt, so dass die Transkription eines oder mehrerer der eingebrachten Gene unter der Kontrolle der endogenen vorstehend genannten Nukleinsäuren mit Promotoraktivität erfolgt oder
b3) ein oder mehrere Nukleinsäurekonstrukte, enthaltend eine vorstehend genannte Nukleinsäure mit Promotoraktivität und funktionell verknüpft eine oder mehrere, zu transkribierende Nukleinsäuren, in den Mikroorganismus einbringt.

Die Erfindung betrifft ferner einen genetisch verändertem Mikroorganismus der Gattung Corynbacterium, wobei die genetische Veränderung zu einer Erhöhungder Expressionsrate mindestens eines Gens im Vergleich zum Wildtyp führt und bedingt ist durch

Regulation der Expression von Genen im Mikroorganismus durch Expressionseinheiten enthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist), wobei die Gene im Bezug auf die Expressionseinheiten heterolog sind.

Wie vorstehend bei den Verfahren beschrieben, wird die Regulation der Expression von Genen im Mikroorganismus durch vorstehend genannten Expressionseinheiten gemäß dadurch erreicht, dass man
d1) eine oder mehrere vorstehend genannte Expressionseinheiten in das Genom des Mikroorganismus einbringt, so dass die Expression eines oder mehrerer endogenen Gene unter der Kontrolle der eingebrachten Expressionseinheiten erfolgt oder
d2) ein oder mehrere Gene in das Genom des Mikrorganismus einbringt, so dass die Expression eines oder mehrerer der eingebrachten Gene unter der Kontrolle der endogenen vorstehend genannten Expressionseinheiten erfolgt oder
d3) ein oder mehrere Nukleinsäurekonstrukte, enthaltend eine vorstehend genannte Expressionseinheit und funktionell verknüpft eine oder mehrere, zu exprimierende Nukleinsäuren, in den Mikroorganismus einbringt.

Ferner ist beschrieben ein genetisch veränderter Mikroorganismus der Gattung Corynbacterium, enthaltend eine vorstehend genannte Expressionseinheit und funktionell verknüpft ein zu eprimierendes Gen, wobei das Gen im Bezug auf die Expressionseinheit heterolog ist.

Besonders bevorzugt enthält dieser genetisch veränderte Mikroorganismus der Gattung Corynbacterium eine vorstehend genannte Expressionskasette.

Beschrieben ist auch ein gentisch veränderte Mikroorgansismen der Gattung Cornybacterium, die einen Vektor, insbesondere Pendelvektor oder Plasmidvektor, der wenigstens ein rekombinantes vorstehend genanntes Nukleinsäurekonstrukt trägt, enthalten.

In einer bevorzugten Ausführungsform der genetisch veränderten Mikroorganismen der Gattung Corynbacterium sind die vorstehend beschriebenen Gene mindestens eine Nukleinsäuren kodierend ein Protein aus den Biosyntheseweg von Feinchemikalien.

In einer besonders bevorzugten Ausführungsform der genetisch veränderten Mikroorganismen der Gattung Corynbacterium sind die vorstehend beschriebenen Gene ausgewählt sind aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

Bevorzugte Proteine aus dem Biosyntheseweg von Aminosäuren sind ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase

Besonders bevorzugte Beispiele der Proteine und Gene aus dem Biosyntheseweg von Aminosäuren sind vorstehend in Tabelle 1 und Tabelle 2 beschrieben.

Bakterien der Gattung Corynebacterium sind insbesondere die Arten Corynebacterium glutamicum, Corynebacterium acetoglutamicum, Corynebacterium acetoacidophilum, Corynebacterium thermoaminogenes, Corynebacterium melassecola und Corynebacterium efficiens.

Besonders bevorzugte Bakterien der Gattungen Corynebacterium sind ausgewählt aus der Gruppe Corynebacterium glutamicum ATCC 13032, Corynebacterium acetoglutamicum ATCC 15806, Corynebacterium acetoacidophilum ATCC 13870, Corynebacterium thermoaminogenes FERM BP-1539, Corynebacterium melassecola ATCC 17965, Corynebacterium efficiens DSM 44547, Corynebacterium efficiens DSM 44548. Corynebacterium efficiens DSM 44549, Corynebacterium glutamicum KFCC10065 und Corynebacterium glutamicum ATCC21608.

Mit der Abkürzung KFCC ist die Korean Federation of Culture Collection gemeint, mit der Abkürzung ATCC die American type strain culture collection, mit der Abkürzung DSM die Deutsche Sammlung von Mikroorganismen.

Weitere besonders bevorzugte Bakterien der Gattungen Corynebacterium sind in Tabelle 3 aufgelistet:

| Bakterium | | Hinterlegungsnummer | | | | | | | |
|---|---|---|---|---|---|---|---|---|---|
| Genus | species | ATCC | FERM | NRRL | CECT | NCIMB | CBS | NCTC | DSMZ |
| Corynebacterium | acetoacidophilum | 21476 | | | | | | | |
| Corynebacterium | acetoacidophilum | 13870 | | | | | | | |
| Corynebacterium | acetoglutamicum | | B1147 | B11473 | | | | | |
| Corynebacterium | acetoglutamicum | | | B11475 | | | | | |
| Corynebacterium | acetoglutamicum | 15806 | | | | | | | |
| Corynebacterium | acetoglutamicum | 21491 | | | | | | | |
| Corynebacterium | acetoglutamicum | 31270 | | | | | | | |
| Corynebacterium | acetophilum | | | B3671 | | | | | |
| Corynebacterium | ammoniagenes | 6872 | | | | | | 2399 | |
| Corynebacterium | ammoniagenes | 15511 | | | | | | | |
| Corynebacterium | fujiokense | 21496 | | | | | | | |
| Corynebacterium | glutamicum | 14067 | | | | | | | |
| Corynebacterium | glutamicum | 39137 | | | | | | | |
| Corynebacterium | glutamicum | 21254 | | | | | | | |
| Corynebacterium | glutamicum | 21255 | | | | | | | |
| Corynebacterium | glutamicum | 31830 | | | | | | | |
| Corynebacterium | glutamicum | 13032 | | | | | | | |
| Corynebacterium | glutamicum | 14305 | | | | | | | |
| Corynebacterium | glutamicum | 15455 | | | | | | | |
| Corynebacterium | glutamicum | 13058 | | | | | | | |
| Corynebacterium | glutamicum | 13059 | | | | | | | |
| Corynebacterium | glutamicum | 13060 | | | | | | | |
| Corynebacterium | glutamicum | 21492 | | | | | | | |
| Corynebacterium | glutamicum | 21513 | | | | | | | |
| Corynebacterium | glutamicum | 21526 | | | | | | | |
| Corynebacterium | glutamicum | 21543 | | | | | | | |
| Corynebacterium | glutamicum | 13287 | | | | | | | |
| Corynebacterium | glutamicum | 21851 | | | | | | | |
| Corynebacterium | glutamicum | 21253 | | | | | | | |
| Corynebacterium | glutamicum | 21514 | | | | | | | |
| Corynebacterium | glutamicum | 21516 | | | | | | | |
| Corynebacterium | glutamicum | 21299 | | | | | | | |
| Corynebacterium | glutamicum | 21300 | | | | | | | |
| Corynebacterium | glutamicum | 39684 | | | | | | | |
| Corynebacterium | glutamicum | 21488 | | | | | | | |
| Corynebacterium | glutamicum | 21649 | | | | | | | |
| Corynebacterium | glutamicum | 21650 | | | | | | | |
| Corynebacterium | glutamicum | 19223 | | | | | | | |
| Corynebacterium | glutamicum | 13869 | | | | | | | |
| Corynebacterium | glutamicum | 21157 | | | | | | | |
| Corynebacterium | glutamicum | 21158 | | | | | | | |
| Corynebacterium | glutamicum | 21159 | | | | | | | |
| Corynebacterium | glutamicum | 21355 | | | | | | | |
| Corynebacterium | glutamicum | 31808 | | | | | | | |
| Corynebacterium | glutamicum | 21674 | | | | | | | |
| Corynebacterium | glutamicum | 21562 | | | | | | | |
| Corynebacterium | glutamicum | 21563 | | | | | | | |
| Corynebacterium | glutamicum | 21564 | | | | | | | |
| Corynebacterium | glutamicum | 21565 | | | | | | | |
| Corynebacterium | glutamicum | 21566 | | | | | | | |
| Corynebacterium | glutamicum | 21567 | | | | | | | |
| Corynebacterium | glutamicum | 21568 | | | | | | | |
| Corynebacterium | glutamicum | 21569 | | | | | | | |
| Corynebacterium | glutamicum | 21570 | | | | | | | |
| Corynebacterium | glutamicum | 21571 | | | | | | | |
| Corynebacterium | glutamicum | 21572 | | | | | | | |
| Corynebacterium | glutamicum | 21573 | | | | | | | |
| Corynebacterium | glutamicum | 21579 | | | | | | | |
| Corynebacterium | glutamicum | 19049 | | | | | | | |
| Corynebacterium | glutamicum | 19050 | | | | | | | |
| Corynebacterium | glutamicum | 19051 | | | | | | | |
| Corynebacterium | glutamicum | 19052 | | | | | | | |
| Corynebacterium | glutamicum | 19053 | | | | | | | |
| Corynebacterium | glutamicum | 19054 | | | | | | | |
| Corynebacterium | glutamicum | 19055 | | | | | | | |
| Corynebacterium | glutamicum | 19056 | | | | | | | |
| Corynebacterium | glutamicum | 19057 | | | | | | | |
| Corynebacterium | glutamicum | 19058 | | | | | | | |
| Corynebacterium | glutamicum | 19059 | | | | | | | |
| Corynebacterium | glutamicum | 19060 | | | | | | | |
| Corynebacterium | glutamicum | 19185 | | | | | | | |
| Corynebacterium | glutamicum | 13286 | | | | | | | |
| Corynebacterium | glutamicum | 21515 | | | | | | | |
| Corynebacterium | glutamicum | 21527 | | | | | | | |
| Corynebacterium | glutamicum | 21544 | | | | | | | |
| Corynebacterium | glutamicum | 21492 | | | | | | | |
| Corynebacterium | glutamicum | | | B8183 | | | | | |
| Corynebacterium | glutamicum | | | B8182 | | | | | |
| Corynebacterium | glutamicum | | | B12416 | | | | | |
| Corynebacterium | glutamicum | | | B12417 | | | | | |
| Corynebacterium | glutamicum | | | B12418 | | | | | |
| Corynebacterium | glutamicum | | | B11476 | | | | | |
| Corynebacterium | glutamicum | 21608 | | | | | | | |
| Corynebacterium | lilium | | P973 | | | | | | |
| Corynebacterium | nitrilophilus | 21419 | | | | 11594 | | | |
| Corynebacterium | spec. | | P4445 | | | | | | |
| Corynebacterium | spec. | | P4446 | | | | | | |
| Corynebacterium | spec. | 31088 | | | | | | | |
| Corynebacterium | spec. | 31089 | | | | | | | |
| Corynebacterium | spec. | 31090 | | | | | | | |
| Corynebacterium | spec. | 31090 | | | | | | | |
| Corynebacterium | spec. | 31090 | | | | | | | |
| Corynebacterium | spec. | 15954 | | | | | | | 20145 |
| Corynebacterium | spec. | 21857 | | | | | | | |
| Corynebacterium | spec. | 21862 | | | | | | | |
| Corynebacterium | spec. | 21863 | | | | | | | |

Die Abkürzungen haben folgende Bedeutung:
   ATCC: American Type Culture Collection, Rockville, MD, USA
   FERM: Fermentation Research Institute, Chiba, Japan
   NRRL: ARS Culture Collection, Northern Regional Research Laboratory, Peoria, IL, USA
   CECT: Coleccion Espanola de Cultivos Tipo, Valencia, Spain
   NCIMB: National Collection of Industrial and Marine Bacteria Ltd., Aberdeen, UK
   CBS: Centraalbureau voor Schimmelcultures, Baam, NL
   NCTC: National Collection of Type Cultures, London, UK
   DSMZ: Deutsche Sammlung von Mikroorganismen und Zellkulturen, Braunschweig, Germany

Durch die vorstehend genannten Nukleinsäuren mit Promotoraktivität und den vorstehend genannten Expressionseinheiten ist es mit Hilfe der vorstehend beschriebenen, erfindungsgemäßen Verfahren möglich in den vorstehend beschriebenen, erfindungsgemäßen genetisch veränderten Mikroorganismen die Stoffwechselwege zu spezifischen biosynthetischen Produkten zu regulieren.

Dazu werden beispielsweise Stoffwechselwege die zu einem spezifischen biosynthetischen Produkt führen durch Erhöhung der Transkriptionrate bzw. Expressionsrate von Genen dieses Biosyntheseweges verstärkt in dem die erhöhte Proteinmenge zu einer erhöhten Gesamtaktivität dieser Proteine des gewünschten Biosyntheseweges und damit zu einem verstärkten Stoffwechselfluß zu dem gewünschen biosynthetischen Produkt führt.

Weiterhin können Stoffwechselwege die von einem spezifischen biosynthetischen Produkt wegführen durch Reduzierung der Transkriptionrate bzw. Expressionsrate von Genen dieses wegführenden Biosyntheseweges abgeschwächt werden in dem die reduzierte Proteinmenge zu einer reduzierten Gesamtaktivität dieser Proteine des unerwünschten Biosyntheseweges und damit zusätzlich zu einem verstärkten Stoffwechseifluß zu dem gewünschen biosynthetischen Produkt führt.

Die erfindungsgemäßen genetisch veränderten Mikroorganismen sind beispielsweise in der Lage aus Glucose, Saccharose, Lactose, Fructose, Maltose, Melasse, Stärke, Cellulose oder aus Glycerin und Ethanol biosynthetische Produkte herzustellen.

Beschrieben ist daher ein Verfahren zur Herstellung von biosynthetischen Produkten durch Kultivierung von erfindungsgemäßen, genetisch veränderten Mikroorganismen.

Je nach gewünschtem biosynthetischen Produkt muss die Transkriptionsrate bzw. Expressionsrate verschiedener Gene erhöht bzw. reduziert werden. In der Regel ist es vorteilhaft die Transkrioptionsrate bzw. Expressionsrate mehrere Gene zu verändern, d.h. die Transkrioptionsrate bzw. Expressionsrate einer Kombination von Gene zu Erhöhen und/oder die Transkrioptionsrate bzw. Expressionsrate einer Kombination von Gene zu reduzieren.

In den erfindungsgemäßen genetisch veränderten Mikroorganismen ist mindestens eine veränderte, dass heißt erhöhte Transkriptionsrate bzw. Expressionsrate eines Gens auf eine vorstehend genannte Nukleinsäure mit Promotoraktivität bzw. vorstehend genannte Expressionseinheit zurückzuführen.

Weitere, zusätzliche veränderte, d.h. zusätzlich erhöhte oder zusätzlich reduzierte Transkriptionsraten bzw. Expressionsraten von weiteren Genen im genetisch veränderten Mikroorganismus können, müssen aber nicht auf die vorstehend genannten Nukleinsäuren mit Promotoraktivität bzw. die vorstehend genannten Expressionseinheiten zurück gehen.

Weiterhin beschrieben ist ein Verfahren zur Herstellung von biosynthetischen Produkten durch Kultivierung von erfindungsgemäßen, genetisch veränderten Mikroorganismen der Gattung Corynbacterium.

Bevorzugte biosynthetische Produkte sind Feinchemikalien.

Der Begriff "Feinchemikalie" ist im Fachgebiet bekannt und beinhaltet Verbidnungen, die von einem Organismus produziert werden und in verschiedenen Industriezweigen Anwendungen finden, wie bspw., jedoch nicht beschränkt auf die pharmazeutische Industrie, die Landwirtschafts-, Kosmetik, Food und Feed-Industrie. Diese Verbindungen umfassen organische Säuren, wie beispielsweise Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme und sämtliche anderen von Gutcho (1983) in Chemicals by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### I. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ullmann's Encyclopedia of Industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthese mit der Ernährung aufgenommen werden müssen, werden durch einfache Biosyntheseswege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mo-nonatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakte-riellen Aminosäure-Biosynthese und ihrer Regulation, s. Umbarger, H.E. (1978) Ann. Rev. Biochem. 47: 533 - 606). Glutamat wird durch reduktive Aminierung von · - Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-· -Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf der Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### I Vitamine, Cofaktoren und Nutrazeutika-Metabolismus sowie Verwendungen

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullmann's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)-· -alanin) kann entweder durch chemische Synthese oder durch Fermentation hergestellt werden. Die letzten Schritte bei der Pantothenat-Biosynthese bestehen aus der ATP-getriebenen Kondensation von · -Alanin und Pantoinsäure. Die für die Biosyntheseschritte für die Umwandlung in Pantoinsäure, in · -Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des · -Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### III. Purin-, Pyrimidin-, Nukleosid- und Nakleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die bspw. als Immunsuppressionsmittel oder Antiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, werden gewöhnlich als Geschmacksverstärker verwendet (bspw. IMP oder GMP) oder für viele medizinische Anwendungen (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular biology, Bd. 42, Academic Press, S. 259-287; und Michal, G. (1999) "Nucleotides and Nucleosides"; Kap. 8 in : Biochemical Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### IV. Trehalose-Metabolismus und Verwendungen

Trehalose besteht aus zwei Glucosemolekülen, die über ·,· -1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. Trends Biotech. 16 (1998) 460-467; Paiva, C.L.A. und Panek, A.D. Biotech Ann. Rev. 2 (1996) 293-314; und Shiosaka, M. J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

Besonders bevorzugte biosynthetische Produkte sind ausgewählt aus der Gruppe organische Säuren, Proteine, Nukleotide und Nukleoside, sowohl proteinogene als auch nicht-proteinogene Aminosäuren, Lipide und Fettsäuren, Diole, Kohlehydrate, aromatische Verbindungen, Vitamine und Cofaktoren, Enzyme und Proteine.

### Bevorzugte organische Säuren sind Weinsäure, Itaconsäure und Diaminopimelinsäure

Bevorzugte Nukleoside und Nukleotide sind beispielsweise beschrieben in Kuninaka, A. (1996) Nucleotides and related compounds, S. 561-612, in Biotechnology Bd. 6, Rehm et al., Hrsg. VCH: Weinheim und den darin enthaltenen Zitaten.

Bevorzugte biosynthetische Produkte sind weiterhin Lipide, gesättigte und ungesättigte Fettsäuren, wie beispielsweise Arachidonsäure, Diole wie beispielsweise Propandiol und Butandiol, Kohlenhydrate, wie beispielsweise Hyaluronsäure und Trehalose, aromatische Verbindungen, wie beispielsweise aromatische Amine, Vanillin und Indigo, Vitamine und Cofaktoren, wie beispielsweise beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme Polyketide (Cane et a/. (1998) Science 282: 63-68), und sämtliche anderen von Gutcho (1983) in Chemicals by Fermentation, Noyes Data Corporation, ISBN: 0818805086 und den darin angegebenen Literaturstellen, beschriebenen Chemikalien.

Besonders bevorzugte biosynthetische Produkte sind Aminosäuren, besonders bevorzugt essentielle Aminosäuren, insbeondere L-Glycin, L-Alanin, L-leucin, L-Methionin, L-Phenylalanin, L-Tryptophan, L-Lysin, L-Glutamin, L-Glutaminsäure, L-Serin, L-Prolin, L-Valin, L-Isoleucin, L-Cystein, L-Tyrosin, L-Histidin, L-Arginin, L-Asparagin, L-Asparaginsäure und L-Threonin, L-Homoserin, insbesondere L-Lysin, L-Methionin und L-Threonin. Im folgenden wird unter unter einer Aminosäure, wie beispielsweise Lysin, Methionin und Threonin, sowohl jeweils die L- und die D-Form der Aminosäure, vorzugsweise die L-Form, also beispielsweise L-Lysin, L-Methionin und L-Threonin verstanden.

Insbesondere ist beschrieben ein Verfahren zur Herstellung von Lysin durch Kultivierung von genetisch veränderten Mikroorganismen der Gattung Corynbacterium mit erhöhter Expressionsrate mindestens eines Gens im Vergleich zum Wildtyp, wobei man
die Expression von Genen im Mikroorganismus durch Expressionseinheitenenthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist
) reguliert, wobei die Gene im Bezug auf die Expressionseinheiten heterolog sind,

und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Diaminopimelat- Dehydrogenase, Nukleinsäuren kodierend eine Diaminopimelat- Decarboxylase, Nukleinsäuren kodierend eine Dihydrodipicolinate-Synthetase, Nukleinsäuren kodierend eine Dihydridipicolinate-Reduktase, Nukleinsäuren kodierend eine Glycerinaldehyd-3-Phosphat Dehydrogenase, Nukleinsäuren kodierend eine 3-Phosphoglycerat-Kinase, Nukleinsäuren kodierend eine Pyruvat Carboxylase, Nukleinsäuren kodierend eine Triosephosphat-Isomerase, Nukleinsäuren kodierend einen Transkriptionellen Regulator LuxR, Nukleinsäuren kodierend einen Transkriptionellen Regulator LysR1, Nukleinsäuren kodierend einen Transkripti-onellen Regulator LysR2, Nukleinsäuren kodierend eine Malat-Quinon-Oxodoreduktase, Nukleinsäuren kodierend eine Glucose-6-Phosphat-Deydrognease, Nukleinsäuren kodierend eine 6-Phosphogluconat-Dehydrognease, Nukleinsäuren kodierend eine Transketolase, Nukleinsäuren kodierend eine Transaldolase, Nukleinsäuren kodierend einen Lysin Exporter, Nukleinsäuren kodierend eine Biotin-Ligase, Nukleinsäuren kodierend eine Arginyl-t-RNA-Synthetase, Nukleinsäuren kodierend eine Phosphoenolpyruvat-Carboxylase, Nukleinsäuren kodierend eine Fruktose-1,6-bisphosphatase, Nukleinsäuren kodierend ein Protein OPCA, Nukleinsäuren kodierend eine 1-Phosphofructokinase und Nukleinsäuren kodierend eine 6-Phosphofructokinase.

Wie vorstehend bei den Verfahren beschrieben, wird die Regulation der Expression dieser Genen im Mikroorganismus durch vorstehend genannte Expressionseinheiten dadurch erreicht, dass man
dh1) eine oder mehrere vorstehend genannte Expressionseinheiten in das Genom des Mikroorganismus einbringt, so dass die Expression eines oder mehrerer dieser endogenen Gene unter der Kontrolle der eingebrachten Expressionseinheitenerfolgt oder
dh2) ein oder mehrere dieser Gene in das Genom des Mikrorganismus einbringt, so dass die Expression eines oder mehrerer der eingebrachten Gene unter der Kontrolle der endogenen, vorstehend genannten Expressionseinheiten erfolgt oder
dh3) ein oder mehrere Nukleinsäurekonstrukte, enthaltend eine vorstehend genannte Expressionseinheitund funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Lysin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe
Aspartatkinase-Aktivität, Aspartat-Semialdehyd-Dehydrogenase-Aktivität, Diaminopimelat- Dehydrogenase-Aktivität, Diaminopimelat-Decarboxylase-Aktivität, Dihydrodipicolinate-Synthetase-Aktivität, Dihydridipicolinate-Reduktase-Aktivität, Glycerinaldehyd-3-Phosphat Dehydrogenase-Aktivität, 3-Phosphoglycerat-Kinase-Aktivität, Pyruvat Carboxylase-Aktivität, Triosephosphat-Isomerase-Aktivität, Aktivität des Transkriptionellen Regulators LuxR, Aktivität des Transkriptionellen Regulators LysR1, Aktivität des Transkriptionellen Regulators LysR2, Malat-Quinon-Oxodoreduktase-Aktivität, Glucose-6-Phosphat-Deydrognease-Aktivität, 6-Phosphogluconat-Dehydrognease-Aktivität, Transketolase-Aktivität, Transaldolase-Aktivität, Lysin-Exporter-Aktivität, Argi-nyl-t-RNA-Synthetase-Aktivität, Phosphoenolpyruvat-Carboxylase-Aktivität, Fruktose-1,6-bisphosphatase-Aktivität, Protein OpcA-Aktivität, 1-Phosphofructokinase-Aktivität, 6-Phosphofructokinase-Aktivität und Biotin-Ligase-Aktivität aufweisen.

Eine weiter besonders bevorzugte Ausführungsform des vorstehend beschriebenen Verfahrens zur Herstellung von Lysin ist dadurch gekennzeichnet, dass die genetisch veränderten Mikroorganismen im Vergleich zum Wildtyp zusätzlich eine reduzierte Ak-tivität, mindestens einer der Aktivitäten, ausgewählt aus der Gruppe Threonin Dehydratase-Aktivität, Homoserin O-Acetyltransferase-Aktivität, O-Acetylhomoserin-Sulfhydrylase-Aktivität, Phosphoenolpyruvat-Carboxykinase-Aktivität, Pyruvat-Oxidase-Aktivität, Homoserine-Kinase-Aktivität, Homoserin-Dehydrogenase-Aktivität, Threonin-Exporter-Aktivität, Threonin-Efflux-Protein-Aktivität, Asparaginase-Aktivität, Aspartat-Decarboxylase-Aktivität und Threonin-Synthase-Aktivität aufweisen.

Diese zusätzlichen, erhöhten oder reduzierten Aktivitäten mindestens einer der vorstehend beschriebenen Aktivitäten können, müssen aber nicht durch eine vorstehend genannte Nukleinsäure mit Promotoraktivität und/oder eine vorstehend genannte Expressionseinheit verursacht sein.

Beschrieben ist auch ein Verfahren zur Herstellung von Methionin durch Kultivierung von genetisch veränderten Mikroorganismen der Gattung Corynbacterium mit erhöhter Expressionsrate mindestens eines Gens im Vergleich zum Wildtyp, wobei man
die Expression von Genen im Mikroorganismus durch Expressionseinheiten enthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist
reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Homoserin Dehydrogenase, Nukleinsäuren kodierend eine Giycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin O-Acetyltransferase, Nukleinsäuren kodierend eine Cystahionin-gamma-Synthase, Nukleinsäuren kodierend eine Cystahionin-beta-Lyase, Nukleinsäuren kodierend eine Serin-Hydroxymethyltransferase, Nukleinsäuren kodierend eine O-Acetylhomoserin-Sulfhydrylase, Nukleinsäuren kodierend eine Methylen-Tetrahydrofolat-Reduktase, Nukleinsäuren kodierend eine Phosphoserin-Aminotransferase, Nukleinsäuren kodierend eine Phosphoserin-Phosphatase, Nukleinsäuren kodierend eine Serine Acetyl-TransferaseNukleinsäuren kodierend eine Cystein-Synthase Aktivität I, Nukleinsäuren kodierend eine Cystein-Synthase Aktivität II, Nukleinsäuren kodierend eine Coenzym B12-abhängige Methionin-Synthase-Aktivität, Nukleinsäuren kodierend eine Coenzym B12-unabhängige Methionin-Synthase-Aktivität, Nukleinsäuren kodierend eine Sulfat-Adenylyltransferase-Aktivität, Nukleinsäuren kodierend eine Phosphoadenosin-Phosphosulfat-Reductase-Aktivität, Nukleinsäuren kodierend eine Ferredoxin-Sulfit-Reduktase-Aktivität, Nukleinsäuren kodierend eine Ferredoxin NADPH-Reduktase Aktivität, Nukleinsäuren kodierend eine Ferredoxin-Aktivität, Nukleinsäuren kodierend ein Protein der Sulfat-Reduktion RXA077, Nukleinsäuren kodierend ein Protein der Sulfat-Reduktion RXA248, Nukleinsäuren kodierend ein Protein der Sulfat-Reduktion RXA247, Nukleinsäuren kodierend eine, RXA0655 Regulator undNukleinsäuren kodierend einen RXN2910 Regulator.

Wie vorstehend bei den Verfahren beschrieben, wird die Regulation der Expression dieser Genen im Mikroorganismus durch vorstehend genannte Expressionseinheiten dadurch erreicht, dass man
dh1) eine oder mehrere vorstehend genannte Expressionseinheiten, in das Genom des Mikroorganismus einbringt, so dass die Expression eines oder mehrerer dieser endogenen Gene unter der Kontrolle der eingebrachten Expressionseinheiten erfolgt oder
dh2) ein oder mehrere dieser Gene in das Genom des Mikrorganismus einbringt, so dass die Expression eines oder mehrerer der eingebrachten Gene unter der Kontrolle der endogenen, vorstehend genannten Expressionseinheiten erfolgt oder
dh3) ein oder mehrere Nukleinsäurekonstrukte, enthaltend eine e vorstehend genannte Expressionseinheit und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Methionin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Homoserin Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Homose-rin O-Acetyltransferase-Aktivität, Cystahionin-gamma-Synthase-Aktivität, Cystahionin-beta-Lyase-Aktivität Serin-Hydroxymethyltransferase-Aktivität, O-Acetylhomoserin-Sulfhydrylase-Aktivität, Methylen-Tetrahydrofolat-Reduktase-Aktivität, Phosphoserin-Aminotransferase-Aktivität, Phosphoserin-Phosphatase-Aktivität, Serine AcetylTransferase-Aktivität, Cystein-Synthase-Aktivität, Cystein-Synthase II -Aktivität, Coenzym B12-abhängige Methionin-Synthase-Aktivität, Coenzym B12-unabhängige Methionin-Synthase-Aktivität, Sulfat-Adenylyltransferase-Aktivität, Phosphoadenosin-Phosphosulfat-Reductase-Aktivität, Ferredoxin-Sulfit-Reduktase-Aktivität, Ferredoxin NADPH-Reduktase Aktivität, Ferredoxin-Aktivität Aktivität Proteins der Sulfat-Reduktion RXA077, Aktivität eines Proteins der Sulfat-Reduktion RXA248, Aktivität eines Proteins der Sulfat-Reduktion RXA247, Aktivität eines RXA655-Regulators und Aktivität eines RXN2910-Regulators aufweisen

Eine weiter besonders bevorzugte Ausführungsform des vorstehend beschriebenen Verfahrens zur Herstellung von Methionin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Homoserine-Kinase-Aktivität, Threonin-Dehydratase-Aktivität, Threonin Synthase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthase-Aktivität, Dihydrodipicolinat Reduktase-Aktivität, und Diaminopicolinat Decarboxylase-Aktivität aufweisen.

Diese zusätzlichen, erhöhten oder reduzierten Aktivitäten mindestens einer der vorstehend beschriebenen Aktivitäten können, müssen aber nicht durch eine vorstehend genannte Nukleinsäure mit Promotoraktivität und/oder eine vorstehend genannte Expressionseinheit verursacht sein.

Ferner beschrieben ist ein Verfahren zur Herstellung von Threonin durch Kultivierung von genetisch veränderten Mikroorganismen der Gattung Corynbacterium mit erhöhter Expressionsrate mindestens eines Gens im Vergleich zum Wildtyp, wobei man

die Expression von Genen im Mikroorganismus durch Expressionseinheiten enthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist
reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin-Kinase, Nukleinsäuren kodierend eine Threonin Synthase, Nukleinsäuren kodierend einen Threonin Exporter Carrier, Nukleinsäuren kodierend eine Glucose-6-Phosphat-Dehydrogenase, Nukleinsäuren kodierend eine Transaldolase, Nukleinsäuren kodierend eine Transketolase, Nukleinsäuren kodierend einer Malat-Quinon-Oxidoreductase, Nukleinsäuren kodierend eine 6-Phosphogluconat-Dehydrogenase, Nukleinsäuren kodierend einen Lysin-Exporter, Nukleinsäuren kodierend eine Biotin-Ligase, Nukleinsäuren kodierend eine Phos-phoenolpyruvat-Carboxylase, Nukleinsäuren kodierend ein Threonin Efflux-Protein, Nukleinsäuren kodierend eine Fruktose-1,6-bisphosphatase, Nukleinsäuren kodierend ein OpcA Protein, Nukleinsäuren kodierend eine 1-Phosphofructokinase, Nukleinsäuren kodierend eine 6-Phosphofructokinase, und Nukleinsäuren kodierend eine Homoserin-Dehydrogenase

Wie vorstehend bei den Verfahren beschrieben, wird die Regulation der Expression dieser Genen im Mikroorganismus durch vorstehend genannte Expressionseinheiten) dadurch erreicht, dass man
dh1) eine oder mehrere vorstehend genannte Expressionseinheiten in das Genom des Mikroorganismus einbringt, so dass die Expression eines oder mehrerer dieser endogenen Gene unter der Kontrolle der eingebrachten Expressionseinheiten, erfolgt oder
dh2) ein oder mehrere dieser Gene in das Genom des Mikrorganismus einbringt, so dass die Expression eines oder mehrerer der eingebrachten Gene unter der Kontrolle der endogenen, vorstehend genannten Expressionseinheiten, erfolgt oder
dh3) ein oder mehrere Nukleinsäurekonstrukte, enthaltend eine vorstehend genannte Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Threonin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe
Aspartatkinase-Aktivität, Aspartat-Semialdehyd-Dehydrogenase-Aktivität, Glycerinal-dehyd-3-Phosphat Dehydrogenase-Aktivität, 3-Phosphoglycerat Kinase-Aktivität, Pyruvat Carboxylase-Aktivität, Triosephosphat Isomerase-Aktivität, Threonin Synthase-Aktivität, Aktivität eines Threonin Export-Carriers, Transaldolase-Aktivität, Transketolase-Aktivität, Glucose-6-Phosphat-dehydrogenase-Aktivität, Malat-Qinon-Oxidoreductase-Aktivität, Homoserin-Kinase-Aktivität, Biotin-Ligase-Aktivität, Phosphoenolpyruvat-Carboxylase-Aktivität, Threonin-Efflux-Protein-Aktivität, Protein OpcA-Aktivität, 1-Phosphofructokinase-Aktivität, 6-Phosphofructokinase-Aktivität, Fruktose 1,6 bisphosphatase-Aktivität, 6-Phosphogluconat-Dehydrogenase und Homoserin-Dehydrogenase-Aktivität aufweisen.

Eine weiter besonders bevorzugte Ausführungsform des vorstehend beschriebenen Verfahrens zur Herstellung von Threonin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, Serin-Hydroxymethyltransferase-Aktivität, O-Acetylhomoserin-Sulfhydrylase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthetase-Aktivität, Dihydrodipicolinat Reduktase-Aktivität, Asparaginase-Aktivität, Aspartat-Decarboxylase-Aktivität, Lysin-Exporter-Aktivität, Acetolactat-Synthase-Aktivität, Ketol-Aid-Reductoisomerase-Aktivität, Branched chain aminotransferase- Aktivität, Coenzym B12-abhängige Methionin Synthase- Aktivität , Coenzym B12-unabhängige Methion Synthase- Aktivität, Dihydroxy-acid Dehydratase- Aktivität und Diaminopicolinat Decarboxylase-Aktivität aufweisen.

Diese zusätzlichen, erhöhten oder reduzierten Aktivitäten mindestens einer der vorste-hend beschriebenen Aktivitäten können, müssen aber nicht durch eine vorstehend genannte Nukleinsäure mit Promotoraktivität und/oder eine vorstehend genannte Expressionseinheit verursacht sein.

Unter dem Begriff der "Aktivität" eines Proteins wird bei Enzymen die Enzymaktivität des entsprechenden Proteins, bei anderen Proteinen, beispielsweise Struktur oder Transport-Proteinen die physiologische Aktivität der Proteine verstanden.

Die Enzyme sind in der Regel in der Lage ein Substrat in ein Produkt umzuwandeln bzw. diesen Umwandlunsgschritt zu katalysieren.

Dementsprechend wird unter der "Aktivität" eines Enzyms die in einer bestimmten Zeit durch das Enzym umgesetzte Menge Substrat bzw. gebildete Menge Produkt verstanden.

Bei einer erhöhten Aktivität im Vergleich zum Wildtyp wird somit im Vergleich zum Wildtyp in einer bestimmten Zeit durch das Enzym die umgesetzte Menge Substrat bzw. die gebildete Menge Produkt erhöht.

Vorzugsweise beträgt diese Erhöhung der "Aktivität" bei allen vorstehend und nachstehend beschriebenen Aktivitäten mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der "Aktivität des Wildtyps".

Bei einer reduzierten Aktivität im Vergleich zum Wildtyp wird somit im Vergleich zum Wildtyp in einer bestimmten Zeit durch das Enzym die umgesetzte Menge Substrat bzw. die gebildete Menge Produkt reduziert.

Unter einer reduzierten Aktivität wird vorzugsweise die teilweise oder im wesentlichen vollständige, auf unterschiedliche zellbiologische Mechanismen beruhende Unterbindung oder Blockierung der Funktionalität diese Enzyms in einem Mikroorganismus verstanden.

Eine Reduzierung der Aktivität umfasst eine mengenmäßige Verringerung eines Enzyms bis hin zu einem im wesentlichen vollständigen Fehlen des Enzyms (d.h. fehlende Nachweisbarkeit der entsprechenden Aktivität oder fehlende immunologische Nachweisbarkeit des Enzyms). Vorzugsweise wird die· Aktivität im Mikroorganismus im Vergleich zum Wildtyp um mindestens 5 %, weiter bevorzugt um mindestens 20 %, weiter bevorzugt um mindestens 50 %, weiter bevorzugt um 100 % reduziert. Insbe-sondere meint "Reduzierung" auch das vollständigen Fehlen der entsprechenden Aktivität.

Die Aktivität bestimmter Enzyme in genetisch veränderten Mikroorganismen sowie im Wildtyp und damit die Erhöhung oder Reduzierung der Enzymaktivität lassen sich nach bekannten Verfahren, wie beispielsweise Enzymassays ermitteln.

Beispielsweise wird unter eine Pyruvatcarboxylase ein Protein verstanden, das die enzymatische Aktivität aufweist, Pyruvat in Oxaloacetat umzuwandeln.

Dementsprechend wird unter einer Pyruvatcarboxylase-Aktivität die in einer bestimmten Zeit durch das Protein Pyruvatcarboxylase umgesetzte Menge Pyruvat bzw. gebildete Menge Oxaloacetat verstanden.

Bei einer erhöhten Pyruvatcarboxylase-Aktivität gegenüber dem Wildtyp wird somit im Vergleich zum Wildtyp in einer bestimmten Zeit durch das Protein Pyruvatcarboxylase die umgesetzte Menge Pyruvat bzw. die gebildete Menge Oxaloacetat erhöht.

Vorzugsweise beträgt diese Erhöhung der Pyruvatcarboxylase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Pyruvatcarboxylase -Aktivität des Wildtyps.

Weiterhin wir beispielsweise unter eine Phosphoenolpyruvat-Carboxykinase-Aktivität die Enzymaktivität einer Phosphoenolpyruvat-Carboxykinase-verstanden.

Unter einer Phosphoenolpyruvat-Carboxykinase wird ein Protein verstanden, das die enzymatische Aktivität aufweist, Oxaloacetat in Phosphoenolpyruvat umzuwandeln.

Dementsprechend wird unter Phosphoenolpyruvat-Carboxykinase-Aktivität die in einer bestimmten Zeit durch das Protein Phosphoenolpyruvat umgesetzte Menge Oxaloacetat bzw. gebildete Menge Phosphoenolpyruvat verstanden.

Bei einer reduzierten Phosphoenolpyruvat-Carboxykinase-Aktivität gegenüber dem Wildtyp wird somit im Vergleich zum Wildtyp in einer bestimmten Zeit durch das Protein Phosphoenolpyruvat-Carboxykinase die umgesetzte Menge Oxaloacetat bzw. die gebildete Menge Phosphoenolpyruvat reduziert.

Eine Reduzierung der Phosphoenolpyruvat-Carboxykinase-Aktivität umfasst eine mengenmäßige Verringerung einer· Phosphoenolpyruvat-Carboxykinase bis hin zu einem im wesentlichen vollständigen Fehlen der· Phosphoenolpyruvat-Carboxykinase· (d.h. fehlende Nachweisbarkeit von· Phosphoenolpyruvat-Carboxykinase-Aktivität oder fehlende immunologische Nachweisbarkeit der· Phosphoenolpyruvat-Carboxykinase). Vorzugsweise wird die· Phosphoenolpyruvat-Carboxykinase-Aktivität im Vergleich zum Wildtyp um mindestens 5 %, weiter bevorzugt um mindestens 20 %, weiter bevorzugt um mindestens 50 %, weiter bevorzugt um 100 % reduziert. Insbe-sondere meint "Reduzierung" auch das vollständigen Fehlen der· Phos-phoenolpyruvat-Carboxykinase -Aktivität.

Die zusätzliche Erhöhung von Aktivitäten kann durch verschiedene Wege erfolgen, beispielsweise durch Ausschalten von hemmenden Regulationsmechanismen auf Expressions- und Proteinebene oder durch Erhöhung der Genexpression von Nuk-leinsäuren kodierend die vorstehend beschriebenen Proteine gegenüber dem Wildtyp.

Die Erhöhung der Genexpression der Nukleinsäuren kodierend die vorstehend beschriebenen Proteine gegenüber dem Wildtyp kann ebenfalls durch verschiedene Wege erfolgen, beispielsweise durch Induzierung des Gens durch Aktivatoren oder wie vorstehend beschrieben durch Erhöhung der Promotoraktivität oder Erhöhung der Expressionsaktivität oder durch Einbringen von einer oder mehrerer Genkopien in den Mikroorganismus.

Unter Erhöhung der Genexpression einer Nukleinsäure codierend ein Protein wird erfindungsgemäß auch die Manipulation der Expression der Mikroorganismus eigenen, endogenen Proteine verstanden.

Es ist, wie vorstehend beschrieben, möglich, die Expression der endogenen Proteine durch die Applikation exogener Stimuli zu verändern. Dies kann durch besondere physiologische Bedingungen, also durch die Applikation von Fremdsubstanzen erfolgen.

Zur Erzielung einer Erhöhung der Genexpression kann der Fachmann weitere unter-schiedliche Maßnahmen einzeln oder in Kombination ergreifen. So kann beispielsweise die Kopienzahl der entsprechenden Gene erhöht werden, oder es kann die Promotor- und Regulationsregion oder die Ribosomenbindungsstelle, die sich stromaufwärts des Strukturgens befindet, mutiert werden. Durch induzierbare Promotoren ist es zusätzlich möglich, die Expression im Verlaufe der fermentativen Produktion zu steigern. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Biontechnology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0472869, im US Patent 4,601,893, bei Schwarzer und Pühler (Biotechnology 9, 84-87 (1991), bei Remscheid et al. (Applied and Environmental Microbiology 60,126-132 (1994), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58,.191-195 (1998)), bei Makrides (Microbiological Reviews 60 : 512-538 (1996) und in bekannten Lehrbüchern der Genetik und Molekularbiologie.

Weiterhin kann es für die Produktion von biosynthetischen Produkten, insbesondere L-Lysin, L-Methionin und L-Threonin, vorteilhaft sein, neben der Expression bzw. Verstärkung eines Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

In einer bevorzugten Ausführungsform erfolgt die Erhöhung der Genexpression einer Nukleinsäure kodierend eines der vorstehend beschriebenen Proteine durch Einbringen von mindestens einer Nukleinsäure kodierend ein entsprechendes Protein in den Mikroorganismus. Das Einbringen der Nukleinsäure kann chromsomal oder extrachromosomal erfolgen, also durch Erhöhung der Kopienzahl auf dem Chromosom und oder eine Kopie des Gens auf einem sich replizierenden Plasmid in dem Wirtsmikroorganismus.

Vorzugsweise erfolgt das Einbringen der Nukleinsäure, beispielsweise in Form einer Expressionskassete, enthaltend die Nukleinsäure, chromosomal, insbesondere durch die vorstehend beschriebene SacB-Methode.

Dazu kann prinzipiell jedes Gen, das eines der vorstehend beschriebenen Proteine kodiert verwendet werden.

Bei genomischen Nukleinsäure-Sequenzen aus eukaryontischen Quellen, die Introns enthalten, sind für den Fall das der Wirtsmikroorganismus nicht in der Lage ist oder nicht in die Lage versetzt werden kann, die entsprechenden Proteine zu exprimieren, bevorzugt bereits prozessierte Nukleinsäuresequenzen, wie die entsprechenden cDNAs zu verwenden.

Beispiele für die entsprechenden Gene sind in Tabelle 1 und 2 aufgelistet.

Vorzugsweise erfolgt die Reduzierung der vorstehend beschriebenen Aktivitäten in Mikroorganismen durch mindestens eines der nachfolgenden Verfahren:
- Einbringen mindestens einer sense-Ribonukleinsäuresequenz zur Induktion einer Kosuppression oder einer deren Expression gewährleistenden Expressionskassette
- Einbringen mindestens eines DNA- oder Protein-bindenden Faktors gegen das entsprechede -Gen, -RNA oder -Protein oder einer dessen Expression gewährleistenden Expressionskassette
- Einbringen mindestens einer den RNA-Abbau bewirkenden viralen Nukleinsäuresequenz oder einer deren Expression gewährleistenden Expressionskassette
- Einbringen mindestens eines Konstruktes zur Erzeugung eines Funktionsverlustes, wie beispielsweise die Generierung von Stopp-Kodons oder eine Verschiebungen im Leseraster, an einem Gen beispielsweise durch Erzeugung einer Insertion, Deletion, Inversion oder Mutation in einem Gen. Bevorzugt können Knockout-Mutanten mittels gezielter Insertion in das gewünschte Zielgen durch homologe Rekombination oder Einbringen von sequenzspezifischen Nukleasen gegen · das Zielgen generiert werden.
- Einbringen eines Promotors mit reduzierter Promotoraktivität oder einer Expressionseinheit mit reduzierter Expressionsaktivität.

Dem Fachmann ist bekannt, dass auch weitere Verfahren im Rahmen der vorliegenden Erfindung zur Reduzierung seiner Aktivität oder Funktion eingesetzt werden können. Beispielsweise kann auch das Einbringen einer dominant-negativen Variante eines Proteins oder einer deren Expression gewährleistenden Expressionskassette vorteilhaft sein.

Dabei kann jedes einzelne dieser Verfahren eine Verminderung der Proteinmenge, mRNA-Menge und/oder Aktivität eines Proteins bewirken. Auch eine kombinierte Anwendung ist denkbar. Weitere Methoden sind dem Fachmann bekannt und können die Behinderung oder Unterbindung der Prozessierung des Proteins, des Transports des Proteins oder dessen mRNA, Hemmung der Ribosomenanlagerung, Hemmung des RNA-Spleißens, Induktion eines· · RNA abbauenden Enzyms und/oder Hemmung der Translationselongation oder -termination umfassen.

Im erfindungsgemäßen Verfahren zur Herstellung von biosynthetischen Produkten wird vorzugsweise dem Kultivierungsschritt der genetisch veränderten Mikroorganismen der Gattung Corynbacterium ein Isolieren von biosynthetischen Produkten aus den Mikroorganismen oder/oder aus der Fermentationsbrühe angeschlossen. Diese Schritte können gleichzeitig und/oder vorzugsweisie nach dem Kultivierungsschritt stattfinden.

Die erfindungsgemäßen genetisch veränderten Mikroorganismen der Gattung Corynbacterium können kontinuierlich oder diskontinuierlich im batch- Verfahren (Satzkultivierung) oder im fed batch (Zulaufverfahren) oder repeated fed batch Verfahren (repetitives Zulaufverfahren) zur Produktion von biosynthetischen Produkten, insbesondere L-Lysin, L-Methionin und L-Threonin, kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linofsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen

Als Schwefelquelle für die Herstellung von Feinchemikalien, insbesondere von Methionin, können anorganische Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht läßt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft, in die Kultur eingetragen. Die Temperatur der Kultur liegt normalerweise bei 20°C bis 45°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf 0 bis 3 g/l gehalten, beziehungsweise abgesenkt wird.

Die Isolierung von biosynthetischen Produkten aus der Fermentationsbrühe und/oder den Mikroorganismen erfolgt in an sich bekannter Weise entsprechend den physikalisch-chemischen Eigenschaften des biosynthetischen Wertprodukts und den biosynthetischen Nebenprodukten.

Die Fermentationsbrühe kann anschließend beispielsweise weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann anschließend durch Gefriertrocknung, Sprühtrocknung, Sprühgranulation oder durch anderweitige Verfahren aufgearbeitet werden.

Es ist aber auch möglich die biosynthetischen Produkte, insbesonder L-Lysin, L-Methionin und L-Threonin, weiter aufzureinigen. Hierzu wird die produkthaltige Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die biosynthetischen Produkte können in unterschiedlichen Formen anfallen, beispielsweise in Form ihrer Salze oder Ester.

Die Identität und Reinheit der isolierten Verbindung(en) kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die Erfindung wird nun anhand der folgenden nicht-limitierenden Beispiele näher beschrieben:

### Beispiel 1

### Herstellung des Vektors pCLiK5MCS

Zunächst wurden Ampicillinresistenz und Replikationsursprung des Vektors pBR322 mit den Oligonukleotidprimern SEQ ID NO: 5 und SEQ ID NO: 6 mit Hilfe der Polymerase-Kettenreaktion (PCR) amplifiziert.
SEQ ID NO: 5
SEQ ID NO: 6

Neben den zu pBR322 komplementären Sequenzen, enthält der Oligonukleotidprimer SEQ ID NO: 5 in 5'-3' Richtung die Schnittstellen für die Restriktionsendonukleasen Smal, BamHl, Nhel und Ascl und der Oligonukleotidprimer SEQ ID NO: 6 in 5'-3' Richtung die Schnittstellen für die Restriktionsendonukleasen Xbal, Xhol, Notl und Dral. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,1 kb wurde mit dem GFX™PCR, DNA and Gel Band Purification Kit (Amersham Pharmacia, Freiburg) nach Angaben des Herstellers gereinigt. Die stumpfen Enden des DNA-Fragmentes wurden mit dem Rapid DNA Ligation Kit (Roche Diagnostics, Mannheim) nach Angaben des Herstellers miteinander ligiert und der Ligationsansatz nach Standardmethoden wie in Sambrook et al. (Molecular Cloning. A Laboratory Manual, Cold Spring Harbor, beschrieben (1989)), in kompetente E.coli XL1Blue (Stratagene, La Jolla, USA) transformiert. Eine Selektion auf Plasmid tragende Zellen wurde durch das Ausplattieren auf Ampicillin (50µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK1.

Ausgehend vom Plasmid pWLT1 (Liebl et al., 1992) als Template für eine PCR Reaktion wurde mit den Oligonukleotidprimern SEQ ID NO: 7 und SEQ ID NO: 8 eine Kanamyci n-Resistenzcassette amplifiziert.
SEQ ID NO: 7:
   5'-GAGATCTAGACCCGGGGATCCGCTAGCGGGCTGCTAAAGGAAGCGGA-3'
SEQ ID NO: 8:
   5'-GAGAGGCGCGCCGCTAGCGTGGGCGAAGAACTCCAGCA-3'

Neben den zu pWLT1 komplementären Sequenzen, enthält der Oligonukleotidprimer SEQ ID NO: 7 in 5'-3' Richtung die Schnittstellen für die Restriktionsendonukleasen Xbal, Smal, BamHl, Nhel und der Oligonukleotidprimer SEQ ID NO: 8 in 5'-3' Richtung die Schnittstellen für die Restriktionsendonukleasen Ascl und Nhel. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 1,3 kb wurde mit dem GFX™PCR, DNA and Gel Band Purification Kit (Amersham Pharmacia, Freiburg) nach Angaben des Herstellers gereinigt. Das DNA-Fragment wurde mit den Restriktionsendonukleasen Xbal und Ascl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK1 wurde ebenfalls mit den Restriktionsendonukleasen Xbal und Ascl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,1kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Ampicillin (50µg/ml) und Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK2.

Der Vektor pCLiK2 wurde mit der Restriktionsendonuklease Dral (New England Biolabs, Beverly, USA) geschnitten. Nach Elektrophorese in einem 0,8%igen Agarosegel wurde ein ca. 2,3 kb großes Vektorfragment mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers religiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK3.

Ausgehend vom Plasmid pWLQ2 (Liebl et al., 1992) als Template für eine PCR Reaktion wurde mit den Oligonukleotidprimern SEQ ID NO: 9 und SEQ ID NO: 10 der Replikationsursprung pHM1519 amplifiziert.
SEQ ID NO: 9:
   5'-GAGAGGGCGGCCGCGCAAAGTCCCGCTTCGTGAA-3'
SEQ ID NO: 10:
   5'-GAGAGGGCGGCCGCTCAAGTCGGTCAAGCCACGC-3'

Neben den zu pWLQ2 komplementären Sequenzen, enthalten die Oligonukleotidprimer SEQ ID NO: 9 und SEQ ID NO: 10 Schnittstellen für die Restriktionsendonuklease Notl. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,7 kb wurde mit dem GFX™PCR, DNA and Gel Band Purification Kit (Amersham Pharmacia, Freiburg) nach Angaben des Herstellers gereinigt. Das DNA-Fragment wurde mit der Restriktionsendonuklease Notl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK3 wurde ebenfalls mit der Restriktionsendonuklease Notl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,3kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5.

Für die Erweiterung von pCLiK5 um eine "multiple cloning site" (MCS) wurden die beide synthetischen, weitestgehend komplementären Oligonukleotide SEQ ID NO: 11 und SEQ ID NO:12, die Schnittstellen für die Restriktionsendonukleasen SwaI, XhoI, AatI, Apal, Asp718, MluI, NdeI, SpeI, EcoRV, SalI, ClaI, BamHI, XbaI und Smal enthalten, durch gemeinsames erhitzen auf 95°C und langsames abkühlen zu einem doppelsträngigen DNA-Fragment vereinigt.
SEQ ID NO: 11:
SEQ ID NO: 12:

Der Vektor pCLiK5 wurde mit den Restriktionsendonuklease XhoI und BamHI (New England Biolabs, Beverly, USA) geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 5,0 kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem synthetischen Doppelsträngigen DNA-Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5MCS.

Sequenzierungsreaktionen wurden nach Sanger et al. (1977) Proceedings of the National Academy of Sciences USA 74:5463-5467 durchgeführt. Die Sequenzierreaktionen wurden mittels ABl Prism 377 (PE Applied Biosystems, Weiterstadt) aufgetrennt und ausgewertet.

Das entstandene Plasmid pCLiK5MCS ist als SEQ ID NO: 13 aufgeführt.

### Beispiel 2

### Herstellung des Plasmids PmetA metA

Chromosomale DNA aus C. glutamicum. ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 14 und SEQ ID NO 15, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein das metA Gen inklusice des nichkodierenden 5'-Bereichs amplifiziert.
SEQ ID NO 14
   5'-GCGCGGTACCTAGACTCACCCCAGTGCT -3'
und
SEQ ID NO 15
   5'-CTCTACTAGTTTAGATGTAGAACTCGATGT-3'

Das erhaltene DNA Fragment von ca. 1,3 kb Größe wurde mit dem GFXT™PCR, DNA and Gel Band Purification Kit (Amersham Pharmacia, Freiburg) nach Angaben des Herstellers gereinigt. Im Anschluß daran wurde es mit den Restriktionsenzymen Asp718 und Spel (Roche Diagnostics, Mannheim) gespalten und das DNA Fragment mit GFX™PCR, DNA and Gel Band Purification Kit aufgereinigt.

Der Vektor pClik5MCS SEQ ID NO: 13 wurde mit den Restriktionsenzymen Asp718 und SpeI geschnitten und ein 5 kb großes Fragment nach elektrophoretischer Auftrennung mit GFX™PCR, DNA and Gel Band Purfication Kit isoliert.

Das Vektorfragment wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS PmetA metA ist als SEQ ID NO: 16 aufgeführt.

### Beispiel 3

### Herstellung des Plasmids pCLiK5MCS P EF-TU metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 17 und SEQ ID NO 18, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 200 Basenpaaren aus dem nichtkodierenden 5'-Bereich (Promotorregion) der Superoxiddismutase (Psod) amplifiziert.
SEQ ID NO 17
   5'-GAGACTCGAGGGCCGTTACCCTGCGAATG -3'
und
SEQ ID NO 18
   5'-CCTGAAGGCGCGAGGGTGGGCATTGTATGTCCTCCTGGAC -3'

Das erhaltene DNA Fragment wurde mit dem GFX™PCR, DNA and Gel Band Purification Kit (Amersham Pharmacia, Freiburg) nach Angaben des Herstellers gereinigt.

Ausgehend vom Plasmid PmetA metA SEQ ID 16 als Template für eine PCR Reaktion wurde mit den Oligonukleotidprimern SEQ ID NO: 19 und SEQ ID NO: 20 ein Teil von metA amplifiziert.
SEQ ID NO 19
   5'-CCCACCCTCGCGCCTTCAG -3'
und
SEQ ID NO 20
   5'-CTGGGTACATTGCGGCCC -3'

Das erhaltene DNA Fragment von ungefähr 470 Basenpaaren wurde mit dem GFX™PCR, DNA and Gel Band Purification Kit nach Angaben des Herstellers gereinigt.

In einer weiteren PCR Reaktion wurden die beiden oben erhaltenen Fragmente gemeinsam als Template eingesetzt. Durch die mit dem Oligonukleotidprimer SEQ ID NO: 18 eingebrachten, zu metA homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer SEQ ID NO: 17 und SEQ ID NO: 20 erst mit Beginn des 2. Zykluses dem Reaktionsansatz zugegeben wurden.

Das amplifizierte DNA Fragment von ungefähr 675 Basenpaaren wurde mit dem GFX™PCR, DNA and Gel Band Purification Kit nach Angaben des Herstellers gereinigt. Im Anschluß daran wurde es mit den Restriktionsenzymen Xhol und Ncol (Roche Diagnostics, Mannheim) gespalten und gelelektrophoretisch aufgetrennt. Anschließend wurde das ca. 620 Basenpaar große DNA Fragment mit GFX™PCR, DNA and Gel Band Purification Kit (Amersham Pharmacia, Freiburg) aus der Agarose aufgereinigt. Das Plasmid PmetA metA SEQ ID NO: 16 wurde mit den Restriktionsenzymen Ncol und SpeI (Roche Diagnostics, Mannheim) gespalten. Nach gelelektrophoretischer Auftrennung wurde ein ca. 0,7 kb großes metA Fragment mit GFX™PCR, DNA and Gel Band Purification Kit aus der Agarose aufgereinigt.

Der Vektor pClik5MCS SEQ ID NO: 13 wurde mit den Restriktionsenzymen Xhol und SpeI (Roche Diagnostics, Mannheim) geschnitten und ein 5 kb großes Fragment nach elektrophoretischer Auftrennung mit GFX™MPCR, DNA and Gel Band Purification Kit isoliert.

Das Vektorfragment wurde zusammen mit dem PCR-Fragment und dem metA-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS P_EFTUmetA ist als SEQ ID NO: 21 aufgeführt.

### Beispiel 4

### MetA-Aktivitäten

Der Stamm Corynebacterium glutamicum ATCC13032 wurde jeweils mit den Plasmiden pClikS MCS, pClik MCS PmetA metA und pCLiK5MCS P EF-TU metA nach der beschriebenen Methode (Liebl, et al. (1989) FEMS Microbiology Letters 53:299-303) transformiert. Die Transformationsmischung wurde auf CM-Platten plattiert, die zusätzlich 20mg/l Kanamycin enthielten, um eine Selektion auf Plasmid-haltige Zellen zu erreichen. Erhaltene Kan-resistente Klone wurden gepickt und vereinzelt.

C. glutamicum Stämme, die eines dieser Plasmidkonstrukte enthielten, wurden in MMA-Medium ((40 g/l Saccharose, 20 g/l (NH₄)₂SO₄, 1 g/l KH₂PO₄, 1 g/l K₂HPO₄, 0,25g/l MgSO₄ x 7H₂O, 54 g Aces, 1 ml CaCl2 (10 g/l), 1 ml Protocatechoat (300 mg/10 ml), 1 ml Spurenelementelösung (10 g/l FeSO₄ x /H₂O, 10 g/l MnSO₄ x H₂O, 2 g/l ZnSO₄ x 7 H₂O, 0,2 g/l CuSO₄, 0,02 g/l NiCl₂ x 6 H₂O), 100 µg/l Vitamin B₁₂, 0,3 mg/l Thiamin, 1 mM Leucin, 1 mg/l Pyridoxal HCl, 1 ml Biotin (100 mg/l), pH7,0) bei 30°C über Nacht angezogen. Die Zellen wurden bei 4°C abzentrifugiert und das zweimal mit kaltem Tris-HCl-Puffer (0, 1 %, pH 8,0) gewaschen. Nach erneuter Zentrifugation wurden die Zellen in kalten Tris-HCl-Puffer (0,1%, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendororfcup überführt. Der Proteingehalt wurde nach Bradford, M.M. (1976) Anal. Biochem. 72:248-254 bestimmt.

Die enzymatische Aktivität von MetA wurde wie folgt durchgeführt. Die Reaktionsansätze von 1 ml enthielten 100 mM Kaliumphosphatpuffer (pH 7,5), 5mM MgCl2, 100 µM Acetyl CoA, 5mM L-Homoserine, 500 µM DTNB (Ellmans Reagenz) und Zellextrakt. Der Test wurde durch Zugabe von dem jeweiligen Proteinlysat gestartet und bei Raumtemperatur inkubiert. Es wurde dann eine Kinetig bei 412 nm über 10 min aufgenommen.

Die Ergebnisse sind in Tabelle 1 a gezeigt.

**Tabelle 1a**

| Stamm | spezifische Aktivität [nMol/mg/min] |
|---|---|
| ATCC 13032 pClik5MCS | 12,6 |
| ATCC 13032 pClik5MCS PmetA metA | 50,7 |
| ATCC 13032 pClik5MCSP EF-TU metA | 98,4 |

Die Aktivität von MetA konnte durch die Verwendung der heterologen Expressionseinheit erheblich gesteigert werden.

### Beispiel 5

### Konstruktion von Plasmid pCIS lysC

Im ersten Schritt der Stammkonstruktion wurde ein allelischer Austausch des lysc Wildtypgens in C. glutamicum ATCC13032 durchgeführt. Dabei wurde im lysc Gen ein Nukleotidaustausch durchgeführt, so daß im resultierenden Protein die Aminosäure Thr an der Position 311 durch ein IIe ausgetauscht wurde. Ausgehend von der chromosomalen DNA aus ATCC13032 als Template für eine PCR Reaktion wurde mit den Oligonukleotidprimern SEQ ID NO 22 und SEQ ID NO: 23 lysC mit Hilfe des Pfu-Turbo PCR Systems (Stratagene USA) nach Angaben des Herstellers amplifiziert. Chromosomale DNA aus C. glutamicum ATCC 13032 wurde nach Tauch et al. (1995) Plasmid 33:168-179 oder Eikmanns et al. (1994) Microbiology 140:1817-1828 präpariert. Das amplifizierte Fragment wird an seinem 5'-Ende von einem Sall Restriktionsschnitt und an seinem 3'-Ende von einem Mlul Restriktionsschnitt flankiert. Vor der Klonierung wurde das amplifizierte Fragment durch diese beiden Restriktionsenzyme verdaut und mit GFX™PCR, DNA and Gel Band Purification Kit (Amersham Pharmacia, Freiburg) aufgereinigt.
SEQ ID NO:22
   5'-GAGAGAGAGACGCGTCCCAGTGGCTGAGACGCATC-3'
SEQ ID NO:23
   5'-CTCTCTCTGTCGACGAATTCAATCTTACGGCCTG-3'

Das erhaltenen Polynukleotid wurde über die Sall und Mlul Restriktionsschnitte in pCLlK5 MCS integrativ SacB im folgenden pCIS genannt (SEQ ID NO: 24) kloniert und in E.coli XL-1 blue transformiert. Eine Selektion auf Plasmid tragende Zellen wurde durch das Ausplattieren auf Kanamycin (20µg/ml) haltigen LB Agar (Lennox, 1955, Virology, 1:190) erreicht. Das Plasmid wurden isoliert und durch Sequenzierung die erwartete Nukleotidsequenz bestätigt. Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABl Prism 377 (PE Applied Biosystems, Weiterstadt) aufgetrennt und ausgewertet. Das erhaltene Plasmid pCIS lysC ist als SEQ ID NO:25 aufgeführt.

### Beispiel 6

### Mutagenese des lysC Gens aus C. glutamicum

Die gerichtete Mutagenese des lysC Gens aus C. glutamicum wurde mit dem QuickChange Kit (Fa. Stratagene/USA) nach Angaben des Herstellers durchgeführt. Die Mutagenese wurde im Plasmid pCIS lysC, SEQ ID NO:25 durchgeführt. Für den Austausch von thr 311 nach 311 ile mit Hilfe der Quickchange Methode (Stratagene) wurden folgende Oligonukleotidprimer synthetisiert:
SEQ ID NO:26
   5'-CGGCACCACCGACATCATCTTCACCTGCCCTCGTTCCG-3'
SEQ ID NO:27
   5'-CGGAACGAGGGCAGGTGAAGATGATGTCGGTGGTGCCG-3'

Der Einsatz dieser Oligonukleotidprimer in der Quickchange Reaktion führt in dem lysC Gen SEQ ID NO:28 zu einem Austausch des Nukleotids in Position 932 (von C nach T). Der resultierende Aminosäureaustausch Thr311lle im lysC Gen wurde nach Transformation in E.coli XL1-blue und Plasmidpräparation durch ein Sequenzierungsreaktionen bestätigt. Das Plasmid erhielt die Bezeichnung pCIS lysC thr311ile und ist als SEQ ID NO:29 aufgeführt.

Das Plasmid pCIS lysC thr311ile wurde in C. glutamicum ATCC13032 mittels Elektroporation wie bei Liebl, et al. (1989) FEMS Microbiology Letters 53:299-303 beschrieben, transformiert. Modifikationen des Protokolls sind in DE 10046870 beschrieben. Die chromosomale Anordnung des lysC-Lokus einzelner Transformanten wurde mit Standardmethoden durch Southernblot und Hybridisierung wie in Sambrook et al. (1989), Molecular Cloning. A Laboratory Manual, Cold Spring Harbor, beschrieben, überprüft. Dadurch wurde sichergestellt, daß es sich bei den Transformanten um solche handelt, die das transformierte Plasmid durch homologe Rekombination am lysCLokus integriert haben. Nach Wachstum solcher Kolonien über Nacht in Medien, die kein Antibiotikum enthalten, werden die Zellen auf ein Saccharose-CM-Agarmedium (10 g/l Pepton, 5 g/l Beef-Extrakt, 5 g/l Hefe-Extrakt, 2,5 g/l NaCl, 2 g/l Harnstoff, 1 % Glucose,10% Saccharose, pH 6,8) ausplattiert und bei 30°C für 24 Stunden inkubiert. Da das im Vektor pCIS lysC thr311ile enthaltende sacB Gen Saccharose in ein toxisches Produkt umwandelt, können nur solche Kolonien anwachsen, die das sacB Gen durch einen zweiten homologen Rekombinationsschritt zwischen dem Wildtyp lysC Gen und dem mutierten Gen lysC thr311ile deletiert haben. Während der homologen Rekombination kann entweder das Wildtyp Gen, oder das mutierte Gen zusammen mit dem sacB Gen deletiert werden. Wenn das sacB Gen zusammen mit dem Wildtyp Gen entfernt wird, resultiert eine mutierte Transformante.
Anwachsende Kolonien wurden gepickt, und auf eine Kanamycin-sensitiven Phänotyp hin untersucht. Klone mit deletiertem SacB Gen müssen gleichzeitg Kanamycin sensitives Wachstumsverhalten zeigen. Solche Kan-sensitiven Klone wurde im einem Schüttelkolben auf ihre Lysin-Produktivivät hin untersucht (siehe Beispiel 6). Zum Vergleich wurde der nicht behandelte C. glutamicum ATCC13032 angezogen. Klone mit einer gegenüber der Kontrolle erhöhten Lysin-Produktion wurden selektiert, chromosomale DNA gewonnen und der entsprechende Bereich des lysC Gens durch eine PCR-Reaktion amplifiziert und sequenziert. Ein solcher Klon mit der Eigenschaft erhöhter Lysin-Synthese und nachgewiesener Mutation in lysC an der Stelle 932 wurde mit ATCC13032 lysC^{fbr} bezeichnet.

### Beispiel 7

### Herstellung eines Integrationsplasmids zur Überexpression des lysC-Gens mit Hilfe des heterologen Expressionseinheit Peftu (SEQ ID 2)

Zur Amplifizierung des Promotors des Gens, das für den Elongationsfaktor Tu kodiert, wurden die folgenden Oligonukleotide definiert.
SEQ ID:30
   CK 352: 5'- CGCCAATTGTGGCCGTTACCCTGCGAATG-3'
SEQ ID :31
   CK 353: 5'-TTCTGTACGACCAGGGCCACTGTATGTCCTCCTGGACTTC-3'

Die Primer wurden in eine PCR-Reaktion mit chromosomaler DNA von C. glutamicum ATCC 13032 eingesetzt. Mit diesem Ansatz konnte ein DNA-Fragment amplifziert werden, das der erwarteten Größe von ca. 200 bp entsprach.

Zur Amplifizierung des Gens, das für die Aspartokinase kodiert, wurden sie folgenden Oligonukleotide definiert.
SEQ ID:32
   CK 354: 5'-GAAGTCCAGGAGGACATACAGTGGCCCTGGTCGTACAGAA-3'
SEQ ID:33
   CK 355: 5'- CATGCCCGGGACAGCAGCAAGTTCCAGCAT-3'

Die Primer wurden in eine PCR-Reaktion mit chromosomaler DNA von C. glutamicum ATCC13032 lysC^{fbr} eingesetzt. Mit diesem Ansatz konnte ein DNA-Fragment amplifiziert werden, das der erwarteten Größe von ca 620 bp entsprach.

Die Primer CK 354 und CK 353 enthalten eine überlappende Sequenz und sind an ihren 5'-Enden homolog zueinander.
Die oben erhaltenen PCR-Produkte wurden als Template für eine weitere PCR einsetzt, in der die Primer CK 352 und CK 355 genutzt wurden.

Mit diesem Ansatz konnte ein DNA-Fragment amplifiziert werden, das der erwarteten Größe von ca. 820 bp entsprach. Diese Fusion Peftu/lysC^{fbr} wurde mit den Restriktionsenzymen Munl und Smal geschnitten.

Zur Amplifikation eines 5'-Bereiches des lysC-Gens wurden folgende Oligonukleotide definiert:
SEQ ID :34
   CK 356: 5'-CGCGACGTCCGTCCCAAAACGATCATGAT-3'
SEQ ID :35
   CK 357: 5'- CGCCAATTGCTTTGTGCACCTTTCGATCT-3'

Die Primer wurden in eine PCR-Reaktion mit chromosomaler DNA von C. glutamicum eingesetzt. Mit diesem Ansatz konnte ein DNA-Fragment amplifiziert werden, das der erwarteten Größe von ca. 600 bp entsprach. Dieses DNA-Fragment wurde mit den Restriktionesenzymen Aatll und Munl verdaut. Diese Fragment und die verdaute Peftu/lysC^{fbr}-Fusion wurden dann anschließend in den Vektor pCIS kloniert, der zuvor mit den Restriktionsenzymen Aatll und Smal verdaut worden war. Das resultierende Plasmid wurde mit pCIS Peftu lysC^{fbr} (SEQ ID: 36) bezeichnet. Bis zu diesem Schritt wurden alle Klonierungen in Escherichia coli XL-1 Blue (Firma Stratagene, Amsterdam, Nierderlande) durchgeführt.

Mit dem Transformationsplasmid pCIS Peftu lysC^{fbr} wurde dann E. coli Mn522 (Firma Stratagene, Amsterdam, Nierderlande) zusammen mit dem Plasmid pTc15AcglM nach Liebl, et al. (1989) FEMS Microbiology Letters 53:299-303 transformiert. Das Plasmid pTc15AcglM ermöglicht die Methylierung von DNA nach dem Methylierungsmuster von Corynebacterium glutamicum (DE 10046870). Durch diesen Schritt wird eine anschließende Elektroporation von Corynebacterium glutamicum mit dem Integrationsplasmid pCIS Peftu lysC^{fbr} ermöglicht. Aus dieser Elektroporation und der nachfolgenden Selektion auf CM-Platten mit Kanamycin (25 µg/ml) wurden mehrere Transkonjuganten erhalten. Zur Selektion auf das zweite Rekombinationsereignis, das zur Excision des Vectors samt dem lysC-Promotor und dem lysC-Gen führen soll, wurden diese Transkonjuganten in CM-Medium über Nacht ohne Kanamycin angezogen und anschließend zur Selektion auf CM-Platten mit 10% Saccharose ausplattiert. Das auf dem Vektor pCIS vorhandenen sacB-Gen kodiert für das Enzym Laevansucrase und führt bei Wachstum auf Saccharose zur Synthese von Laevan. Da Laevan für C. glutamicum toxisch ist, können nur C. glutamicum Zellen, die das Integrationsplasmid durch den zweiten Rekombinationsschritt verloren haben, auf Saccharose-haltigem Medium wachsen (Jäger et al., Journal of Bacteriology 174 (1992) 5462-5466). 150 Saccharose-resistente Klone wurden auf ihre Kanamycin-Sensitivität hin überprüft. Für 56 der getesteten Klone konnte neben der Resistenz gegenüber Saccharose auch eine Sensitivität gegenüber Kanamycin nachgewiesen werden. Ob auch der gewünschte Austausch des natürlichen Promotors durch den Peftu-Promotor erfolgt war, wurde mittels Polymerase-Kettenreaktion (PCR) überprüft. Für diese Analyse wurde chromosomale DNA aus dem Ausgangsstamm und 20 Klonen isoliert. Hierzu wurden die jeweiligen Klone mit einem Zahnstocher von der Agarplatte abgenommen und in 100 µl H₂O suspendiert und 10 min bei 95°C aufgekocht. Jeweils 10 µl der erhaltenen Lösung wurden als Template in die PCR eingesetzt. Als Primer wurden Oligonukleotide verwendet, die zum Peftu-Promotor und dem lysC-Gen homolog sind. Die PCR-Bedingungen wurden wie folgt gewählt: Vorabdenaturierung:5 min bei 95°C; Denaturierung 30 sec bei 95°C; Hybridisierung 30 sec bei 55°C; Amplifizierung 2 min bei 72°C; 30 Zyklen,; End-Exrension 5 min bei 72°C. Im Ansatz mit der DNA des Ausgangsstammes konnte durch Wahl der Oligonukleotide kein PCR-Produkt entstehen. Nur bei Klonen, die durch die 2. Rekombination den Austausch des natürlichen Promotors (PlysC) gegen Peftu vollzogen haben, wurde ein Bande mit einer Größe von 552 bp erwartet. Insgesamt waren von den getesteten 20 Klonen 3 Klone positiv.

### Beispiel 8

### Aspartokinase (lysC) Assay

C. glutamicum Stämme, die entweder eine chromosomale Copy des lysC^{fbr}-Gens mit dem natürlichen Promotor oder eine chromosomale Copie des Peftu lysC^{fbr} Konstruktes enthielten, wurden in CM-Medium (10 g/l Pepton, 5 g/l Beef-Extrakt, 5 g/l HefeExtrakt, 2,5 g/l NaCl, 2 g/l Harnstoff, 1 % Glucose, pH 6,8) bei 30°C bis zu einer OD600 von 8 angezogen. Die Zellen wurden bei 4°C abzentrifugiert und das zweimal mit kaltem Tris-HCl-Puffer (0,1 %, pH 8,0) gewaschen. Nach erneuter Zentrifugation wurden die Zellen in kalten Tris-HCl-Puffer (0,1%, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendororfcup überführt. Der Proteingehalt wurde nach Bradford, M.M. (1976) Anal. Biochem. 72:248-254 bestimmt.

Die enzymatische Aktivität der Aspartokinase wurde wie folgt durchgeführt. Reaktionsansätze von 1 ml mit 100 mM Tris-HCl (pH8,0), 10 mM MgCl2, 600 mM Hydroxylamin-HCl (pH 73,0 mit 10 N KOH), 4 mM ATP, 200 mM Aspartat (Natriumsalz) und H₂O ad 1 ml wurden für 10 min bei 30°C inkubiert. Der Test wurde durch Zugabe von dem jeweiligen Proteinlysat gestartet und bei 30°C für 30 min inkubiert. Zum Abstoppen der Reaktion wurde 1 ml der Stoplösung (10% Eisenchlorid, 3,3 % Trichloressigsäure, 0,7 N NaCl) zum Reaktionsgemisch hinzugegeben. Nach einem Zentrifugationsschritt wurde OD₅₄₀ des Überstandes gemessen. 1 Unit entspricht dabei 1 nmol Aspartat Hydroxamat, das pro mg Protein pro Minute gebildet wird.

Die Ergebnisse sind in Tabelle 2a gezeigt.

**Tabelle 2a**

| Stamm | spezifische Aktivität [nmol/mg/min] |
|---|---|
| ATCC 13032 lysC^{fbr} | 19,3 |
| ATCC 13032 Peftu lysC^{fbr} | 49,37 |

Die Aktivität der Aspartokinase konnte durch die Integration des Peftu lysC^{fbr} Konstruktes in das Chromosom um das 2,5-fache gesteigert werden.

### Beispiel 9

### Produktion von Lysin

Zur Untersuchung der Auswirkung des Peftu lysC^{fbr} Konstruktes auf die Lysin-Produktion wurde der Stämm ATCC13032, ATCC13032 lysC^{fbr} und ATCC13032 Peftu lysC^{fbr} auf CM-Platten (10,0 g/L D-glucose, 2,5 g/L NaCl, 2,0 g/L Harnstoff, 10,0 g/L Bacto Pepton (Difco), 5,0 g/L Yeast Extract (Difco), 5,0 g/L Beef Extract (Difco), 22,0 g/L Agar (Difco), autoklaviert (20 min. 121°C)) für 2 Tag bei 30°C angezogen. Anschließend wurden die Zellen von der Platte abgekratzt und in Saline resuspendiert. Für die Hauptkultur wurden 10 ml Medium I und 0,5 g autoklaviertes CaCO₃ (Riedel de Haen) in einem 100 ml Erlenmeyerkolben mit der Zellsuspension bis zu einer OD₆₀₀ von 1,5 beimpft und für 39h auf einem vom Typ Infors AJ118 (Fa. Infors, Bottmingen, Schweiz) bei 220 upm inkubiert. Anschließend wurde die Konzentration des in das Medium ausgeschiedene Lysin bestimmt.

### Medium I:

40g/l Saccharose
60g/l Melasse (auf 100% Zuckergehalt berechnet)
10g/l (NH₄)₂SO₄
0.4g/l MgSO₄*7H₂O
0.6g/l KH₂PO₄
0.3mg/l Thiamin*HCl
1mg/l Biotin(aus einer 1 mg/ml steril flitrierten Stammlösung die mit NH₄OH auf pH 8,0 eingestellt wurde)
2mg/l FeSO₄
2mg/l MnSO₄
mit NH₄OH auf pH 7,8 eingestellt, autoklaviert (121°C, 20 min).
zusäztlich wird Vitamin B12 (Hydroxycobalamin Sigma Chemicals) aus einer Stammlösung (200 µg/ml, steril filtriert) bis zu einer Endkonzentration von 100 µg/l zugegeben

Die Bestimmung der Aminosäurekonzentration erfolgte mittels Hochdruckflüssigkeitschromatographie nach Agilent auf einer Agilent 1100 Series LC System HPLC. Eine Vorsäulenderivatisierung mit Ortho-Pthalaldehyd erlaubt die Quantifizierung der gebildeten Aminosäuren, die Auftrennung des Aminosäuregemisch findet auf einer Hypersil AA-Säule (Agilent) statt.
Das Ergebnis der Untersuchung ist in Tabelle 3a dargestellt

**Tabelle 3a**

| Stamm | L-Lysin (g/l) |
|---|---|
| ATCC13032 | 0 |
| ATCC13032 lysC^{fbr} | 10,15 |
| ATCC13032 Peftu lysC^{fbr} | 13,2 |

## Patentansprüche

1. Verfahren zur Erhöhung der Transkriptionsrate von Genen in Mikroorganismen der Gattung Corynebacterium im Vergleich zum Wildtyp durch Regulation der Transkription von Genen im Mikroorganismus durch Nukleinsäuren mit Promotoraktivität, enthaltend
A) die Nukleinsäuresequenz SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist
wobei die Gene in Bezug auf die Nukleinsäuren mit Promotoraktivität heterolog sind.

2. Verfahren nach Anspruch 1, **dadurch gekennzeichnet, dass** die Regulation der Transkription von Genen im Mikroorganismus durch die Nukleinsäuren mit Promotoraktivität dadurch erreicht wird, dass man
b1) eine oder mehrere der Nukleinsäuren mit Promotoraktivität gemäss Anspruch 1 in das Genom des Mikroorganismus einbringt, so dass die Transkription eines oder mehrerer endogenen Gene unter der Kontrolle der eingebrachten Nukleinsäure mit Promotoraktivität erfolgt oder
b2) ein oder mehrere Gene in das Genom des Mikroorganismus einbringt, so dass die Transkription eines oder mehrerer der eingebrachten Gene unter der Kontrolle der endogenen Nukleinsäuren mit Promotoraktivität gemäss Anspruch 1 erfolgt oder
b3) ein oder mehrere Nukleinsäurekonstrukte, enthaltend eine Nukleinsäure mit Promotoraktivität gemäss Anspruch 1 und funktionell verknüpft eine oder mehrere zu transkribierende Nukleinsäuren in den Mikroorganismus einbringt.

3. Verfahren nach Anspruch 1 oder 2, **dadurch gekennzeichnet, dass** die Gene ausgewählt sind aus der Gruppe Nukleinsäuren kodierend ein Protein aus dem Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindungen, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

4. Verfahren nach Anspruch 3, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulater LuxR, Transkriptioneller Regulator LysR!, Transkriptioneller Regulator LysR2, Malat-Quinon-Oxireduktase, Glucose-6-Phosphat-Dehydrogenase, 6-Phosphogluconat-Dehydrogenase, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase,Threonin-Synthase, Threonin-Exporter-Carrier, Threonin-Dehydratase, Pyruvat-Oxidase, Lysin-Exporter, Biotin-Ligase, Cystein-Synthase I, Cystein-Synthase II, Coenzym B12-abhängige Methionin-Synthase, Coenzym B12-unabhängige Methionin-Synthase, Sulfatadenyltransferase Untereinheit 1 und 2, Phosphoadenosin Phosphosulfat Reduktase, Ferredoxin-Sulfit-Reduktase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Frukose-1,6-biphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

5. Genetisch veränderter Mikroorganismus der Gattung Corynebacterium, wobei die genetische Veränderung zu einer Erhöhung der Transkriptionsrate von mindestens einem Gen im Vergleich zum Wildtyp führt und bedingt ist durch Regulation der Transkription von Genen im Mikroorganismus durch Nukleinsäuren mit Promotoraktivität, enthaltend
A) die Nukleinsäuresequenz SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist
wobei die Gene in Bezug auf die Nukleinsäuren mit Promotoraktivität heterolog sind.

6. Genetisch veränderter Mikroorganismus nach Anspruch 5, **dadurch gekennzeichnet, dass** die Regulation der Transkription von Genen im Mikroorganismus durch Nukleinsäuren mit Promotoraktivität dadurch erreicht wird, dass man
b1) eine oder mehrere der Nukleinsäuren mit Promotoraktivität gemäss Anspruch 5 in das Genom des Mikroorganismus einbringt, so dass die Transkription eines oder mehrerer endogenen Gene unter der Kontrolle der eingebrachten Nukleinsäure mit Promotoraktivität erfolgt oder
b2) ein oder mehrere Gene in das Genom des Mikroorganismus einbringt, so dass die Transkription eines oder mehrerer der eingebrachten Gene unter der Kontrolle der endogenen Nukleinsäuren mit Promotoraktivität gemäss Anspruch 5 erfolgt oder
b3) ein oder mehrere Nukleinsäurekonstrukte, enthaltend eine Nukleinsäure mit Promotoraktivität gemäss Anspruch 5 und funktionell verknüpft eine oder mehrere zu transkribierende Nukleinsäuren in den Mikroorganismus einbringt.

7. Genetisch veränderter Mikroorganismus nach Anspruch 5 oder 6, **dadurch gekennzeichnet, dass** die Gene ausgewählt sind aus der Gruppe Nukleinsäuren kodierend ein Protein aus dem Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindungen, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

8. Genetisch veränderter Mikroorganismus gemäß Anspruch 7, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulater LuxR, Transkriptioneller Regulator LysRI, Transkriptioneller Regulator LysR2, Malat-Quinon-Oxireduktase, Glucose-6-Phosphat-Dehydrogenase, 6-Phosphogluconat-Dehydrogenase, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthase I, Cystein-Synthase II, Coenzym B12-abhängige Methionin-Synthase, Coenzym B12-unabhängige Methionin-Synthase, Sulfatadenyltransferase Untereinheit 1 und 2, Phosphoadenosin Phosphosulfat Reduktase, Ferredoxin-Sulfit-Reduktase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Frukose-1,6-biphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

## Claims

1. A method for increasing the transcription rate of genes in microorganisms of the genus Corynebacterium compared with the wild type by regulating the transcription of genes in the microorganism by nucleic acids having promoter activity, comprising
A) the nucleic acid sequence SEQ.ID.NO. 1 or
B) a sequence derived from this sequence by substitution, insertion or deletion of nucleotides and having an identity of at least 90% at the nucleic acid level with the sequence SEQ.ID.NO. 1,
where the genes are heterologous in relation to the nucleic acids having promoter activity.

2. The method according to claim 1, wherein the regulation of the transcription of genes in the microorganism by the nucleic acids having promoter activity is achieved by
b1) introducing one or more of the nucleic acids having promoter activity according to claim 1 into the genome of the microorganism so that transcription of one or more endogenous genes takes place under the control of the introduced nucleic acid having promoter activity or
b2) introducing one or more genes into the genome of the microorganism so that transcription of one or more of the introduced genes takes place under the control of the endogenous nucleic acids having promoter activity according to claim 1 or
b3) introducing one or more nucleic acid constructs comprising a nucleic acid having promoter activity according to claim 1 and functionally linked one or more nucleic acids to be transcribed into the microorganism.

3. The method according to claim 1 or 2, wherein the genes are selected from the group of nucleic acids encoding a protein from the biosynthetic pathway of proteinogenic and non-proteinogenic amino acids, nucleic acids encoding a protein from the biosynthetic pathway of nucleotides and nucleosides, nucleic acids encoding a protein from the biosynthetic pathway of organic acids, nucleic acids encoding a protein from the biosynthetic pathway of lipids and fatty acids, nucleic acids encoding a protein from the biosynthetic pathway of diols, nucleic acids encoding a protein from the biosynthetic pathway of carbohydrates, nucleic acids encoding a protein from the biosynthetic pathway of aromatic compounds, nucleic acids encoding a protein from the biosynthetic pathway of vitamins, nucleic acids encoding a protein from the biosynthetic pathway of cofactors and nucleic acids encoding a protein from the biosynthetic pathway of enzymes, where the genes may optionally comprise further regulatory elements.

4. The method according to claim 3, wherein the proteins from the biosynthetic pathway of amino acids are selected from the group of aspartate kinase, aspartate-semialdehyde dehydrogenase, diaminopimelate dehydrogenase, diaminopimelate decarboxylase, dihydrodipicolinate synthetase, dihydrodipicolinate reductase, glyceraldehyde-3-phosphate dehydrogenase, 3-phosphoglycerate kinase, pyruvate carboxylase, triosephosphate isomerase, transcriptional regulator LuxR, transcriptional regulator LysR1, transcriptional regulator LysR2, malate-quinone oxireductase, glucose-6-phosphate deydrogenase, 6-phosphogluconate dehydrogenase, transketolase, transaldolase, homoserine O-acetyltransferase, cystathionine gamma-synthase, cystathionine beta-lyase, serine hydroxymethyltransferase, O-acetylhomoserine sulfhydrylase, methylenetetrahydrofolate reductase, phosphoserine aminotransferase, phosphoserine phosphatase, serine acetyltransferase, homoserine dehydrogenase, homoserine kinase, threonine synthase, threonine exporter carrier, threonine dehydratase, pyruvate oxidase, lysine exporter, biotin ligase, cysteine synthase I, cysteine synthase II, coenzyme B12-dependent methionine synthase, coenzyme B12-independent methionine synthase, sulfate adenylyltransferase subunit 1 and 2, phosphoadenosine-phosphosulfate reductase, ferredoxin-sulfite reductase, ferredoxin NADP reductase, 3-phosphoglycerate dehydrogenase, RXA00655 regulator, RXN2910 regulator, arginyl-tRNA synthetase, phosphoenolpyruvate carboxylase, threonine efflux protein, serine hydroxymethyltransferase, fructose-1,6-bisphosphatase, protein of sulfate reduction RXA077, protein of sulfate reduction RXA248, protein of sulfate reduction RXA247, protein OpcA, 1-phosphofructokinase and 6-phosphofructokinase.

5. A genetically modified microorganism of the genus Corynebacterium, where the genetic modification leads to an increase in the transcription rate of at least one gene compared with the wild type, and is dependent on regulating the transcription of genes in the microorganism by nucleic acids having promoter activity, comprising
A) the nucleic acid sequence SEQ.ID.NO. 1 or
B) a sequence derived from this sequence by substitution, insertion or deletion of nucleotides and having an identity of at least 90% at the nucleic acid level with the sequence SEQ.ID.NO. 1
where the genes are heterologous in relation to the nucleic acids having promoter activity.

6. The genetically modified microorganism according to claim 5, wherein the regulation of the transcription of genes in the microorganism by nucleic acids having promoter activity is achieved by
b1) introducing one or more of the nucleic acids having promoter activity according to claim 5 into the genome of the microorganism so that transcription of one or more endogenous genes takes place under the control of the introduced nucleic acid having promoter activity or
b2) introducing one or more genes into the genome of the microorganism so that transcription of one or more of the introduced genes takes place under the control of the endogenous nucleic acids having promoter activity according to claim 5 or
b3) introducing one or more nucleic acid constructs comprising a nucleic acid having promoter activity according to claim 5 and functionally linked one or more nucleic acids to be transcribed into the microorganism.

7. The genetically modified microorganism according to claim 5 or 6, wherein the genes are selected from the group of nucleic acids encoding a protein from the biosynthetic pathway of proteinogenic and non-proteinogenic amino acids, nucleic acids encoding a protein from the biosynthetic pathway of nucleotides and nucleosides, nucleic acid encoding a protein from the biosynthetic pathway of organic acids, nucleic acids encoding a protein from the biosynthetic pathway of lipids and fatty acids, nucleic acids encoding a protein from the biosynthetic pathway of diols, nucleic acids encoding a protein from the biosynthetic pathway of carbohydrates, nucleic acids encoding a protein from the biosynthetic pathway of aromatic compounds, nucleic acids encoding a protein from the biosynthetic pathway of vitamins, nucleic acids encoding a protein from the biosynthetic pathway of cofactors and nucleic acids encoding a protein from the biosynthetic pathway of enzymes, where the genes may optionally comprise further regulatory elements.

8. The genetically modified microorganism according to claim 7, wherein the proteins from the biosynthetic pathway of amino acids are selected from the group of aspartate kinase, aspartate-semialdehyde dehydrogenase, diaminopimelate dehydrogenase, diaminopimelate decarboxylase, dihydrodipicolinate synthetase, dihydrodipicolinate reductase, glyceraldehyde-3-phosphate dehydrogenase, 3-phosphoglycerate kinase, pyruvate carboxylase, triosephosphate isomerase, transcriptional regulator LuxR, transcriptional regulator LysR1, transcriptional regulator LysR2, malate-quinone oxireductase, glucose-6-phosphate deydrogenase, 6-phosphogluconate dehydrogenase, transketolase, transaldolase, homoserine O-acetyltransferase, cystathionine gamma-synthase, cystathionine beta-lyase, serine hydroxymethyltransferase, O-acetylhomoserine sulfhydrylase, methylenetetrahydrofolate reductase, phosphoserine aminotransferase, phosphoserine phosphatase, serine acetyltransferase, homoserine dehydrogenase, homoserine kinase, threonine synthase, threonine exporter carrier, threonine dehydratase, pyruvate oxidase, lysine exporter, biotin ligase, cysteine synthase I, cysteine synthase II, coenzyme B12-dependent methionine synthase, coenzyme B12-independent methionine synthase, sulfate adenylyltransferase subunit 1 and 2, phosphoadenosine-phosphosulfate reductase, ferredoxin-sulfite reductase, ferredoxin NADP reductase, 3-phosphoglycerate dehydrogenase, RXA00655 regulator, RXN2910 regulator, arginyl-tRNA synthetase, phosphoenolpyruvate carboxylase, threonine efflux protein, serine hydroxymethyltransferase, fructose-1,6-bisphosphatase, protein of sulfate reduction RXA077, protein of sulfate reduction RXA248, protein of sulfate reduction RXA247, protein OpcA, 1-phosphofructokinase and 6-phosphofructokinase.

## Revendications

1. Procédé pour augmenter le taux de transcription de gènes dans des microorganismes du genre Corynebacterium par comparaison avec le type sauvage, par régulation de la transcription de gènes dans le microorganisme par des acides aminés ayant une activité promotrice, contenant
A) la séquence d'acide nucléique SEQ ID NO:1 ou
B) une séquence dérivant de cette séquence par substitution, insertion ou délétion de nucléotides, et qui présente une identité d'au moins 90 % au niveau des acides nucléiques avec la séquence SEQ ID NO:1,
les gènes étant hétérologues par rapport aux acides nucléiques ayant une activité promotrice.

2. Procédé selon la revendication 1, **caractérisé en ce que** la régulation de la transcription des gènes dans le microorganisme est réalisée par les acides nucléiques ayant une activité promotrice, par le fait que
b1) on introduit dans le génome du microorganisme un ou plusieurs des acides nucléiques ayant une activité promotrice selon la revendication 1, de telle sorte que la transcription d'un ou plusieurs gènes endogènes s'effectue sous le contrôle de l'acide nucléique introduit ayant une activité promotrice, ou
b2) on introduit dans le génome du microorganisme un ou plusieurs gènes, de telle sorte que la transcription d'un ou plusieurs des gènes introduits s'effectue sous le contrôle des acides nucléiques endogènes ayant une activité promotrice selon la revendication 1, ou
b3) on introduit dans le microorganisme une ou plusieurs constructions d'acides nucléiques, contenant un acide nucléique ayant une activité promotrice selon la revendication 1 et, fonctionnellement liés, un ou plusieurs des acides nucléiques à transcrire.

3. Procédé selon la revendication 1 ou 2, **caractérisé en ce que** les gènes sont choisis dans le groupe des acides nucléiques codant pour une protéine de la voie de biosynthèse des acides aminés protéinogènes et non protéinogènes, des acides nucléiques codant pour une protéine de la voie de biosynthèse des nucléotides et des nucléosides, des acides nucléiques codant pour une protéine de la voie de biosynthèse des acides organiques, des acides nucléiques codant pour une protéine de la voie de biosynthèse des lipides et des acides gras, des acides nucléiques codant pour une protéine de la voie de biosynthèse des diols, des acides nucléiques codant pour une protéine de la voie de biosynthèse des hydrates de carbone, des acides nucléiques codant pour une protéine de la voie de biosynthèse des composés aromatiques, des acides nucléiques codant pour une protéine de la voie de biosynthèse des vitamines, des acides nucléiques codant pour une protéine de la voie de biosynthèse des cofacteurs, et des acides nucléiques codant pour une protéine de la voie de biosynthèse des enzymes, les gènes pouvant éventuellement contenir des éléments régulateurs supplémentaires.

4. Procédé selon la revendication 3, **caractérisé en ce que** les protéines de la biosynthèse des acides aminés sont choisies dans le groupe consistant en l'aspartate kinase, l'aspartate-semialdéhyde-déshydrogénase, la diaminopimélate-déshydrogénase, la diaminopimélate-décarboxylase, la dihydrodipicolinate-synthétase, la dihydrodipicolinate-réductase, la glycérinaldéhyde-3-phosphate-déshydrogénase, la 3-phosphoglycérate-kinase, la pyruvate-carboxylase, la triosephosphate-isomérase, le régulateur transcriptionnel LuxR, le régulateur transcriptionnel LysR1, le régulateur transcriptionnel LysR2, la malate-quinone-oxyréductase, la glucose-6-phosphate-déshydrogénase, la 6-phosphogluconate-déshydrogénase, la transkétolase, la transaldolase, l'homosérine-O-acétyltransférase, la cystathionine-gamma-synthase, la cystathionine-bêta-lyase, la sérine-hydroxyméthyltransférase, l'O-acétylhomosérine-sulfhydrylase, la méthylène-tétrahydrofolate-réductase, la phosphosérine-aminotransférase, la phosphosérine-phosphatase, la sérine-acétyl-transférase, l'homosérine-déshydrogénase, l'homosérine-kinase, la thréonine-synthase, le support d'exportation de la thréonine, la thréonine-déshydratase, la pyruvate-oxydase, l'exportateur de lysine, la biotine-ligase, la cystéine-synthase I, la cystéine-synthase II, la méthionine-synthase coenzyme B12-dépendante, la méthionine-synthase coenzyme B12-indépendante, les sous-unités 1 et 2 de la sulfate adénylyltransférase, la phosphoadénosine phosphosulfate réductase, la ferrédoxine-sulfite-réductase, la ferrédoxine NADP réductase, la 3-phosphoglycérate déshydrogénase, le régulateur RXA00655, le régulateur RXN2910, l'arginyl-ARNt-synthétase, la phosphoénolpyruvate-carboxylase, la protéine d'efflux de la thréonine, la sérine hydroxyméthyltransférase, la fructose-1,6-biphosphatase, la protéine de réduction des sulfates RXA077, la protéine de réduction des sulfates RXA248, la protéine de réduction des sulfates RXA247, la protéine OpcA, la 1-phosphofructokinase et la 6-phosphofructokinase.

5. Microorganisme génétiquement modifié du genre Corynebacterium, la modification génétique conduisant à une augmentation du taux de transcription d'au moins un gène par comparaison avec le type sauvage, et étant provoquée par une régulation de la transcription de gènes dans le microorganisme par des acides nucléiques ayant une activité promotrice, contenant
A) la séquence d'acide nucléique SEQ ID NO:1 ou
B) une séquence dérivant de cette séquence par substitution, insertion ou délétion de nucléotides, et qui présente une identité d'au moins 90 % au niveau des acides nucléiques avec la séquence SEQ ID NO:1,
les gènes étant hétérologues par rapport aux acides nucléiques ayant une activité promotrice.

6. Microorganisme génétiquement modifié selon la revendication 5, **caractérisé en ce que** la régulation de la transcription de gènes dans le microorganisme est réalisée par des acides nucléiques ayant une activité promotrice, par le fait que
b1) on introduit dans le génome du microorganisme un ou plusieurs des acides nucléiques ayant une activité promotrice selon la revendication 5, de telle sorte que la transcription d'un ou plusieurs gènes endogènes s'effectue sous le contrôle de l'acide nucléique introduit ayant une activité promotrice, ou
b2) on introduit dans le génome du microorganisme un ou plusieurs gènes, de telle sorte que la transcription d'un ou plusieurs des gènes introduits s'effectue sous le contrôle des acides nucléiques endogènes ayant une activité promotrice selon la revendication 5, ou
b3) on introduit dans le microorganisme une ou plusieurs constructions d'acides nucléiques, contenant un acide nucléique ayant une activité promotrice selon la revendication 5 et, fonctionnellement liés, un ou plusieurs des acides nucléiques à transcrire.

7. Microorganisme génétiquement modifié selon la revendication 5 ou 6, **caractérisé en ce que** les gènes sont choisis dans le groupe des acides nucléiques codant pour une protéine de la voie de biosynthèse des acides aminés protéinogènes et non protéinogènes, des acides nucléiques codant pour une protéine de la voie de biosynthèse des nucléotides et des nucléosides, des acides nucléiques codant pour une protéine de la voie de biosynthèse des acides organiques, des acides nucléiques codant pour une protéine de la voie de biosynthèse des lipides et des acides gras, des acides nucléiques codant pour une protéine de la voie de biosynthèse des diols, des acides nucléiques codant pour une protéine de la voie de biosynthèse des hydrates de carbone, des acides nucléiques codant pour une protéine de la voie de biosynthèse des composés aromatiques, des acides nucléiques codant pour une protéine de la voie de biosynthèse des vitamines, des acides nucléiques codant pour une protéine de la voie de biosynthèse des cofacteurs, et des acides nucléiques codant pour une protéine de la voie de biosynthèse des enzymes, les gènes pouvant éventuellement contenir des éléments régulateurs supplémentaires.

8. Micro-organisme génétiquement modifié selon la revendication 7, **caractérisée en ce que** les protéines de la biosynthèse des acides aminés sont choisies dans le groupe consistant en l'aspartate kinase, l'aspartate-semialdéhyde-déshydrogénase, la diaminopimélate-déshydrogénase, la diaminopimélate-décarboxylase, la dihydrodipicolinate-synthétase, la dihydrodipicolinate-réductase, la glycérinaldéhyde-3-phosphate-déshydrogénase, la 3-phosphoglycérate-kinase, la pyruvate-carboxylase, la triosephosphate-isomérase, le régulateur transcriptionnel LuxR, le régulateur transcriptionnel LysR1, le régulateur transcriptionnel LysR2, la malate-quinone-oxyréductase, la glucose-6-phosphate-déshydrogénase, la 6-phosphogluconate-déshydrogénase, la transkétolase, la transaldolase, l'homopsérine-O-acétyltransférase, la cystathionine-gamma-synthase, la cystathionine-bêta-lyase, la sérine-hydroxyméthyltransférase, l'O-acétylhomosérine-sulfhydrylase, la méthylène-tétrahydrofolate-réductase, la phosphosérine-aminotransférase, la phosphosérine-phosphatase, la sérine-acétyl-transférase, l'homosérine-déshydrogénase, l'homosérine-kinase, la thréonine-synthase, le support d'exportation de la thréonine, la thréonine-déshydratase, la pyruvate-oxydase, l'exportateur de lysine, la biotine-ligase, la cystéine-synthase I, la cystéine-synthase II, la méthionine-synthase coenzyme B12-dépendante, la méthionine-synthase coenzyme B12-indépendante, les sous-unités 1 et 2 de la sulfate adénylyltransférase, la phosphoadénosine phosphosulfate réductase, la ferrédoxine-sulfite-réductase, la ferrédoxine NADP réductase, la 3-phosphoglycérate déshydrogénase, le régulateur RXA00655, le régulateur RXN2910, l'arginyl-ARNt-synthétase, la phosphoénolpyruvate-carboxylase, la protéine d'efflux de la thréonine, la sérine hydroxyméthyltransférase, la fructose-1,6-biphosphatase, la protéine de réduction des sulfates RXA077, la protéine de réduction des sulfates RXA248, la protéine de réduction des sulfates RXA247, la protéine OpcA, la 1-phosphofructokinase et la 6-phosphofructokinase.
